# EUROPEAN PATENT SPECIFICATION

(11) **EP 1 577 282 B1**
(45) Date of publication and mention of the grant of the patent: **15.06.2011**
(21) Application number: 05447043.0
(22) Date of filing: 25.02.2005
(51) Int. Cl.: C07C 6/00, C07F 19/00, C07F 15/00

(54) **Metal complexes for use in olefin metathesis and atom or group transfer reactions**
Metallkomplexe und deren Verwendung in der Olefinmetathese und Atom- oder Gruppenübertragungsreaktionen
Complexes de métaux pour utilisation dans la métathèse d'oléfines et les réactions à transfert d'atome ou de groupe

(30) Priority: 26.02.2004 US 547953 P
(43) Date of publication of application: 21.09.2005
(73) Proprietor: Telene SAS, 62320 Drocourt (FR)
(72) Inventor: Verpoort, Francis Walter Cornelius, 8300 Gits (BE); Drozdzak, Renata Anna, 59100 Roubaix (FR); Ledoux, Nele, 8970 Poperinge (BE); Allaert, Bart Filip, 8650 Houthulst (BE)
(74) Representative: Albrecht, Thomas

(56) References cited:
- WO-A-99/22865
- CLERCQ B D ET AL: "Immobilization of multifunctional Schiff base containing ruthenium complexes on MCM-41" APPLIED CATALYSIS A: GENERAL, vol. 247, no. 2, 25 July 2003 (2003-07-25), pages 345-364, XP004440496 ISSN: 0926-860X

## Description

The present invention relates to transition metal complexes which are useful as catalyst components, either alone or in combination with co-catalysts or initiators, in a wide variety of organic synthesis reactions including olefin metathesis, acetylene metathesis and reactions involving the transfer of an atom or group to an ethylenically or acetylenically unsaturated compound or another reactive substrate, such as atom transfer radical polymerisation, atom transfer radical addition, vinylation, cyclopropanation of ethylenically unsaturated compounds, epoxidation, oxidative cyclisation, aziridination, cyclopropenation of alkynes, Diels-Alder reactions, Michael addition, aldol condensation of ketones or aldehydes, Robinson annulation, hydroboration, hydrosilylation, hydrocyanation of olefins and alkynes, allylic alkylation, Grignard cross-coupling, oxidation of organic compounds (including saturated hydrocarbons, sulfides, selenides, phosphines and aldehydes), hydroamidation, isomerization of alcohols into aldehydes, aminolysis of olefins, hydroxylation of olefins, hydride reduction, Heck reactions, hydroamination of olefins and alkynes, and hydrogenation of olefins or ketones.

The present invention also relates to methods for making said metal complexes and to novel intermediates involved in such methods. More particularly, the present invention relates to Schiff base derivative complexes of metals such as ruthenium, methods for making the same and their use as catalysts for the metathesis of numerous unsaturated hydrocarbons such as non-cyclic mono-olefins, dienes and alkynes, in particular for the ring-opening metathesis polymerisation of cyclic olefins, as well as catalysts for the atom transfer radical polymerisation of styrenes or (meth)acrylic esters, for the cyclopropanation of styrene and for quinoline synthesis.

### BACKGROUND OF THE INVENTION

Olefin metathesis is a catalytic process including, as a key step, a reaction between a first olefin and a first transition metal alkylidene complex, thus producing an unstable intermediate metallacyclobutane ring which then undergoes transformation into a second olefin and a second transition metal alkylidene complex according to equation (1) hereunder. Reactions of this kind are reversible and in competition with one another, so the overall result heavily depends on their respective rates and, when formation of volatile or insoluble products occur, displacement of equilibrium.

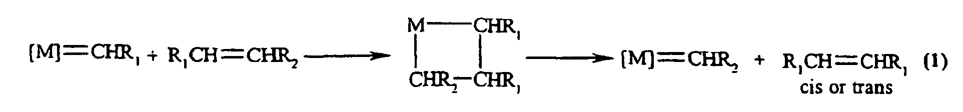

Several exemplary but non-limiting types of metathesis reactions for mono-olefins or di-olefins are shown in equations (2) to (5) herein-after. Removal of a product, such as ethylene in equation (2), from the system can dramatically alter the course and/or rate of a desired metathesis reaction, since ethylene reacts with an alkylidene complex in order to form a methylene (M=CH₂) complex, which is the most reactive and also the least stable of the alkylidene complexes.

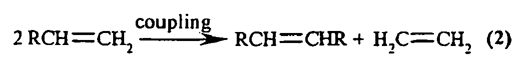

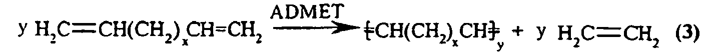

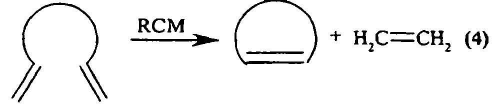

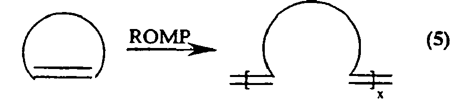

Of potentially greater interest than homo-coupling (equation 2) is cross-coupling between two different terminal olefins. Coupling reactions involving dienes lead to linear and cyclic dimers, oligomers, and, ultimately, linear or cyclic polymers (equation 3). In general, the latter reaction called acyclic diene metathesis (hereinafter referred to as ADMET) is favoured in highly concentrated solutions or in bulk, while cyclisation is favoured at low concentrations. When intra-molecular coupling of a diene occurs so as to produce a cyclic alkene, the process is called ring-closing metathesis (hereinafter referred to as RCM) (equation 4). Strained cyclic olefins can be opened and oligomerised or polymerised (ring opening metathesis polymerisation (hereinafter referred to as ROMP) shown in equation 5). When the alkylidene catalyst reacts more rapidly with the cyclic olefin (e.g. a norbornene or a cyclobutene) than with a carbon-carbon double bond in the growing polymer chain, then a "living ring opening metathesis polymerisation" may result, i.e. there is little termination during or after the polymerization reaction.

A large number of catalyst systems comprising well-defined single component metal carbene complexes have been prepared and utilized in olefin metathesis. One major development in olefin metathesis was the discovery of the ruthenium and osmium carbene complexes by Grubbs and co-workers. U.S.Patent No. 5,977,393 discloses Schiff base derivatives of such compounds, which are useful as olefin metathesis catalysts, wherein the metal is coordinated by a neutral electron donor, such as a triarylphosphine or a tri(cyclo)alkylphosphine, and by an anionic ligand. Such catalysts show an improved thermal stability while maintaining metathesis activity even in polar protic solvents. They are also able to cyclise diallylamine hydrochloride to dihydropyrrole hydrochloride. Remaining problems to be solved with the carbene complexes of Grubbs are (i) improving both catalyst stability (i.e. slowing down decomposition) and metathesis activity at the same time and (ii) broadening the range of organic products achievable by using such catalysts, e.g. providing ability to ring-close highly substituted dienes into tri- and tetra-substituted olefins.

On the other hand, living polymerisation systems were reported for anionic and cationic polymerisation, however their industrial application has been limited by the need for high-purity monomers and solvents, reactive initiators and anhydrous conditions. In contrast, free-radical polymerisation is the most popular commercial process to yield high molecular weight polymers. A large variety of monomers can be polymerised and copolymerised radically under relatively simple experimental conditions which require the absence of oxygen but can be carried out in the presence of water. However free-radical polymerisation processes often yield polymers with ill-controlled molecular weights and high polydispersities. Combining the advantages of living polymerisation and radical polymerisation is therefore of great interest and was achieved by the atom (or group) transfer radical polymerisation process (hereinafter referred as ATRP) of U.S. Patent No. 5,763,548 involving (1) the atom or group transfer pathway and (2) a radical intermediate. This type of living polymerization, wherein chain breaking reactions such as transfer and termination are substantially absent, enables control of various parameters of the macromolecular structure such as molecular weight, molecular weight distribution and terminal functionalities. It also allows the preparation of various copolymers, including block and star copolymers. Living/controlled radical polymerization requires a low stationary concentration of radicals in equilibrium with various dormant species. It makes use of novel initiation systems based on the reversible formation of growing radicals in a redox reaction between various transition metal compounds and initiators such as alkyl halides, aralkyl halides or haloalkyl esters. ATRP is based on a dynamic equilibrium between the propagating radicals and the dormant species which is established through the reversible transition metal-catalysed cleavage of the covalent carbon-halogen bond in the dormant species. Polymerisation systems utilising this concept have been developed for instance with complexes of copper, ruthenium, nickel, palladium, rhodium and iron in order to establish the required equilibrium.

Due to the development of ATRP, further interest appeared recently for the Kharash addition reaction, consisting in the addition of a polyhalogenated alkane across an olefin through a radical mechanism (first published by Kharash et al. in Science (1945) 102:169) according to the following scheme (wherein X may be hydrogen or chloro or bromo, and R and R' may be each independently selected from hydrogen, C₁₋₇ alkyl, phenyl and carboxylic acid or ester):

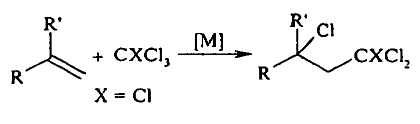

Because ATRP is quite similar to the Kharasch addition reaction, the latter may also be called Atom Transfer Radical Addition (hereinafter referred as ATRA) and attracted interest in transition metal catalysis. Research in this field also focused on the use of new olefins and telogens and a wide range of internal, terminal and cyclic olefins and diolefins were tested with a wide range of polyhalides including fluoro, chloro, bromo and iodo as halogen atoms, as described for instance in Eur. Polym. J. (1980) 16:821 and Tetrahedron (1972) 28:29.

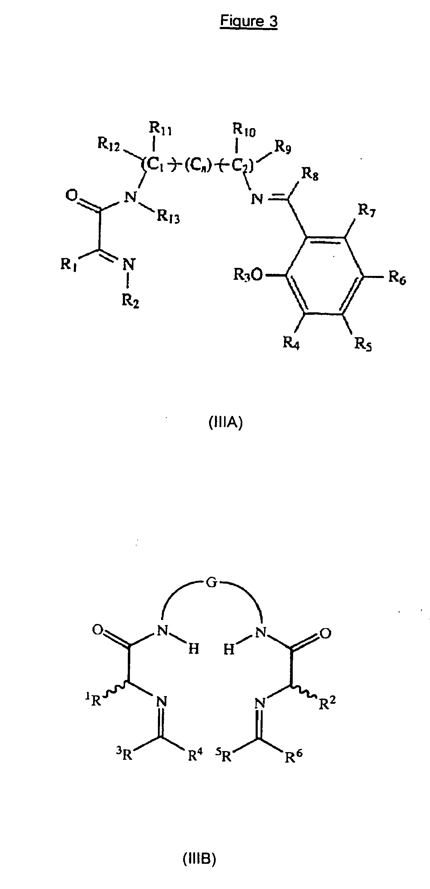

International patent application published as WO 03/062253 discloses five-coordinate metal complexes, salt, solvates or enantiomers thereof, comprising a carbene ligand, a multidentate ligand and one or more other ligands, wherein at least one of said other ligands is a constraint steric hindrance ligand having a pKa of at least 15. More specifically, the said document discloses five-coordinate metal complexes having one of the general formulae (IA) and (IB) referred to in figure 3, wherein:
- M is a metal selected from the group consisting of groups 4, 5, 6, 7, 8, 9, 10, 11 and 12 of the Periodic Table, preferably a metal selected from ruthenium, osmium, iron, molybdenum, tungsten, titanium, rhenium, copper, chromium, manganese, rhodium, vanadium, zinc, gold, silver, nickel and cobalt;
- Z is selected from the group consisting of oxygen, sulphur, selenium, NR"", PR"", AsR"" and SbR"";
- R", R"' and R"" are each a radical independently selected from the group consisting of hydrogen, C₁₋₆ alkyl, C₃₋₈ cycloalkyl, C₁₋₆ alkyl-C₁₋₆ alkoxysilyl, C₁₋₆ alkyl-aryloxysilyl, C₁₋₆ alkyl-C₃-₁₀ cycloalkoxysilyl, aryl and heteroaryl, or R" and R"' together form an aryl or heteroaryl radical, each said radical (when different from hydrogen) being optionally substituted with one or more, preferably 1 to 3, substituents R₅ each independently selected from the group consisting of halogen atoms, C₁₋₆ alkyl, C₁₋₆ alkoxy, aryl, alkylsulfonate, arylsulfonate, alkylphosphonate, arylphosphonate, C₁₋₆ alkyl-C₁₋₆ alkoxysilyl, C₁₋₆ alkyl-aryloxysilyl, C₁₋₆ alkyl-C₃₋₁₀ cycloalkoxysilyl, alkylammonium and arylammonium;
- R' is either as defined for R", R"' and R"" when included in a compound having the general formula (IA) or, when included in a compound having the general formula (IB), is selected from the group consisting of C₁₋₆ alkylene and C₃₋₈ cycloalkylene, the said alkylene or cycloalkylene group being optionally substituted with one or more substituents R₅;
- R₁ is a constraint steric hindrance group having a pKa of at least about 15;
- R₂ is an anionic ligand;
- R₃ and R₄ are each hydrogen or a radical selected from the group consisting of C₁₋₂₀ alkyl, C₂₋₂₀ alkenyl, C₂₋₂₀ alkynyl, C₁₋₂₀ carboxylate, C₁₋₂₀ alkoxy, C₂₋₂₀ alkenyloxy, C₂₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium and arylammonium;
- R' and one of R₃ and R₄ may be bonded to each other to form a bidentate ligand;
- R"' and R"" may be bonded to each other to form an aliphatic ring system including a heteroatom selected from the group consisting of nitrogen, phosphorous, arsenic and antimony;
- R₃ and R₄ together may form a fused aromatic ring system, and
- y represents the number of sp₂ carbon atoms between M and the carbon atom bearing R₃ and R₄ and is an integer from 0 to 3 inclusive,
salts, solvates and enantiomers thereof.
These five-coordinate metal complexes proved to be very efficient olefin metathesis catalysts but also very efficient components in the catalysis or initiation of atom (or group) transfer radical reactions such as ATRP or ATRA, as well as vinylation reactions, e.g. enol-ester synthesis. The same document also discloses that the Schiff base derivatives of ruthenium and osmium of U.S. Patent No. 5,977,393 as well as the corresponding derivatives of other transition metals, may also be used in the catalysis or initiation of atom (or group) transfer radical reactions such as ATRP or ATRA, as well as vinylation reactions, e.g. enol-ester synthesis.

However there is a continuous need in the art for improving catalyst efficiency, i;e. improving the yield of the reaction catalysed by the said catalyst component after a certain period of time under given conditions (e.g. temperature, pressure, solvent and reactant/catalyst ratio) or else, at a given reaction yield, providing milder conditions (lower temperature, pressure closer to atmospheric pressure, easier separation and purification of product from the reaction mixture) or requiring a smaller amount of catalyst (i.e. a higher reactant/catalyst ratio) and thus resulting in more economic and environment-friendly operating conditions. This need is still more stringent for use in reaction-injection molding (RIM) processes such as, but not limited to, the bulk polymerisation of endo- or exo-dicyclopentadiene, or formulations thereof.

WO 93/20111 describes osmium- and ruthenium-carbene compounds with phosphine ligands as purely thermal catalysts for ring-opening metathesis polymerization of strained cycloolefins, in which cyclodienes such as dicyclopentadiene act as catalyst inhibitors and cannot be polymerized. This is confirmed for instance by example 3 of U.S. Patent No. 6,284,852, wherein dicyclopentadiene did not yield any polymer, even after days in the presence of certain ruthenium carbene complexes having phosphine ligands. However, U. S. Patent No. 6,235,856 teaches that dicyclopentadiene is accessible to thermal metathesis polymerization with a single-component catalyst if carbene-free ruthenium(II)- or osmium(II)-phosphine catalysts are used.

U.S. Patent No. 6,284,852 discloses enhancing the catalytic activity of a ruthenium carbene complex of the formula AₓL_{y}X_{z}Ru=CHR', wherein x=0, 1 or 2, y=0, 1 or 2, and z=1 or 2 and wherein R' is hydrogen or a substituted or unsubstituted alkyl or aryl, L is any neutral electron donor, X is any anionic ligand, and A is a ligand having a covalent structure connecting a neutral electron donor and an anionic ligand, by the deliberate addition of specific amounts of acid not present as a substrate or solvent, the said enhancement being for a variety of olefin metathesis reactions including ROMP, RCM, ADMET and cross-metathesis and dimerization reactions. According to U.S. Patent No. 6,284,852, organic or inorganic acids may be added to the catalysts either before or during the reaction with an olefin, with longer catalyst life being observed when the catalyst is introduced to an acidic solution of olefin monomer. The amounts of acid disclosed in examples 3 to 7 of U.S. Patent No. 6,284,852 range from 0.3 to 1 equivalent of acid, relative to alkylidene. In particular, the catalyst systems of example 3 (in particular catalysts being Schiff-base-substituted complexes including an alkylidene ligand and a phosphine ligand) in the presence of HCl as an acid achieve ROMP of dicyclopentadiene within less than 1 minute at room temperature in the absence of a solvent, and ROMP of an oxanorbornene monomer within 15 minutes at room temperature in the presence of a protic solvent (methanol), however at monomer/catalyst ratios which are not specified.

U.S. Patent No. 6,284,852 also shows alkylidene ruthenium complexes which, after activation in water with a strong acid, quickly and quantitatively initiate living polymerization of water-soluble polymers, resulting in a significant improvement over existing ROMP catalysts. It further alleges that the propagating species in these reactions is stable (a propagating alkylidene species was observed by proton nuclear magnetic resonance) and that the effect of the acid in the system appears to be twofold: in addition to eliminating hydroxide ions which would cause catalyst decomposition, catalyst activity is also enhanced by protonation of phosphine ligands. It is also taught that, remarkably, the acids do not react with the ruthenium alkylidene bond.

Although providing an improvement over existing ROMP catalysts, the teaching of U.S. Patent No. 6,284,852 is limited in many aspects, namely:
- because its alleged mechanism of acid activation involves the protonation of phosphine ligands, it is limited to alkylidene ruthenium complexes including at least one phosphine ligand;
- it does not disclose reacting a Schiff-base-substituted ruthenium complex with an acid under conditions such that said acid at least partly cleaves a bond between the metal and the Schiff base ligand of said ruthenium complex.

U.S. Patent No. 6,284,852 does not either teach the behaviour, in the presence of an acid, of ruthenium complexes wherein ruthenium is coordinated with a vinylidene ligand, an allenylidene ligand or a N-heterocyclic carbene ligand.

U.S. Patent No. 6,284,852 therefore has left open ways for the study of metal complexes, in particular multicoordinated ruthenium and osmium complexes in an acidic, preferably a strongly acidic, environment when used for olefin metathesis reactions including ROMP, RCM, ADMET and cross-metathesis and dimerization reactions.

Applied catalysis A (2003) 247:345 discloses the catalytic behavior of Schiff base and phosphine containing ruthenium complexes having been covalently anchored to a solid support. In this document, vinylation experiments are described where a large amount of a carboxylic acid (110 eq/cat) is used as a reactant. This document further postulates a mechanism that explains for observed selectivities in vinylation experiments. In that mechanism, a first step involves a "one arm" dissociation of the bidentate Schiff base ligand, followed by coordination of an allylic carboxylate group. Applied catalysis A (2003) 247:345 does not report improvement of the catalytic activity that would be induced by the presence of the acid.

Therefore one goal of this invention is the design of new and useful catalytic species, especially based on multicoordinated transition metal complexes, having unexpected properties and improved efficiency in olefin metathesis reactions as well as in other atom or group transfer reactions such as ATRP or ATRA. Another goal of this invention is to efficiently perform olefin metathesis reactions, in particular ring opening polymerization of strained cyclic olefins (including cationic forms of such monomers such as, but not limited to, strained cyclic olefins including quaternary ammonium salts), in the presence of multicoordinated transition metal complexes without being limited by the requirement of a phosphine ligand in said complexes.

There is also a specific need in the art, which is yet another goal of this invention, for improving reaction-injection molding (RIM) processes, resin transfer molding (RTM) processes and reactive rotational molding (RRM) processes such as, but not limited to, the bulk polymerisation of endo- or exo-dicyclopentadiene, or copolymerization thereof with other monomers, or formulations thereof, with the use of multicoordinated transition metal complexes, in particular ruthenium complexes, having various combinations of ligands but which do not necessarily comprise phosphine ligands. All the above needs constitute the various goals to be achieved by the present invention, nevertheless other advantages of this invention will readily appear from the following description.

### SUMMARY OF THE INVENTION

The present invention is based on the unexpected finding that improved catalysts useful in a number of organic synthesis reactions such as, but not limited to, olefin metathesis and atom or group transfer reactions can be obtained by bringing into contact a multi-coordinated ruthenium, osmium or iron complex, preferably an at least tetra-coordinated complex of these metals, comprising a multidentate Schiff base ligand and one or more other ligands such as, but not limited to, the metal complexes of WO 03/062253, with an acid under conditions such that said acid is able to at least partly cleave a bond between the metal and the multidentate Schiff base ligand of said metal complex.

The present invention is based on the unexpected finding that new and useful catalytic species can be suitably obtained by reacting an acid with a multi-coordinated ruthenium, osmium or iron complex, preferably an at least tetra-coordinated complex of these metals, comprising a multidentate Schiff base ligand and further comprising one or more other ligands such as, but not limited to, anionic ligands, N-heterocyclic carbene ligands, alkylidene ligands, vinylidene ligands, indenylidene ligands and allenylidene ligands, under conditions that do not involve the protonation of a phosphine ligand. In particular this invention is based on the unexpected finding that new and useful catalytic species can be suitably obtained by reacting an acid with a multi-coordinated ruthenium, osmium or iron complex, preferably an at least tetra-coordinated complex of these metals, comprising a multidentate Schiff base ligand and further comprising a set of other ligands, wherein said set of other ligands is free from any phosphine ligand. More specifically, this invention is based on the finding that suitable conditions for the acid activation reaction between the acid and the multi-coordinated ruthenium, osmium or iron complex are conditions which permit, in one or several steps, the at least partial protonation of the multidentate Schiff base ligand and the at least partial decoordination of the multidentate Schiff base ligand through cleavage of the imine bond to the metal center.

Based on these findings, the present invention thus provides new catalytic species or products, or mixtures of species, deriving from the reaction (hereinafter also referred as "activation") between the starting multi-coordinated Schiff-base-substituted ruthenium, osmium or iron complex, preferably an at least tetra-coordinated complex of these metals, comprising a multidentate Schiff base ligand and further comprising one or more other ligands (said other ligands being preferably other than phosphine ligands) and said acid, preferably under conditions suitable for protonation of the multidentate Schiff base ligand and/or decoordination of the multidentate Schiff base ligand through cleavage of the imine bond to the metal center. In the broader acceptance, these species may be monometallic species represented by the general formula:

[M(L_{c})(L₂)(X)(SB⁺)]X⁻

wherein
- M is a metal selected from the group consisting of ruthenium, osmium and iron;
- SB⁺ is a protonated Schiff base ligand;
- L_{c} is a carbene ligand selected from the group consisting of alkylidene ligands, vinylidene ligands, indenylidene ligands and allenylidene ligands;
- L₂ is a non-anionic ligand, preferably other than a phosphine ligand;
- X is an anionic ligand; and
- X⁻ is an anion,
including salts, solvates and enantiomers thereof.

These species may also be bimetallic species represented by the general formula:

[M(L_{c})(SB⁺)(X₁)(X₂)(M')(X₃)(L)]X⁻

wherein
- M and M' are each a metal independently selected from the group consisting of ruthenium, osmium and iron;
- SB⁺ is a protonated Schiff base ligand;
- L_{c} is a carbene ligand, preferably selected from the group consisting of alkylidene ligands, vinylidene ligands, indenylidene ligands and allenylidene ligands;
- L is a non-anionic ligand,
- X₁, X₂ and X₃ are each independently selected from anionic ligands; and
- X⁻ is an anion,
including salts, solvates and enantiomers thereof.

When starting from a multi-coordinated Schiff-base-substituted monometallic complex, such new species or products may for instance take the form of one or more cationic monometallic species being represented by the general formula (VI): 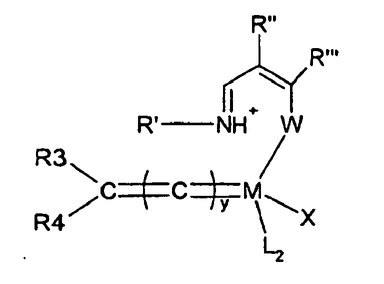 or one or more cationic monometallic species being represented by the general formula (VII): 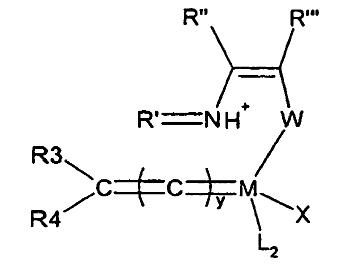 wherein
- M is a metal selected from the group consisting of ruthenium, osmium and iron;
- W is selected from the group consisting of oxygen, sulphur, selenium, NR"", PR"", AsR"" and SbR"";
- R", R"' and R"" are each a radical independently selected from the group consisting of hydrogen, C₁₋₆ alkyl, C₃₋₈ cycloalkyl, C₁₋₆ alkyl-C₁₋₆ alkoxysilyl, C₁₋₆ alkyl-aryloxysilyl, C₁₋₆ alkyl-C₃₋₁₀ cycloalkoxysilyl, aryl and heteroaryl, or R" and R"' together form an aryl or heteroaryl radical, each said radical (when different from hydrogen) being optionally substituted with one or more, preferably 1 to 3, substituents R₅ each independently selected from the group consisting of halogen atoms. C₁₋₆ alkyl, C₁₋₆ alkoxy, aryl, alkylsulfonate, arylsulfonate, alkylphosphonate, arylphosphonate, C₁₋₆ alkyl-C₁₋₆ alkoxysilyl, C₁₋₆ alkyl-aryloxysilyl, C₁₋₆ alkyl-C₃₋₁₀ cycloalkoxysilyl, alkylammonium and arylammonium;
- R' is either as defined for R", R"' and R"" when included in a compound having the general formula (VI) or, when included in a compound having the general formula (VII), is selected from the group consisting of C₁₋₆ alkylene and C₃₋₈ cycloalkylene, the said alkylene or cycloalkylene group being optionally substituted with one or more substituents R₅;
- L₂ is a non-anionic ligand;
- X is an anionic ligand;
- R₃ and R₄ are each hydrogen or a radical selected from the group consisting of C₁₋₂₀ alkyl, C₂₋₂₀ alkenyl, C₂₋₂₀ alkynyl, C₁₋₂₀ carboxylate, C₁₋₂₀ alkoxy, C₂₋₂₀ alkenyloxy, C₂₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium and arylammonium;
- R' and one of R₃ and R₄ may be bonded to each other to form a bidentate ligand;
- R'" and R"" may be bonded to each other to form an aliphatic ring system including a heteroatom selected from the group consisting of nitrogen, phosphorous, arsenic and antimony;
- R₃ and R₄ together may form a fused aromatic ring system, and
- y represents the number of sp₂ carbon atoms between M and the carbon atom bearing R₃ and R₄ and is an integer from 0 to 3 inclusive,
salts, solvates and enantiomers thereof, each of said cationic species being represented by the general formulae (VI) and (VII) being associated with an anion derived from the acid used in the acid activation reaction.

When starting from a multi-coordinated Schiff-base-substituted bimetallic complex, such new species or products may for instance take the form of one or more cationic bimetallic species being represented by the general formula (X): 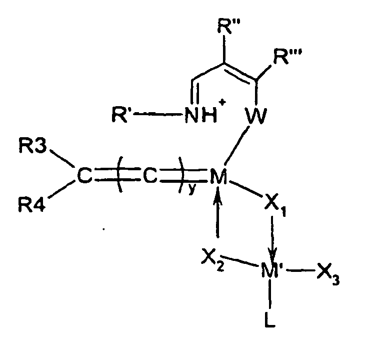 or one or more cationic bimetallic species being represented by the general formula (XI): 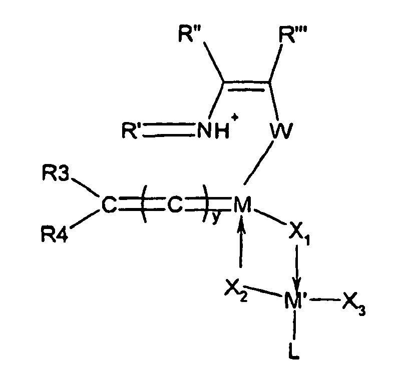 wherein
- M and M' are each a metal independently selected from the group consisting of ruthenium, osmium and iron;
- W, R', R", R"', R"", y, R₃ and R₄ are as defined in formulae (VI) and (VII) hereinabove;
- X₁, X₂ and X₃ are each independently selected from anionic ligands; and
- L is a non-anionic ligand,
including salts, solvates and enantiomers thereof.

The new species or products of this invention may also take the form of one or more monometallic complexes being represented by the general formula (VIII): 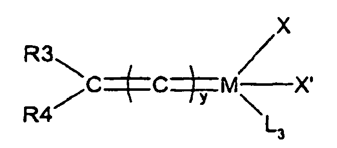 wherein
- M, X, y, R₃ and R₄ are as defined in formulae (VI) and (VII);
- X' is a anionic ligand; and
- L₃ is a non-anionic ligand,
including salts, solvates and enantiomers thereof.

The new species or products of this invention may also take the form of one or more metal monohydride complexes being represented by the general formula (IX): 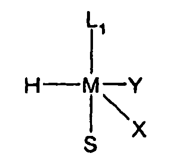 wherein:
- M and X are as defined in formulae (VI) and (VII);
- S is a solvent, e.g. water;
- Y is a solvent or Y is CO when S is an alcohol; and
- L₁ is a non-anionic ligand,
including salts, solvates and enantiomers thereof.

The new catalytic species of the invention may be produced extra-temporaneously, separated, purified and conditioned for separate use in organic synthesis reactions later on, or they may be produced *in situ* during the relevant chemical reaction (e.g. metathesis) by introducing the acid into the reaction mixture before, simultaneously with, or alternatively after the introduction of the starting Schiff base metal complex. The present invention also provides catalytic systems including, in addition to said new catalytic species or reaction products, a second catalyst component (such as a Lewis acid co-catalyst, or an initiator having a radically transferable atom or group, or a radical initiator, or a bimetallic metal complex) and/or a carrier suitable for supporting said catalytic species or reaction products.

The present invention also provides the use of such new catalytic species or reaction products, or any mixture of such species, or such catalytic systems, in a wide range of organic synthesis reactions such as olefin metathesis reactions, acetylene metathesis reactions and certain reactions involving the transfer of an atom or group to an ethylenically or acetylenically unsaturated compound or another reactive substrate, such as atom transfer radical polymerisation, atom transfer radical addition, vinylation, cyclopropanation of ethylenically unsaturated compounds, and the like. In particular, this invention provides an improved process for the ring opening polymerization of strained cyclic olefins such as, but not limited to, dicyclopentadiene.

### BRIEF DESCRIPTION OF THE DRAWINGS

Figure 1 shows bidentate Schiff base ligands having the general formulae (I A) and (I B) that may be included in multicoordinated metal complexes suitable for modification with an acid according to an embodiment of the present invention.
Figure 2 shows tetradentate Schiff base ligands having the general formulae (II A) and (II B) that may be included in multicoordinated metal complexes suitable for modification with an acid according to another embodiment of the present invention.
Figure 3 shows tetradentate Schiff base ligands having the general chemical formulae (III A) and (III B) that may be included in multicoordinated metal complexes suitable for modification with an acid according to this invention.
Figure 4 shows tridentate Schiff base ligands having the general chemical formulae (IV D) that may be included in multicoordinated metal complexes suitable for modification with an acid according to the present invention, as well as bimetallic complexes having the general formulae (IV A) and (IV B) suitable for modification with an acid according to the present invention, and a fused aromatic ring system having the formula (IV C) that may present in a carbene ligand of such metal complexes.
Figure 5 shows the scheme of manufacture of a multicoordinated metal complex to be modified by an acid according to this invention.

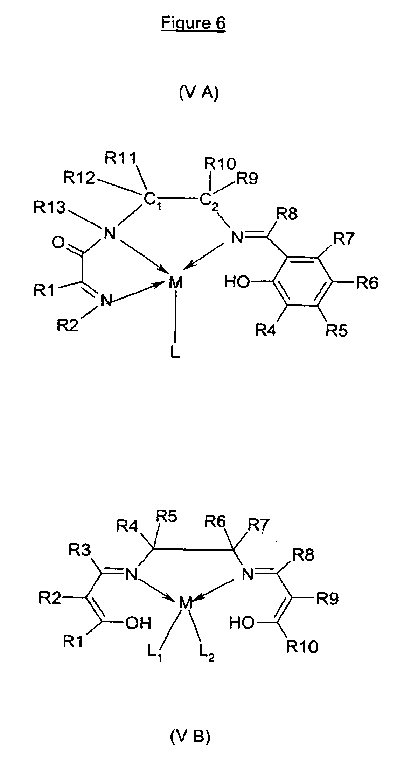

Figure 6 shows monometallic complexes having the general formula (VA), derived from a tetradentate Schiff base ligand (IIIA), and the general formula (VB) suitable for modification with an acid according to the present invention
Figure 7 shows the ¹H NMR spectrum, in deuterated chloroform, of a first Schiff-base-substituted ruthenium complex (example 12) before acid activation.
Figure 8 shows the ¹H NMR spectrum, in deuterated chloroform, of the product resulting from 5 minutes of acid activation of the same first Schiff-base-substituted ruthenium complex.
Figure 9 shows the ¹H NMR spectrum, in deuterated chloroform, of the product resulting from 50 minutes of acid activation of the same first Schiff-base-substituted ruthenium complex.
Figure 10 shows the ¹H NMR spectrum, in deuterated chloroform, of the product resulting from 90 minutes of acid activation of the same first Schiff-base-substituted ruthenium complex.
Figure 11 shows the ¹H NMR spectrum, in deuterated chloroform, of the product resulting from 24 hours of acid activation of the same first Schiff-base-substituted ruthenium complex.
Figure 12 shows the ¹H NMR spectrum, in deuterated chloroform, of the product resulting from 91 hours of acid activation of the same first Schiff-base-substituted ruthenium complex.
Figure 13 shows the ¹H NMR spectrum, in deuterated chloroform, of the mixture resulting from 90 minutes of acid activation of the same first Schiff-base-substituted ruthenium complex followed by cyclooctene addition and 30 minutes polymerization.
Figure 14 shows the ¹H NMR spectrum, in deuterated chloroform, of a second Schiff-base-substituted ruthenium complex (example 43) before acid activation.
Figure 15 shows the ¹H NMR spectrum, in deuterated chloroform, of the product resulting from 10 minutes of acid activation of the second Schiff-base-substituted ruthenium complex.

### DEFINITIONS

As used herein, the term complex, or coordination compound, refers to the result of a donor-acceptor mechanism or Lewis acid-base reaction between a metal (the acceptor) and several neutral molecules or ionic compounds called ligands, each containing a non-metallic atom or ion (the donor). Ligands that have more than one atom with lone pairs of electrons (i.e. more than one point of attachment to the metal center) and therefore occupy more than one coordination site are called multidentate ligands. The latter, depending upon the number of coordination sites occupied, include bidentate, tridentate and tetradentate ligands.

As used herein, the term " monometallic " refers to a complex in which there is a single metal center. As used herein, the term " heterobimetallic " refers to a complex in which there are two different metal centers. As used herein, the term " homobimetallic " refers to a complex having two identical metal centers, which however need not have identical ligands or coordination number.

As used herein with respect to a substituting radical, ligand or group, the term "C₁₋₇ alkyl" means straight and branched chain saturated acyclic hydrocarbon monovalent radicals having from 1 to 7 carbon atoms such as, for example, methyl, ethyl, propyl, n-butyl, 1-methylethyl (isopropyl), 2-methylpropyl (isobutyl), 1,1-dimethylethyl (ter-butyl), 2-methylbutyl, n-pentyl, dimethylpropyl, n-hexyl, 2-methylpentyl, 3-methylpentyl, n-heptyl and the like; optionally the carbon chain length of such group may be extended to 20 carbon atoms.

As used herein with respect to a linking group, the term " C₁₋₇ alkylene " means the divalent hydrocarbon radical corresponding to the above defined C₁₋₇ alkyl, such as methylene, bis(methylene), tris(methylene), tetramethylene, hexamethylene and the like.

As used herein with respect to a substituting radical, ligand or group, the term " C₃₋₁₀ cycloalkyl" mean a mono- or polycyclic saturated hydrocarbon monovalent radical having from 3 to 10 carbon atoms, such as for instance cyclopropyl, cyclobutyl, cyclopentyl, cyclohexyl, cycloheptyl, cyclooctyl and the like, or a C₇₋₁₀ polycyclic saturated hydrocarbon monovalent radical having from 7 to 10 carbon atoms such as, for instance, norbornyl, fenchyl, trimethyltricycloheptyl or adamantyl.

As used herein with respect to a linking group, and unless otherwise stated, the term " C₃₋₁₀ cycloalkylene" means the divalent hydrocarbon radical corresponding to the above defined C₃₋₁₀ cycloalkyl, such as 1,2-cyclohexylene and 1,4-cyclohexylene.

As used herein with respect to a substituting radical, ligand or group, and unless otherwise stated, the term " aryl " designates any mono- or polycyclic aromatic monovalent hydrocarbon radical having from 6 up to 30 carbon atoms such as but not limited to phenyl, naphthyl, anthracenyl, phenantracyl, fluoranthenyl, chrysenyl, pyrenyl, biphenylyl, terphenyl, picenyl, indenyl, biphenyl, indacenyl, benzocyclobutenyl, benzocyclooctenyl and the like, including fused benzo-C₄₋₈ cycloalkyl radicals (the latter being as defined above) such as, for instance, indanyl, tetrahydronaphtyl, fluorenyl and the like, all of the said radicals being optionally substituted with one or more substituents selected from the group consisting of halogen, amino, nitro, hydroxyl, sulfhydryl and nitro, such as for instance 4-fluorophenyl, 4-chlorophenyl, 3,4-dichlorophenyl, 2,6-diisopropyl-4-bromophenyl, pentafluorophenyl and 4-cyanophenyl.

As used herein with respect to a linking group, and unless otherwise stated, the term "arylene" means the divalent hydrocarbon radical corresponding to the above defined aryl, such as phenylene, naphtylene and the like.

As used herein with respect to a combination of two substituting hydrocarbon radicals, and unless otherwise stated, the term " homocyclic" means a mono- or polycyclic, saturated or mono-unsaturated or polyunsaturated hydrocarbon radical having from 4 up to 15 carbon atoms but including no heteroatom in the said ring; for instance the said combination forms a C₂₋₆ alkylene radical, such as tetramethylene, which cyclizes with the carbon atoms to which the said two substituting hydrocarbon radicals are attached.

As used herein with respect to a substituting radical (including the combination of two substituting radicals), ligand or group, and unless otherwise stated, the term "heterocyclic" means a mono- or polycyclic, saturated or mono-unsaturated or polyunsaturated monovalent hydrocarbon radical having from 2 up to 15 carbon atoms and including one or more heteroatoms in one or more heterocyclic rings, each of said rings having from 3 to 10 atoms (and optionally further including one or more heteroatoms attached to one or more carbon atoms of said ring, for instance in the form of a carbonyl or thiocarbonyl or selenocarbonyl group, and/or to one or more heteroatoms of said ring, for instance in the form of a sulfone, sulfoxide, N-oxide, phosphate, phosphonate or selenium oxide group), each of said heteroatoms being independently selected from the group consisting of nitrogen, oxygen, sulfur, selenium and phosphorus, also including radicals wherein a heterocyclic ring is fused to one or more aromatic hydrocarbon rings for instance in the form of benzo-fused, dibenzo-fused and naphto-fused heterocyclic radicals; within this definition are included heterocyclic radicals such as, but not limited to, diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzo-thiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, oxabicycloheptyl, azabenzimidazolyl, azacycloheptyl, azacyclooctyl, azacyclononyl, azabicyclononyl, tetrahydrofuryl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thiourazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl (benzothiofuranyl), phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, indolinyl, indolizidinyl, benzofuryl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl, cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl, thietanyl, diazabicyclooctyl, diazetyl, diaziridinonyl, diaziridinethionyl, chromanyl, chromanonyl, thiochromanyl, thiochromanonyl, thiochromenyl, benzofuranyl, benzisothiazolyl, benzocarbazolyl, benzochromonyl, benzisoalloxazinyl, benzocoumarinyl, thiocoumarinyl, phenometoxazinyl, phenoparoxazinyl, phentriazinyl, thiodiazinyl, thiodiazolyl, indoxyl, thioindoxyl, benzodiazinyl (e.g. phtalazinyl), phtalidyl, phtalimidinyl, phtalazonyl, alloxazinyl, dibenzopyronyl (i.e. xanthonyl), xanthionyl, isatyl, isopyrazolyl, isopyrazolonyl, urazolyl, urazinyl, uretinyl, uretidinyl, succinyl, succinimido, benzylsultimyl, benzylsultamyl and the like, including all possible isomeric forms thereof, wherein each carbon atom of said heterocyclic ring may be independently substituted with a substituent selected from the group consisting of halogen, nitro, C₁₋₇ alkyl (optionally containing one or more functions or radicals selected from the group consisting of carbonyl (oxo), alcohol (hydroxyl), ether (alkoxy), acetal, amino, imino, oximino, alkyloximino, amino-acid, cyano, carboxylic acid ester or amide, nitro, thio C₁₋₇ alkyl, thio C₃₋₁₀ cycloalkyl, C₁₋₇ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino, phenylhydrazino, sulfonyl, sulfonamido and halogen), C₂₋₇ alkenyl, C₂₋₇ alkynyl, halo C₁₋₇ alkyl, C₃₋₁₀ cycloalkyl, aryl, arylalkyl, alkylaryl, alkylacyl, arylacyl, hydroxyl, amino, C₁₋₇ alkylamino, cycloalkylamino, alkenylamino, cyclo-alkenylamino, alkynylamino, arylamino, arylalkylamino, hydroxyalkylamino, mercaptoalkylamino, heterocyclic amino, hydrazino, alkylhydrazino, phenylhydrazino, sulfhydryl, C₁₋₇ alkoxy, C₃₋₁₀ cycloalkoxy, aryloxy, arylalkyloxy, oxyheterocyclic, heterocyclic-substituted alkyloxy, thio C₁₋₇ alkyl, thio C₃₋₁₀ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof; depending upon the number of unsaturations in the 3 to 10 membered ring, heterocyclic radicals may be sub-divided into heteroaromatic (or " heteroaryl") radicals and non-aromatic heterocyclic radicals; when a heteroatom of the said non-aromatic heterocyclic radical is nitrogen, the latter may be substituted with a substituent selected from the group consisting of C₁₋₇ alkyl, C₃₋₁₀ cycloalkyl, aryl, arylalkyl and alkylaryl.

As used herein with respect to a substituting radical, ligand or group, and unless otherwise stated, the terms " C₁₋₇ alkoxy ", " C₂₋₇ alkenyloxy ", " C₂₋₇ alkynyloxy ", "C₃₋₁₀ cycloalkoxy ", " aryloxy", "arylalkyloxy ", " oxyheterocyclic ", "thio C₁₋₇ alkyl", " thio C₃₋₁₀ cycloalkyl", " arylthio ", " arylalkylthio " and " thioheterocyclic" refer to substituents wherein a C₁₋₇ alkyl, C₂₋₇ alkenyl or C₂₋₇ alkynyl (optionally the carbon chain length of such groups may be extended to 20 carbon atoms), respectively a C₃₋₁₀ cycloalkyl, aryl, arylalkyl or heterocyclic radical (each of them such as defined herein), are attached to an oxygen atom or a divalent sulfur atom through a single bond, such as but not limited to methoxy, ethoxy, propoxy, butoxy, pentoxy, isopropoxy, sec-butoxy, tert-butoxy, isopentoxy, cyclopropyloxy, cyclobutyloxy, cyclopentyloxy, thiomethyl, thioethyl, thiopropyl, thiobutyl, thiopentyl, thiocyclopropyl, thiocyclobutyl, thiocyclopentyl, thiophenyl, phenyloxy, benzyloxy, mercaptobenzyl, cresoxy and the like.

As used herein with respect to a substituting atom or ligand, the term halogen means any atom selected from the group consisting of fluorine, chlorine, bromine and iodine.

As used herein with respect to a substituting radical or group, and unless otherwise stated, the term " halo C₁₋₇ alkyl " means a C₁₋₇ alkyl radical (such as above defined, i.e. optionally the carbon chain length of such group may be extended to 20 carbon atoms) in which one or more hydrogen atoms are independently replaced by one or more halogens (preferably fluorine, chlorine or bromine), such as but not limited to fluoromethyl, difluoromethyl, trifluoromethyl, chloromethyl, dichloromethyl, trichloromethyl, 2,2,2-trifluoroethyl, 2-fluoroethyl, 2-chloroethyl, 2,2,2-trichloroethyl, octafluoropentyl, dodecafluoroheptyl, dichloromethyl and the like.

As used herein with respect to a substituting radical, ligand or group, and unless otherwise stated, the term " C₂₋₇ alkenyl " means a straight or branched acyclic hydrocarbon monovalent radical having one or more ethylenical unsaturations and having from 2 to 7 carbon atoms such as, for example, vinyl, 1-propenyl, 2-propenyl (allyl), 1-butenyl, 2-butenyl, 3-butenyl, 2-pentenyl, 3-pentenyl, 3-methyl-2-butenyl, 3-hexenyl, 2-hexenyl, 2-heptenyl, 1,3-butadienyl, n-penta-2,4-dienyl, hexadienyl, heptadienyl, heptatrienyl and the like, including all possible isomers thereof; optionally the carbon chain length of such group may be extended to 20 carbon atoms (such as n-oct-2-enyl, n-dodec-2-enyl, isododecenyl, n-octadec-2-enyl and n-octadec-4-enyl).

As used herein with respect to a substituting radical, ligand or group, and unless otherwise stated, the term " C₃₋₁₀ cycloalkenyl " means a monocyclic mono- or polyunsaturated hydrocarbon monovalent radical having from 3 to 8 carbon atoms, such as for instance cyclopropenyl, cyclobutenyl, cyclopentenyl, cyclopentadienyl, cyclohexenyl, cyclohexadienyl, cycloheptenyl, cycloheptadienyl, cycloheptatrienyl, cyclooctenyl, cyclooctadienyl, cyclooctatrienyl, 1,3,5,7-cyclooctatetraenyl and the like, or a C₇₋₁₀ polycyclic mono- or polyunsaturated hydrocarbon monovalent radical having from 7 to 10 carbon atoms such as dicyclopentadienyl, fenchenyl (including all isomers thereof, such as α-pinolenyl), bicyclo[2.2.1]hept-2-enyl (norbornenyl), bicyclo[2.2.1]hepta-2,5-dienyl (norbornadienyl), cyclofenchenyl and the like.

As used herein with respect to a substituting radical, ligand or group, the term "C₂₋₇ alkynyl " defines straight and branched chain hydrocarbon radicals containing one or more triple bonds (i.e. acetylenic unsaturation) and optionally at least one double bond and having from 2 to 7 carbon atoms such as, for example, acetylenyl, 1-propynyl, 2-propynyl, 1-butynyl, 2-butynyl, 2-pentynyl, 1-pentynyl, 3-methyl-2-butynyl, 3-hexynyl, 2-hexynyl, 1-penten-4-ynyl, 3-penten-1-ynyl, 1,3-hexadien-1-ynyl and the like, including all possible isomers thereof; optionally the carbon chain length of such group may be extended to 20 carbon atoms.

As used herein, and unless otherwise stated, the terms "arylalkyl", "arylalkenyl" and "heterocyclic-substituted alkyl" refer to an aliphatic saturated or unsaturated hydrocarbon monovalent radical (preferably a C₁₋₇ alkyl or C₂₋₇ alkenyl radical such as defined above, i.e. optionally the carbon chain length of such group may be extended to 20 carbon atoms) onto which an aryl or heterocyclic radical (such as defined above) is already bonded, and wherein the said aliphatic radical and/or the said aryl or heterocyclic radical may be optionally substituted with one or more substituents selected from the group consisting of halogen, amino, nitro, hydroxyl, sulfhydryl and nitro, such as but not limited to benzyl, 4-chlorobenzyl, phenylethyl, 3-phenylpropyl, α-methylbenzyl, phenbutyl, α,α-dimethylbenzyl, 1-amino-2-phenylethyl, 1-amino-2-[4-hydroxyphenyl]ethyl, 1-amino-2-[indol-2-yl]ethyl, styryl, pyridylmethyl, pyridylethyl, 2-(2-pyridyl)isopropyl, oxazolylbutyl, 2-thienylmethyl and 2-furylmethyl.

As used herein, and unless otherwise stated, the terms " alkylcycloalkyl ", "alkenyl(hetero)aryl ", "alkyl(hetero)aryl " , and "alkyl-substituted heterocyclic " refer respectively to an aryl, heteroaryl, cycloalkyl or heterocyclic radical (such as defined above) onto which are already bonded one or more aliphatic saturated or unsaturated hydrocarbon monovalent radicals, preferably one or more C₁₋₇ alkyl, C₂₋₇ alkenyl or C₃₋₁₀ cycloalkyl radicals as defined above, such as, but not limited to, o-toluyl, m-toluyl, p-toluyl, 2,3-xylyl, 2,4-xylyl, 3,4-xylyl, o-cumenyl, m-cumenyl, p-cumenyl, o-cymenyl, m-cymenyl, p-cymenyl, mesityl, lutidinyl (i.e. dimethylpyridyl), 2-methylaziridinyl, methylbenzimidazolyl, methylbenzofuranyl, methylbenzothiazolyl, methylbenzo-triazolyl, methylbenzoxazolyl, methylcyclohexyl and menthyl.

As used herein, and unless otherwise stated, the terms " alkylamino ", "cycloalkylamino ", "alkenylamino", "cycloalkenylamino", " arylamino ", "arylalkylamino", "heterocyclic amino " , " hydroxyalkylamino ", "mercaptoalkylamino " and "alkynylamino" mean that respectively one (thus monosubstituted amino) or even two (thus disubstituted amino) C₁₋₇ alkyl, C₃₋₁₀ cycloalkyl, C₂₋₇ alkenyl, C₃₋₁₀ cycloalkenyl, aryl, arylalkyl, heterocyclic, mono- or polyhydroxy C₁₋₇ alkyl, mono- or polymercapto C₁₋₇ alkyl or C₂₋₇ alkynyl radical(s) (each of them as defined herein, respectively) is/are attached to a nitrogen atom through a single bond or, in the case of heterocyclic, include a nitrogen atom, such as but not limited to, anilino, benzylamino, methylamino, dimethylamino, ethylamino, diethylamino, isopropylamino, propenylamino, n-butylamino, ter-butylamino, dibutylamino, morpholinoalkylamino, morpholinyl, piperidinyl, piperazinyl, hydroxymethylamino, β-hydroxyethylamino and ethynylamino; this definition also includes mixed disubstituted amino radicals wherein the nitrogen atom is attached to two such radicals belonging to two different sub-set of radicals, e.g. an alkyl radical and an alkenyl radical, or to two different radicals within the same sub-set of radicals, e.g. methylethylamino; among disubstituted amino radicals, symetrically substituted are usually preferred and more easily accessible.

As used herein, and unless otherwise stated, the terms "(thio)carboxylic acid (thio)ester " and " (thio)carboxylic acid (thio)amide " refer to substituents wherein the carboxyl or thiocarboxyl group is bonded to the hydrocarbonyl residue of an alcohol, a thiol, a polyol, a phenol, a thiophenol, a primary or secondary amine, a polyamine, an amino-alcohol or ammonia, the said hydrocarbonyl residue being selected from the group consisting of C₁₋₇ alkyl, C₂₋₇ alkenyl, C₂₋₇ alkynyl, C₃₋₁₀ cycloalkyl, C₃₋₁₀ cycloalkenyl, aryl, arylalkyl, alkylaryl, alkylamino, cycloalkylamino, alkenylamino, cycloalkenylamino, arylamino, arylalkylamino, heterocyclic amino, hydroxyalkylamino, mercapto-alkylamino or alkynylamino (each such as above defined, respectively).

As used herein with respect to a metal ligand, the terms alkylammonium and arylammonium mean a tetra-coordinated nitrogen atom being linked to one or more C₁₋₇ alkyl, C₃₋₁₀ cycloalkyl, aryl or heteroaryl groups, each such as above defined, respectively.

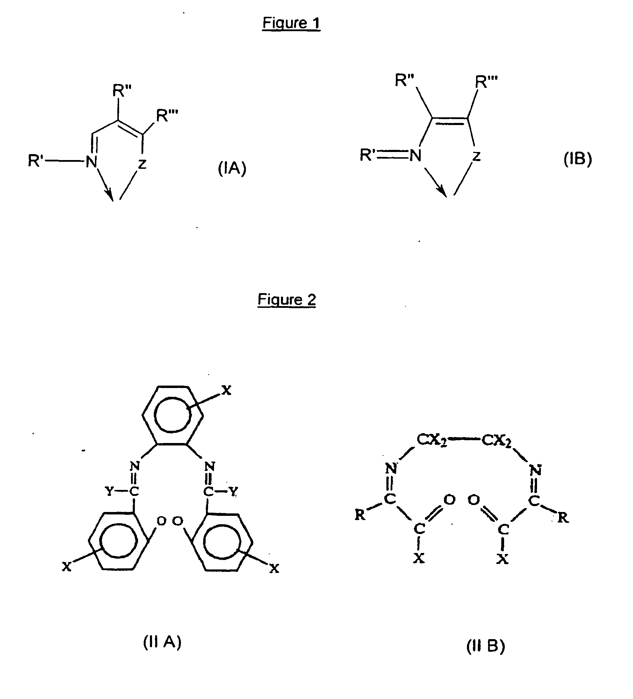

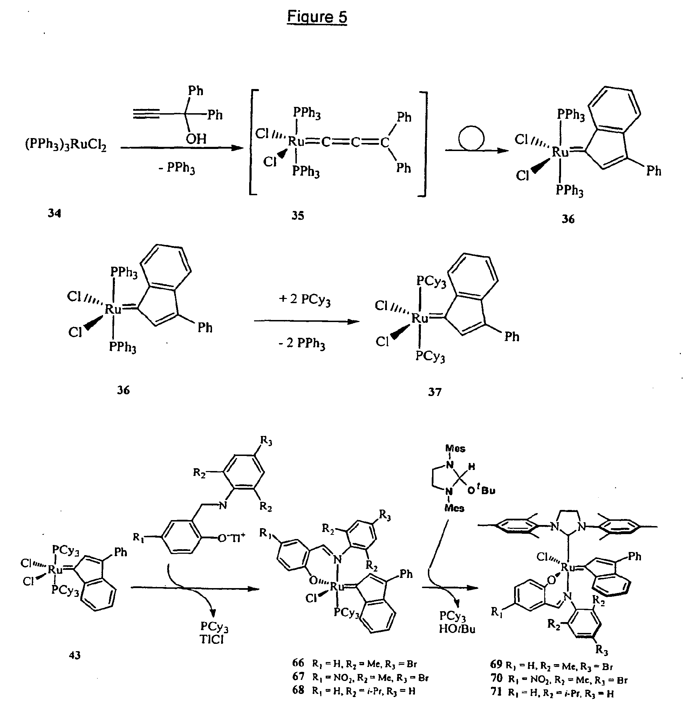

As used herein with respect to a metal ligand, and unless otherwise stated, the term " Schiff base " conventionally refers to the presence of an imino group (usually resulting from the reaction of a primary amine with an aldehyde or a ketone) in the said ligand, being part of a multidentate ligand (such as defined for instance in http://www.ilpi.com/organomet/coordnum.html) and which is coordinated to the metal, in addition to the nitrogen atom of said imino group, through at least one further heteroatom selected from the group consisting of oxygen, sulfur and selenium. The said multidentate ligand may be for instance:
- a N,O-bidentate Schiff base ligand such as a lumazine or substituted lumazine or 2-(2-hydroxyphenyl)benzoxazole or (2'-hydroxyphenyl)-2-thiazoline, or
- a N,S-bidentate Schiff base ligand such as a thiolumazine or substituted thiolumazine, or
- a N,Z-bidentate Schiff base ligand such as shown in figure 1, wherein Z is or includes an atom selected from the group consisting of oxygen, sulfur and selenium; it may be advantageous for the said bidentate Schiff base ligand to further include a carbon-carbon double bond conjugated with the carbon-nitrogen double bond of the imino group, for instance as shown in figure 1, or
- a N,N,O- tridentate Schiff base ligand such as derived from 6-amino-5-formyl-1,3-dimethyluracil and semicarbazide or acetylhydrazine or benzoylhydrazine, or such as derived from 7-formyl-8-hydroxyquinoline(oxine) and 2-aminophenol or 2-aminopyridine, or
- a O,N,O-tridentate Schiff base ligand such as 6-amino-5-formyl-1,3-dimethyluracilbenzoyl-hydrazone or such as shown in formula (IV) of figure 5 or *N*-(2-methoxyphenyl) salicylideneamine or salicylaldehyde-2-hydroxanil or the heterocyclic Schiff base resulting from the reaction of 1-amino-5-benzoyl-4-phenyl-1 H pyrimidin-2-one with 2-hydroxynaphtaldehyde or the thenoyltrifluoroaceto antipyrine Schiff base resulting from the reaction of thenoyl-trifluoroacetone with 4-aminoantipyrine, or
- a O,N,S-tridentate Schiff base ligand such as salicylaldehyde-2-mercaptoanil, S-benzyl-2-[(2-hydroxyphenyl)methylene]dithiocarbazate or 2-[(2-hydroxyphenyl)methylene]-N-phenylhydrazi-necarbothioamide, or
- a N,N,S-tridentate Schiff base ligand such as 6-amino-5-formyl-1,3-dimethyluracilthiosemicarbazonate.
By extension, the multidentate ligand may include more than one Schiff base, for instance two imino groups as shown in formulae (IIA) and (IIB) of figure 2 and in formula (IIIA) of figure 3, thus possibly resulting in O,N,N,O-tetradentate or O,N,N,N-tetradentate Schiff base ligands.

As used herein, the terms " constraint steric hindrance " relates to a group or ligand, usually a branched or substituted group or ligand, which is constrained in its movements, i.e. a group the size of which produces a molecular distortion (either an angular distortion or a lengthening of bonds) being measurable by X-ray diffraction.

As used herein and unless otherwise stated, the term " stereoisomer " refers to all possible different isomeric as well as conformational forms which the compounds of the invention may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

As used herein and unless otherwise stated, the term " solvate " includes any combination which may be formed by a compound of this invention with a suitable inorganic solvent (e.g. hydrates formed with water) or organic solvent, such as but not limited to alcohols (in particular ethanol and isopropanol), ketones (in particular methylethylketone and methylisobutylketone), esters (in particular ethyl acetate) and the like.

### DETAILED DESCRIPTION OF THE INVENTION

In its broadest acceptation, the present invention first relates to a method of modifying a multi-coordinated metal complex, a salt, a solvate or an enantiomer thereof according to claim 1.

Preferably, said conditions include one or more of the following:
a molar ratio between said acid and said multi-coordinated metal complex being above about 1.2, preferably above about 2, more preferably above about 3, and most preferably above about 5;
- a molar ratio between said acid and said multi-coordinated metal complex being not above about 40, preferably not above about 30, more preferably not above about 20, and most preferably not above about 15;
- a contact time above 5 seconds, preferably above 30 seconds, more preferably at least 1 minute, for example at least 10 minutes;
- a contact time below 100 hours, preferably not above 24 hours, more preferably not above 4 hours, and most preferably not above 90 minutes;
- a contact temperature from about -50 °C to about 80 °C, preferably from about 10 °C to about 60 °C, more preferably from about 20 °C to about 50 °C.

It should be understood that any combination of the above reaction conditions is contemplated as being within the framework of the present invention, and that the more suitable conditions depend upon the acid used and upon the set of ligands around the metal center, especially upon the Schiff base ligand, but can easily be determined by the skilled person based on the information contained therein.

In a specific embodiment, the method of the invention comprises the additional step of determining (e.g. measuring) the pKa of said at least one multidentate Schiff base ligand (i), and selecting said acid in such a manner that the pKa of said acid is lower than the pKa of said multidentate Schiff base ligand (ii) as determined (e.g. measured in said measuring step) previously.

For the performance of the method of the invention, it is suitable when one of the following situations occurs:
- at least one of said other ligands (ii) is a constraint steric hindrance ligand having a pKa of at least 15,
- the number of carbon atoms in said at least one multidentate Schiff base ligand (i), between the nitrogen atom of said imino group and said coordinating heteroatom of said at least one multidentate Schiff base ligand (i), is 2 or 3,
- the nitrogen atom of the imino group of the multidentate Schiff base ligand (i) is substituted with a group having substantial steric hindrance such as substituted phenyl or, preferably, C₃₋₁₀ cycloalkyl such as adamantyl,
- at least one of said other ligands (ii) is an anionic ligand,
- at least one of said other ligands (ii) is a non-anionic ligand, e.g. one other than a carbene ligand,
- the acid is a strong inorganic acid such as, but not limited to, chlorhydric acid, bromhydric acid, sulfuric acid or nitric acid, or a strong organic acid such as, but not limited to, p-toluenesulfonic acid.

It should be understood that any combination of the above conditions is contemplated as being within the framework of the present invention, and that the more suitable conditions can easily be determined by the skilled person based on the information contained therein.

Secondly, the present invention relates to a reaction product according to claim 12.

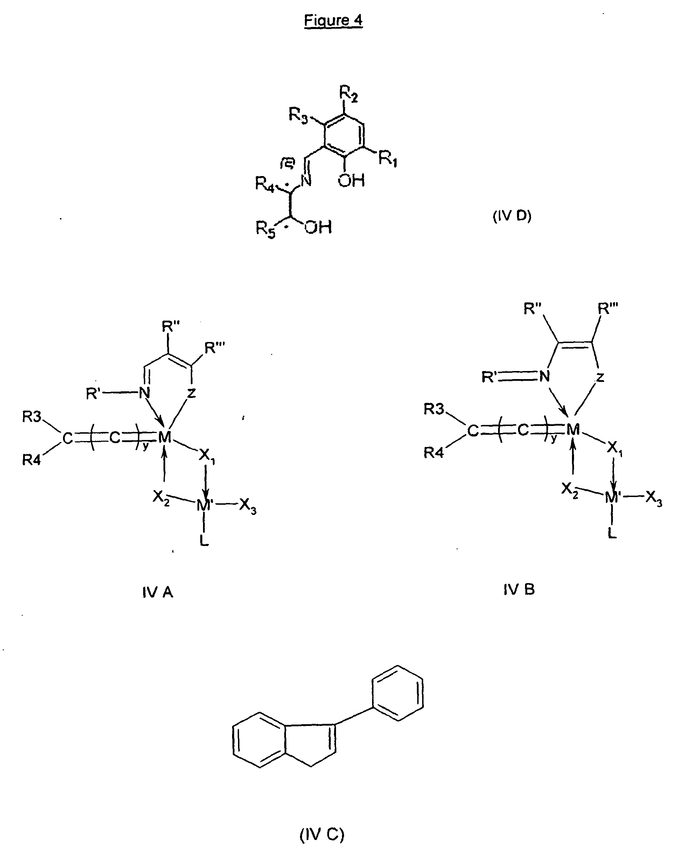

For a more detailed definition of the reaction product of the invention, it is preferred when one of the following situations occurs:
- the pKa of said acid (b) is lower than the pKa of said at least one multidentate Schiff base ligand (i),
- the number of carbon atoms in said at least one multidentate Schiff base ligand (i), between the nitrogen atom of said imino group and said heteroatom of said at least one multidentate Schiff base ligand (i), is 2 or 3,
- at least one of said other ligands (ii) of said multi-coordinated metal complex (a) is a constraint steric hindrance ligand having a pKa of at least 15,
- the nitrogen atom of the imino group of the multidentate Schiff base ligand (i) is substituted with a group having substantial steric hindrance such as substituted phenyl or, preferably, C₃₋₁₀ cycloalkyl such as adamantyl,
- at least one of said other ligands (ii) of said multi-coordinated metal complex (a) is an anionic ligand,
- at least one of said other ligands (ii) of said multi-coordinated metal complex (a) is a non-anionic ligand, e.g. one other than a carbene ligand.
- at least one of said other ligands (ii) of said multi-coordinated metal complex (a) is a solvent S and said complex (a) is a cationic species associated with an anion A,
- said multi-coordinated metal complex (a) is a bimetallic complex (the two metals being the same or being different), in which case preferably (1) one metal of said bimetallic complex is penta-coordinated with said at least one multidentate Schiff base ligand (i) and with said one or more other ligands (ii), and the other metal is tetra-coordinated with one or more neutral ligands and one or more anionic ligands, or (2) each metal of said bimetallic complex is hexa-coordinated with said at least one multidentate Schiff base ligand (i) and with said one or more other ligands (ii);
- said multi-coordinated metal complex (a) is a monometallic complex,
- said multi-coordinated metal complex (a) is a penta-coordinated metal complex or a tetra-coordinated metal complex, for instance wherein (1) said at least one multidentate Schiff base ligand (i) is a bidentate ligand and said multi-coordinated metal complex (a) comprises two other ligands (ii), or wherein (2) said at least one multidentate Schiff base ligand (i) is a tridentate ligand and said multi-coordinated metal complex (a) comprises a single other ligand (ii);
- said at least one multidentate Schiff base ligand (i) has one of the general formulae (IA) and (IB) referred to in figure 1, wherein:
   - Z is selected from the group consisting of oxygen, sulfur and selenium:
   - R" and R"' are each a radical independently selected from the group consisting of hydrogen, C₁₋₇ alkyl, C₃₋₁₀ cycloalkyl, C₁₋₆ alkyl-C₁₋₆ alkoxysilyl, C₁₋₈ alkyl-aryloxysilyl, C₁₋₆ alkyl-C₃₋₁₀ cycloalkoxysilyl, aryl and heteroaryl, or R" and R"' together form an aryl or heteroaryl radical, each said radical being optionally substituted with one or more, preferably 1 to 3, substituents R₅ each independently selected from the group consisting of halogen atoms, C₁₋₆ alkyl, C₁₋₆ alkoxy, aryl, alkylsulfonate, arylsulfonate, alkylphosphonate, arylphosphonate, C₁₋₆ alkyl-C₁₋₆ alkoxysilyl, C₁₋₆ alkyl-aryloxysilyl, C₁₋₆ alkyl-C₃₋₁₀ cycloalkoxysilyl, alkylammonium and arylammonium;
   - R' is either as defined for R" and R"' when included in a compound having the general formula (IA) or, when included in a compound having the general formula (IB), is selected from the group consisting of C₁₋₇ alkylene and C₃₋₁₀ cycloalkylene, the said alkylene or cycloalkylene group being optionally substituted with one or more substituents R₅;
- at least one of said other ligands (ii) of said multi-coordinated metal complex (a) is a derivative, wherein one or more hydrogen atoms is substituted with a group providing constraint steric hindrance, of a N-heterocyclic carbene selected from the group consisting of imidazol-2-ylidene, dihydroimidazol-2-ylidene, oxazol-2-ylidene, triazol-5-ylidene, thiazol-2-ylidene, bis(imidazolin-2-ylidene) bis(imidazolidin-2-ylidene), pyrrolyli-dene, pyrazolylidene, dihydropyrrolylidene, pyrrolylidinylidene and benzo-fused derivatives thereof, or a non-ionic prophosphatrane superbase;
- at least one of said other ligands (ii) of said multi-coordinated metal complex (a) is an anionic ligand selected from the group consisting of C₁₋₂₀ alkyl, C₁₋₂₀ alkenyl, C₁₋₂₀ alkynyl, C₁₋₂₀ carboxylate, C₁₋₂₀ alkoxy, C₁₋₂₀ alkenyloxy, C₁₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium, arylammonium, halogen, C₁₋₂₀ alkyldiketonate, aryldiketonate, nitro and cyano;
- at least one of said other ligands (ii) of said multi-coordinated metal complex (a) is a carbene ligand represented by the general formula =[C=]_{y}CR₃R₄, wherein:
   - y is an integer from 0 to 3 inclusive, and
   - R₃ and R₄ are each hydrogen or a hydrocarbon radical selected from the group consisting of C₁₋₂₀ alkyl, C₁₋₂₀ alkenyl, C₁₋₂₀ alkynyl, C₁₋₂₀ carboxylate, C₁₋₂₀ alkoxy, C₁₋₂₀ alkenyloxy, C₁₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium and arylammonium; or R₃ and R₄ together may form a fused aromatic ring system such as, but not limited to, one having the formula (IVC) referred to in figure 4, i.e. such as a phenylindenylidene ligand;
- said at least one multidentate Schiff base ligand (i) is a tetradentate ligand and said multi-coordinated metal complex (a) comprises one or two other ligands (ii) being non-anionic ligands L⁷ selected from the group consisting of aromatic and unsaturated cycloaliphatic groups, preferably aryl, heteroaryl and C₄₋₂₀ cycloalkenyl groups, wherein the said aromatic or unsaturated cycloaliphatic group is optionally substituted with one or more C₁₋₇ alkyl groups or electron-withdrawing groups such as, but not limited to, halogen, nitro, cyano, (thio)carboxylic acid, (thio)carboxylic acid (thio)ester, (thio)carboxylic acid (thio)amide, (thio)carboxylic acid anhydride and (thio) carboxylic acid halide;

In a first aspect, the present invention will now be described with respect to a few preferred embodiments of the multicoordinated metal complex (a) to be modified by reaction with an acid.

A first embodiment of a multicoordinated metal complex (a) suitable for reaction with an acid according to this invention is a five-coordinate metal complex, a salt, a solvate or an enantiomer thereof, such as disclosed in WO 03/062253 i.e. comprising a carbene ligand, a multidentate ligand and one or more other ligands, wherein:
- at least one of said other ligands is a constraint steric hindrance ligand having a pKa of at least 15 (the said pKa being measured under standard conditions, i.e. at about 25°C usually in dimethylsulfoxide (DMSO) or in water depending upon the solubility of the ligand),
- the multidentate ligand is a multidentate Schiff base ligand comprising an imino group and being coordinated to the metal, in addition to the nitrogen atom of said imino group, through at least one further heteroatom selected from the group consisting of oxygen, sulfur and selenium, and
- said other ligands are unable of protonation by said acid under the reaction conditions.

The five-coordinate metal complex of this first embodiment may be either a monometallic complex or a bimetallic complex wherein one metal is penta-coordinated and the other metal is tetra-coordinated with one or more neutral ligands and one or more anionic ligands. In the latter case, the two metals M and M' may be the same or different. Specific examples of such a bimetallic complexes are shown in the general formulae (IVA) and (IVB) referred to in figure 4, wherein:
- Z, R', R" and R'" are as previously defined with respect to formulae (IA) and (IB),
- M and M' are each a metal independently selected from the group consisting of ruthenium, osmium and iron;
- y represents the number of sp₂ carbon atoms between M and the carbon atom bearing R₃ and R₄ and is an integer from 0 to 3 inclusive;
- R₃ and R₄ are each hydrogen or a radical selected from the group consisting of C₁₋₂₀ alkyl, C₂₋₂₀ alkenyl, C₂₋₂₀ alkynyl, C₁₋₂₀ carboxylate, C₁₋₂₀ alkoxy, C₂₋₂₀ alkenyloxy, C₂₋₂₀ alkynyloxy, aryl, aryloxy, C₁₋₂₀ alkoxycarbonyl, C₁₋₆ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ Alkylammonium and arylammonium;
- R' and one of R₃ and R₄ may be bonded to each other to form a bidentate ligand;
- X₁, X₂ and X₃ are anionic ligands as defined below;
- L is a neutral electron donor; and
- R₃ and R₄ together may form a fused aromatic ring system, i.e. a phenylindenylidene ligand,
including salts, solvates and enantiomers thereof.

The multidentate Schiff base ligand included may be either a bidentate Schiff base ligand, in which case the multicoordinated metal complex (a) of this first embodiment comprises two other ligands, or a tridentate Schiff base ligand in which case the metal complex comprises a single other ligand.

The metal in the five-coordinate metal complex of the invention is a transition metal selected from the group consisting of ruthenium, osmium and iron.

The carbene ligand in the five-coordinate metal complex of the invention may be an alkylidene ligand, a benzylidene ligand, a vinylidene ligand, an indenylidene ligand, a phenylindenylidene ligand, an allenylidene ligand or a cumulenylidene ligand, e.g. buta-1,2,3-trienylidene, penta-1,2,3,4-tetraenylidene and the like, i.e. from 1 to 3 sp₂ carbon atoms may be present between the metal M and the group-bearing carbon atom.

In one aspect which is namely useful when the complex is used in the presence of an organic solvent, one of said other ligands present in the five-coordinate metal complex of the invention is an anionic ligand, the meaning of the term anionic ligand being conventional in the art and preferably being within the definition given in U.S.Patent No. 5,977,393, e.g. a ligand preferably but not exclusively selected from the group consisting of C₁₋₂₀ alkyl, C₂₋₂₀ alkenyl, C₂₋₂₀ alkynyl, C₁₋₂₀ carboxylate, C₁₋₂₀ alkoxy, C₂₋₂₀ alkenyloxy, C₂₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium, arylammonium, halogen (preferably chloro), nitro, C₁₋₂₀ alkyl-diketonate (e.g. acetylacetonate), aryldiketonate and cyano.

In another aspect, which is namely useful when the complex is used in the presence of water, one of said other ligands is a solvent and the complex is a cationic species associated with an anion. Suitable anions for the latter purpose are selected from the group consisting of tetrafluoroborate, tetra(pentafluorophenyl)borate, alkylsulfonates wherein the alkyl group may be substituted with one or more halogen atoms, and arylsulfonates. Suitable solvents for coordinating with the metal in such a cationic species may be selected from the group consisting of protic solvents, polar aprotic solvents and non-polar solvents such as aromatic hydrocarbons, chlorinated hydrocarbons, ethers, aliphatic hydrocarbons, alcohols, esters, ketones, amides, and water.

Methods for making five-coordinate metal complexes according to this first embodiment of the invention are already extensively disclosed in WO 03/062253.

A second embodiment of a multicoordinated metal complex (a) suitable for reaction with an acid according to this invention is a four-coordinate monometallic complex comprising a multidentate ligand and one or more other ligands, wherein:
- at least one of said other ligands is a constraint steric hindrance ligand having a pKa of at least 15, or is a group selected from aromatic and unsaturated cycloaliphatic, preferably aryl and C₄₋₂₀ cycloalkenyl (such as cyclooctadienyl, norbornadienyl, cyclopentadienyl and cyclooctatrienyl) groups, the said group being optionally substituted with one or more C₁₋₇ alkyl groups,
- the multidentate ligand is a multidentate Schiff base ligand comprising an imino group and being coordinated to the metal, in addition to the nitrogen atom of said imino group, through at least one further heteroatom selected from the group consisting of oxygen, sulfur and selenium, and
- said other ligands are unable of protonation by said acid under the reaction conditions.

Alike in the first embodiment, one of said other ligands present in the four-coordinate monometallic complex of the second embodiment of the invention may be an anionic ligand such as defined previously.

More specifically, the constraint steric hindrance ligand having a pKa of at least 15 that may be included in both the first embodiment and the second embodiment of the invention may be a derivative, wherein one or more hydrogen atoms is substituted with a group providing constraint steric hindrance, of the following groups:
- imidazol-2-ylidene (pKa = 24),
- dihydroimidazol-2-ylidene (pKa higher than 24),
- oxazol-2-ylidene,
- triazol-5-ylidene,
- thiazol-2-ylidene,
- pyrrolylidene (pKa = 17.5),
- pyrazolylidene,
- dihydropyrrolylidene,
- pyrrolylidinylidene (pKa = 44),
- bis(imidazoline-2-ylidene) and bis(imidazolidine-2-ylidene),
- benzo-fused derivatives such as indolylidene (pKa = 16), and
- non-ionic prophosphatrane superbases, namely as described in U.S. Patent No. 5,698,737, preferably trimethyltriazaprophosphatrane P(CH₃NCH₂CH₂)₃N known as Verkade superbase.

The constraint steric hindrance group may be for instance a branched or substituted group, e.g. a ter-butyl group, a substituted C₃₋₁₀ cycloalkyl group, an aryl group having two or more C₁₋₇ alkyl substituents (such as 2,4,6-trimethylphenyl (mesityl), 2,6-dimethylphenyl, 2,4,6-triisopropylphenyl or 2,6-diisopropylphenyl), or a heteroaryl group (such as pyridinyl) having two or more C₁₋₇ alkyl substituents.

As previously indicated, the multidentate Schiff base ligand included either in the five-coordinate metal complex of the first embodiment or in the four-coordinate monometallic complex of the second embodiment may have one of the general formulae (IA) and (IB) referred to in figure 1, with Z, R', R" and R"' being as defined above. In the definition of the ligands having the general formula (IA), the group R' is preferably selected from methyl, phenyl and substituted phenyl (e.g. dimethylbromophenyl or diisopropylphenyl). In the definition of the ligands having the general formula (IB), the group R' is preferably methylidene or benzylidene.

Methods for making four-coordinate monometallic complexes according to this second embodiment of the invention are already extensively disclosed in WO 03/062253.

A third embodiment of a multicoordinated metal complex (a) suitable for reaction with an acid according to this invention is an at least tetra-coordinated metal complex, a salt, a solvate or an enantiomer thereof, comprising:
- a multidentate Schiff base ligand comprising an imino group and being coordinated to the metal, in addition to the nitrogen atom of said imino group, through at least one further heteroatom selected from the group consisting of oxygen, sulfur and selenium;
- a non-anionic unsaturated ligand L¹ selected from the group consisting of aromatic and unsaturated cycloaliphatic groups, preferably aryl, heteroaryl and C₄₋₂₀ cycloalkenyl groups, the said aromatic or unsaturated cycloaliphatic group being optionally substituted with one or more C₁₋₇ alkyl groups or with electron-withdrawing groups such as, but not limited to, halogen, nitro, cyano, (thio)carboxylic acid, (thio)carboxylic acid (thio)ester, (thio)carboxylic acid (thio)amide, (thio)carboxylic acid anhydride and (thio) carboxylic acid halide; and
- a non-anionic ligand L² selected from the group consisting of C₁₋₇ alkyl, C₃₋₁₀ cycloalkyl, aryl, arylalkyl, alkylaryl and heterocyclic, the said group being optionally substituted with one or more preferably electron-withdrawing substituents such as, but not limited to, halogen, nitro, cyano, (thio)carboxylic acid, (thio)carboxylic acid (thio)ester, (thio)carboxylic acid (thio)amide, (thio)carboxylic acid anhydride and (thio) carboxylic acid halide,
provided that said other ligands L¹ and L² are unable of protonation by said acid under the reaction conditions.

In this third embodiment of the invention, the multidentate ligand is preferably a N,O-bidentate Schiff base ligand or N,S-bidentate Schiff base ligand, most preferably a bidentate Schiff base ligand as shown in formulae (IA) or (IB) in figure 1 and described in more detail hereinabove, in which case the metal complex is tetra-coordinated. The multidentate ligand may also be a tridentate Schiff base, in which case the metal complex is penta-coordinated.

The at least tetra-coordinated metal complex according to this third embodiment of the invention is preferably a monometallic complex. The metal is a transition metal selected from the group consisting of ruthenium, osmium and iron.

Each of the metal, the ligand L¹ and the ligand L² may, independently from each other, be any of the above-mentioned metals or any of the above-mentioned groups with any of the substituents listed for such groups, including any of the individual meanings for such groups or substituents which are listed in the definitions given hereinabove. Preferably the non-anionic ligand L² has constraint steric hindrance such as, but not limited to, tert-butyl, neopentyl and mono- or polysubstituted phenyl, e.g. pentafluorophenyl. L² may also be a linear C₁₋₇ alkyl such as methyl, or an aryl such as phenyl. Preferably the non-anionic unsaturated ligand L¹ also has constraint steric hindrance (such as, but not limited to, alkylaryl and alkylheteroaryl, e.g. xylyl, cumenyl or mesityl).

The at least tetra-coordinated metal complex according to this third embodiment of the invention may for instance, but without limitation, be made according to the following procedure: a metal (e.g. thallium) salt of the multidentate ligand (e.g. the bidentate or tridentate Schiff base) is first reacted with a preferably bimetallic metal complex of the desired metal, more preferably a homobimetallic complex wherein the desired metal is coordinated with a non-anionic unsaturated ligand L¹ and at least one anionic ligand, such as [RuCl₂(p-cymene)]₂, (RuCl₂(COD)]₂ or [RuCl₂(NBD)]₂, wherein COD and NBD respectively mean cyclooctadiene and norbomadiene. After removal of the metal salt formed with the anionic ligand, e.g. thallium chloride, the intermediate complex produced, i.e. a complex wherein the desired metal is coordinated with a non-anionic unsaturated ligand L¹, the multidentate ligand (e.g. the bidentate or tridentate Schiff base) and an anionic ligand, is reacted with a combination of the non-anionic ligand L² and an alcali or alcaline-earth metal, e.g. a C₁₋₇ alkyllithium, a C₁₋₇ alkylsodium, phenyllithium, or a Grignard reagent such as phenylmagnesium chloride, phenylmagnesium bromide or pentafluorophenylmagnesium chloride. Recovery of the desired at least tetra-coordinated metal complex of the third embodiment of the invention may suitably be achieved by removal of the alcali or alcaline-earth metal salt formed with the anionic ligand, followed by purification using conventional techniques. High yields of the pure at least tetra-coordinated metal complex of this embodiment may thus be achieved in a simple two-steps method.

A fourth embodiment of a multicoordinated metal complex (a) suitable for reaction with an acid according to this invention is a hexa-coordinated metal complex, a salt, a solvate or an enantiomer thereof, comprising:
- a multidentate Schiff base ligand comprising an imino group and being coordinated to the metal, in addition to the nitrogen atom of said imino, group, through at least one further heteroatom selected from the group consisting of oxygen, sulfur and selenium;
- at least one non-anionic bidentate ligand L³ being different from the multidentate ligand: and
- at most two anionic ligands L⁴,
provided that said ligands L³ and L⁴ are unable of protonation by said acid under the reaction conditions.

Said hexa-coordinated metal complex is preferably a bimetallic complex wherein each metal is hexa-coordinated. The two metals may be the same or different. Each metal is a transition metal selected from the group consisting of ruthenium, osmium and iron.

The multidentate ligand is preferably defined as in the previous embodiments of the invention, i.e. preferably is a bidentate or tridentate Schiff base. The non-anionic bidentate ligand L³ is preferably a polyunsaturated C₃₋₁₀ cycloalkenyl group such as, but not limited to, norbornadiene, cyclooctadiene, cyclopentadiene, cyclohexadiene, cycloheptadiene or cycloheptatriene, or a heteroaryl group such as defined hereinabove (preferably wherein the heteroatom is not nitrogen, phosphorus, arsenic or antimony in order to avoid a risk of protonation by the acid used for modifying the metal complex), for instance (but without limitation) a 1-hetero-2,4-cyclopentadiene such as furan or thiophene, or a fused-ring derivative thereof such as benzofuran, thienofuran or benzothiophene, or a six-membered heteroaromatic compound such as pyran or a fused-ring derivative thereof such as cyclopentapyran, chromene or xanthene. Each anionic ligand L⁴ is preferably selected from the group consisting of C₁₋₂₀ carboxylate, C₁₋₂₀ alkoxy, C₂₋₂₀ alkenyloxy, C₂₋₂₀ alkynyloxy, aryloxy, C₁₋₂₀ alkoxycarbonyl, C₁₋₇ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium, arylammonium, alkyldiketonate (e.g. acetylacetonate), aryldiketonate, halogen, nitro and cyano, each of the said groups being as defined above. When said hexa-coordinated metal complex is monometallic, it preferably has only one anionic ligand L⁴.

The hexa-coordinated metal complex according to this fourth embodiment of the invention may for instance, but without limitation, be made in high yield and purity in a one-step procedure, wherein a metal (e.g. thallium) salt of the multidentate ligand (e.g. the bidentate or tridentate Schiff base) is reacted with a preferably bimetallic metal complex of the desired metal, more preferably a homobimetallic complex wherein the desired metal is coordinated with a non-anionic bidentate ligand L³ and at least one anionic ligand, such as [RuCl₂L³]₂, e.g. [RuCl₂(COD)]₂ or [RuCl₂(NBD)]₂, wherein COD and NBD respectively mean cyclooctadiene and norbornadiene. After removal of the metal salt formed with the anionic ligand, e.g. thallium chloride, the desired hexa-coordinated metal complex may be purified using conventional techniques.

In a specific embodiment which is useful namely when the metal complex of this fourth embodiment of the invention is to be used in the presence of water, it may be advantageous when one or more anionic ligands L⁴ of said hexa-coordinated metal complex is (are) abstracted and replaced with a solvent S as a ligand. This anionic ligand abstraction and replacement may be effected for instance by treating, in the presence of the solvent S, the hexa-coordinated metal complex of this fourth embodiment of the invention with an equivalent amount of a compound having the formula A-E, wherein E is a trimethylsilyl group or a metal such as silver, thus resulting in a modified hexa-coordinated metal complex being a cationic species having the solvent S as a ligand (in place of L⁴) and being associated with an anion A. The treatment also results in the formation of a compound L⁴E (e.g. silver chloride or chlorotrimethylsilane) which can be removed from the reaction mixture by conventional techniques. Suitable anions A for this purpose may be, but without limitation, selected from the group consisting of hexafluorophosphate, hexafluoroantimoniate, hexafluoroarseniate, perchlorate, tetrafluoroborate, tetra(penta-fluorophenyl)borate, alkylsulfonates wherein the alkyl group may be substituted with one or more halogen atoms, and arylsulfonates (e.g. toluenesulfonate). Suitable solvents S for coordinating with the metal in such a cationic species may be selected from the group consisting of protic solvents, polar aprotic solvents and non-polar solvents such as aromatic hydrocarbons, chlorinated hydrocarbons, ethers, aliphatic hydrocarbons, alcohols, esters, ketones, amides, and water.

More specifically, both the at least tetra-coordinated metal complex of the third embodiment of the invention and the hexa-coordinated metal complex of the fourth embodiment of the invention may have, as a multidentate ligand, a bidentate Schiff base having one of the general formulae (IA) or (IB) referred to in figure 1, wherein Z, R', R" and R"' are as previously defined. In this specific case, preferably R" and R"' together form a phenyl group which may be substituted with one or more preferably branched alkyl groups such as isopropyl or tert-butyl. The class of bidentate Schiff bases having the general formula (IA) is well known in the art and may be made for instance by condensing a salicylaldehyde with a suitably substituted aniline. The class of bidentate Schiff bases having the general formula (IB) may be made for instance by condensing benzaldehyde with a suitably selected amino-alcohol such as o-hydroxyaniline (when Z is oxygen), an amino-thiol (when Z is sulfur).

A fifth embodiment of a multicoordinated metal complex (a) suitable for reaction with an acid according to this invention is an at least penta-coordinated metal complex, a salt, a solvate or an enantiomer thereof, comprising:
- a tetradentate ligand comprising two Schiff bases, wherein the nitrogen atoms of said two Schiff bases are linked with each other through a C₁₋₇ alkylene or arylene linking group A; and
- one or more non-anionic ligands L⁷ selected from the group consisting of aromatic and unsaturated cycloaliphatic groups, preferably aryl, heteroaryl and C₄₋₂₀ cycloalkenyl groups, wherein the said aromatic or unsaturated cycloaliphatic group is optionally substituted with one or more C₁₋₇ alkyl groups or electron-withdrawing groups such as, but not limited to, halogen, nitro, cyano, (thio)carboxylic acid, (thio)carboxylic acid (thio)ester, (thio)carboxylic acid (thio)amide, (thio)carboxylic acid anhydride and (thio) carboxylic acid halide.

Each of the ligand L⁷ and the substituting groups may, independently from each other, be any of the above-mentioned groups, including any of the individual meanings for such groups or substituents which are listed in the definitions given hereinabove. Preferably the non-anionic ligand L⁷ has constraint steric hindrance such as, but not limited to, mono- or polysubstituted phenyl, e.g. xylyl, cumenyl, cymenyl or mesityl.

The at least penta-coordinated metal complex according to this fifth embodiment of the invention preferably is a monometallic complex. The metal is a transition metal selected from the group consisting of ruthenium, osmium and iron.

More specifically, in such at least penta-coordinated metal complexes of the fifth embodiment, each said non-anionic ligand L⁷ may be cymene, and the C₁₋₇ alkylene or arylene linking group A may be substituted with one or more substituents preferably selected from the group consisting of chloro, bromo, trifluoromethyl and nitro. Preferably the C₁₋₇ alkylene or arylene linking group A, together with the two linked nitrogen atoms, is derived from o-phenylenediamine, ethylenediamine, 1,3-diaminopropane, 1,4-diaminobutane, 1,5-diaminopentane, 1.6-diaminohexane or 1,7-diaminoheptane. Also preferably, each Schiff base of the tetradentate ligand is derived from salicylaldehyde or acetylacetone, wherein the salicylidene or acetylidene group included in each such Schiff base may be substituted with one or more substituents preferably selected from the group consisting of chloro, bromo, trifluoromethyl and nitro.

Suitable but non limiting examples of tetradentate ligands within the scope of this fifth embodiment of the invention have one of the general formulae (IIA) and (IIB) shown in figure 2. More specific examples include the so-called salen (i.e. bis(salicylaldehyde) ethylenediamine), saloph (i.e. bis(salicylaldehyde)o-phenylenediamine), hydroxy-acetoph, and accac (i.e. bis(acetylacetone) ethylenediamine) ligands, and substituted derivatives thereof. In formulae (IIA) and (IIB), substituents X are preferably selected from the group consisting of chloro, bromo, trifluoromethyl and nitro. In formula (IIA) substituents Y are preferably selected from the group consisting of hydrogen and methyl. A preferred tetradentate ligand is N,N'-bis(5-nitro-salicylidene)-ethylenediamine. Other suitable ligands include N,N'-1,2-cyclohexylenebis(2-hydroxyacetophenonylideneimine), 1,2-diphenylethylenebis(2-hydroxy-acetophenonylideneimine) and 1,1'-binaphtalene-2,2'-diaminobis(2-hydroxyaceto-phenonylideneimine), all being described in Molecules (2002) 7:511-516.

The at least penta-coordinated metal complex according to this fifth embodiment of the invention may be made by reacting a suitable tetradentate ligand such as defined hereinabove with a preferably bimetallic complex of the desired metal, more preferably a homobimetallic complex wherein the desired metal is coordinated with a non-anionic ligand L⁷ and at least one anionic ligand, such as [RuCl₂(p-cymene)]₂, [RuCl₂(COD)]₂ or [RuCl₂(NBD)]₂, wherein COD and NBD respectively mean cyclooctadiene and norbornadiene.

In a second aspect, the present invention will now be described with respect to a few preferred embodiments of the acid and the reaction conditions suitable for modification of the multicoordinated metal complex (a). In relation to this aspect, the choice of the acid is an important factor. In particular, it is preferred that the pKa of said acid is lower than the pKa of the multidentate Schiff base ligand. Whereas the pKa of the main available organic and inorganic acids (usually measured at room temperature (about 25°C) in aqueous solutions) is widely available in literature (for instance in Handbook of Chemistry and Physics 81st edition (2000), CRC Press, pages 8-44 to 8-56), data bases for the pKa of various possible multidentate Schiff base ligands are not necessarily available in the art (for instance, the Bordwell pKa table only provides data for a limited number of imines). This has the practical consequence that there may first be a need to determine, measure or estimate the pKa of said multidentate Schiff base ligand before making the choice of the acid. Such pKa measurement is well within the knowledge of the skilled person and may be effected according to standard practice, i.e. usually at room temperature (about 25°C) in dimethylsulfoxide (DMSO) as a solvent. Once the result of such measurement is available, it may be safe to select an acid having a pKa lower than the pKa of the multidentate Schiff base ligand by at least 2 units.

In particular, it is preferred that the pKa (measured at room temperature ― about 25°C ― in aqueous solutions) of said acid used for modification of the multicoordinated metal complex (a) is lower than about 4, i.e. with exclusion of the so-called weak acids. Based on the above criteria, acids suitable for the practice of the invention mainly include inorganic acids such as but not limited to hydrogen iodide, hydrogen bromide, hydrogen chloride, hydrogen fluoride, sulfuric acid, nitric acid, iodic acid, periodic acid and perchloric acid, as well as HOCIO, HOClO₂ and HOlO₃. Some organic acids are also suitable for the practice of the invention, including:
- sulfonic acids such as but not limited to methanesulfonic acid, aminobenzenesulfonic acid (all 3 isomers thereof), benzenesulfonic acid, naphtalenesulfonic acid, sulfanilic acid and trifluoromethanesulfonic acid;
- monocarboxylic acids such as but not limited to acetoacetic acid, barbituric acid, bromoacetic acid, bromobenzoic acid (both ortho and meta isomers thereof), chloroacetic acid, chlorobenzoic acid (all 3 isomers thereof), chlorophenoxyacetic acid (all 3 isomers thereof), chloropropionic acid (both α and β isomers thereof), cis-cinnamic acid, cyanoacetic acid, cyanobutyric acid, cyanophenoxyacetic acid (all 3 isomers thereof), cyanopropionic acid, dichloroacetic acid, dichloroacetylacetic acid, dihydroxybenzoïc acid, dihydroxymalic acid, dihydroxytartaric acid, dinicotinic acid, diphenylacetic acid, fluorobenzoïc acid, formic acid, furancarboxylic acid, furoïc acid, glycolic acid, hippuric acid, iodoacetic acid, iodobenzoïc acid, lactic acid, lutidinic acid, mandelic acid, α-naphtoïc acid, nitrobenzoïc acid, nitrophenylacetic acid (all 3 isomers thereof), *o*-phenylbenzoïc acid, thioacetic acid, thiophene-carboxylic acid, trichloroacetic acid and trihydroxybenzoïc acid; and
- other acidic substances such as but not limited to picric acid (2,4,6-trinitrophenol) and uric acid (trihydroxy 2,6,8-purine or its ketonic form).

Acids suitable for the practice of the invention also include, as an alternative embodiment, one of the above acids being generated *in situ* by methods available in the art. For instance this includes the so-called photoacid generators, i.e. compounds capable of conversion into acids upon exposure to radiation, e.g. visible light sources or deep ultraviolet (UV) light sources at short wavelengths such as the range from about 100 nm to about 350 nm, or ionizing radiation such as electron-beam or X-rays. Exemplary such photoacid generators are well known in the field of transfering images to a substrate, especially in the field of photoresist compositions and patterning processes, and include for instance monomeric generators such as bissulfonyldiazomethanes, bis(cyclohexylsulfonyl)diazomethane, the sulfonyldiazomethanes of U.S. Patent No. 6,689,530, iodonium salts and sulfonium salts (including the sulfonium salt mixtures of U.S. Patent No. 6,638,685, especially wherein two groups of a sulfonium cation together form an oxo substituted alkylene group) wherein the anion component is selected from the group consisting of perfluoroalkylsulfonate, camphorsulfonate, benzensulfonate, alkylbenzensulfonate, fluorine-substituted benzen-sulfonate, fluorine-substituted alkylbenzensulfonate and halogen (provided that said anion is able to form an acid having a pKa lower than about 4), and/or wherein the cation component comprises one or more groups such as naphtyl thienyl and pentafluorophenyl. Such photoacid generators may also include polymeric generators such as polymers with a molecular weight from about 500 to about 1,000,000 which have a sulfonium salt on their backbone and/or side chains and also have one or more organic photo-acid generating groups on side chains to generate acid by exposure to light; such polymers may be such as in preparative examples 1 and 2 of U.S. Patent No; 6,660,479 wherein the salt may be p-toluenesulfonic salt, naphtolsulfonic salt or 9,10-dimethoxy-2-anthracenesulfonic salt.

Two or more of the above-mentioned acids may also be suitable for the practice of the invention, either in the form of mixtures as far as said acids may be used together under the reaction conditions (i.e. as far as their physical form allows for simultaneous reaction with the multicoordinated metal complex) or for sequential reaction with the multicoordinated metal complex in two or more steps.

Preferred reaction conditions between the multicoordinated metal complex and the acid include one or more of the following:
- an efficient contact between the usually solid multicoordinated metal complex and the one or more acids; for instance, when said acid is a gas under the prevailing temperature conditions, it may be flowed one or more times (i.e. possibly with recirculation) through the solid mass of the metal complex with a velocity such that sufficient contact time is allowed for the heterogeneous reaction to proceed; alternatively, when said acid is a liquid or is soluble in the same or a similar solvent system (i.e. one or more preferably miscible solvents) as the multicoordinated metal complex, efficient contact may be achieved by dissolving said multicoordinated metal complex in said solvent system and adding thereto a solution of the acid in said solvent system (or, when the solvent is an ionic liquid, a species able to generate the acid *in situ* in the presence of said solvent) and stirring the mixture with suitable stirring means for sufficient time for the homogeneous reaction to proceed;
- a contact time between the multicoordinated metal complex and the one or more acids (both being optionally dissolved in a solvent system such as above defined) preferably from about 5 seconds to about 100 hours; depending upon the physical form of the reaction medium comprising the multicoordinated metal complex and the one or more acids, depending also from the multidentate Schiff base ligand and the reactivity of the selected acid, as well as other reaction conditions such as temperature, the contact time may vary within a more preferred range from about 30 seconds to about 24 hours, most preferably from 1 minute to 4 hours;
- a contact temperature ranging from about -50°C to about +80°C; it should be understood that the reaction temperature needs not be constant over the whole contact time but may be gradually increased through the above range in order to keep control of the reaction in a manner well known to those skilled in the art. For instance, the one or more acids may be added to the multicoordinated metal complex, optionally in the presence of the solvent system (such as above defined), in a vessel maintained at a relatively low temperature (i.e. below room temperature, however above the solidification temperature of said solvent system) by suitable cooling means and then the temperature is carefully raised, while monitoring any local overheating, up to a higher temperature which may be room temperature.
The molar ratio between the acid and the multicoordinated metal complex is also an important parameter in the practice of the invention. Contrary to the teaching of the prior art (U.S. Patent No. 6,284,852), this ratio is not selected to perform ligand protonation (since another feature of this invention is to avoid the presence of such protonatable ligands) or to avoid catalyst decomposition, but is selected so as to at least partly cleave a bond between the metal center of the complex and at least one multidentate Schiff base ligand. Therefore it was found desirable to select a molar ratio above about 1.2 between the acid and the multicoordinated metal complex. Preferably said ratio is above 2.0, more preferably above 3.0, and even most preferably above 4.0. Said ratio may be achieved step by step by progressively adding the acid to the multicoordinated metal complex, optionally in the presence of the solvent system as previously mentioned, over the contact time such as above defined. The addition rate of the acid may be changed, depending upon the acid, the multidentate Schiff base ligand and the selected temperature, according to routine experimentation.

The level of consumption of the acid and the progress rate of its reaction with the multicoordinated metal complex may be followed by one or more standard analytical techniques such as but not limited to infrared spectroscopy, carbon nuclear magnetic resonance (NMR) and proton NMR. These techniques will also be helpful in the determination of the precise nature of the reaction product of the invention. This nature may also be confirmed, after separation of the reaction product from the reaction medium and after its purification by suitable techniques (such as but not limited to recrystallisation), by obtaining an X-ray diffractogram of the reaction product crystalline powder. Careful examination shows that the reaction product of the invention comprises the product of at least partial cleavage of a bond betxeen the metal center and a multidentate Schiff base ligand. The bond that is partially cleaved as a result of the reaction may be a covalent bond or a coordination bond; it may be the bond between the metal center and the nitrogen atom of the Schiff base imino group, or it may be the bond between the metal center and the heteroatom (oxygen, sulfur or selenium) of the Schiff base ligand, or both such bonds may be simultaneously at least partially cleaved. The present invention does not require the said cleavage to be complete, thus partial bond cleavage leading to a mixture of the staring multicoordinated metal complex and of one or more reaction products is also within the scope of the invention. Because, as disclosed hereinafter, the reaction of the invention may be performed *in situ* in the presence of organic molecules or monomers to be processed by the catalytic activity of the resulting reaction product, it is not essential that said reaction product may be isolated in the form of one single pure chemical entity.

In another aspect, the present invention provides a catalytic system comprising:
(a) as the main catalytic species, the reaction product according to any of claims 12 to 41, and
(b) one or more second catalyst components being selected from the group consisting of Lewis acid co-catalysts (b₁), catalyst activators (b₂) and initiators having a radically transferable atom or group (b₃).

In the catalytic system of this other aspect of the invention, the second component (b) will be selected according to the kind of reaction to be catalyzed. For instance, a co-catalyst (b₁) may be useful for increasing the reaction rate of the ring opening metathesis polymerisation of cyclic olefins and may be selected, but without limitation, from the group consisting of boron trihalides; trialkylboron; triarylboron; organoaluminum compounds; magnesium halides; aluminum halides; tin tetrachloride; titanium or vanadium trihalides or tetrahalides or tetraalkoxides, preferably titanium tetrachloride or tetraisopropoxytitanium; antimony and bismuth pentahalides. For instance a co-catalyst (b₁) may be an organoaluminum compound selected from the group consisting of tri-n-alkylaluminums; dialkylaluminum hydrides, trialkenylaluminums, alkylaluminum alkoxides, dialkylaluminum alkoxides, dialkylaluminum aryloxides and dialkyl-aluminum halides. A catalyst activator (b₂) may also be useful for increasing the reaction rate of the ring opening metathesis polymerisation of cyclic olefins (thus may be combined with a co-catalyst (b₁) such as above defined) and may be a diazo compound such as, but not limited to, ethyldiazoacetate and trimethylsilyldiazomethane, or a radical initiator such as azobis(isobutyronitrile).

On the other hand, an initiator having a radically transferable atom or group (b₃) is usually required, together with the main catalytic species, for performing the radical polymerisation of a monomer since an ATRP catalytic system is based on the reversible formation of growing radicals in a redox reaction between the metal component and an initiator.

Suitable initiators include those compounds having the formula R₃₅R₃₆R₃₇CX₁ wherein:
- X₁ is selected from the group consisting of halogen, OR₃₈ (wherein R₃₈ is selected from C₁₋₂₀ alkyl, polyhaloC₁₋₂₀alkyl, C₂₋₂₀ alkynyl (preferably acetylenyl), C₂₋₂₀ alkenyl (preferably vinyl), phenyl optionally substituted with 1 to 5 halogen atoms or C₁₋₇ alkyl groups and phenyl-substituted C₁₋₇ alkyl), SR₃₉, OC(=O)R₃₉, OP(=O)R₃₉, OP(=O)(OR₃₉)₂, OP(=O)OR₃₉, O--N(R₃₉)₂ and S--C(=S)N(R₃₉)₂, wherein R₃₉ is aryl or C₁₋₂₀ alkyl, or where an N(R₃₉)₂ group is present, the two R₃₉ groups may be joined to form a 5-, 6- or 7-membered heterocyclic ring (in accordance with the definition of heteroaryl above), and
- R₃₅, R₃₆ and R₃₇ are each independently selected from the group consisting of hydrogen, halogen, C₁₋₂₀ alkyl (preferably C₁₋₆ alkyl), C₃₋₈ cycloalkyl, C(=O)R₄₀, (wherein R₄₀ is selected from the group consisting of C₁₋₂₀ alkyl, C₁₋₂₀ alkoxy, aryloxy or heteroaryloxy), C(=O)NR₄₁R₄₂ (wherein R₄₁ and R₄₂ are independently selected from the group consisting of hydrogen and C₁₋₂₀ alkyl or R₄₁ and R₄₂ may be joined together to form an alkylene group of 2 to 5 carbon atoms), COCI, OH, CN, C₂₋₂₀ alkenyl (preferably vinyl), C₂₋₂₀ alkynyl, oxiranyl, glycidyl, aryl, heteroaryl, arylalkyl and aryl-substituted C₂₋₂₀ alkenyl.

In these initiators, X₁ is preferably bromine, which provides both a higher reaction rate and a lower polymer polydispersity.

When an alkyl, cycloalkyl, or alkyl-substituted aryl group is selected for one of R₃₅, R₃₆ and R₃₇, the alkyl group may be further substituted with an X₁ group as defined above. Thus, it is possible for the initiator to serve as a starting molecule for branch or star (co)polymers. One example of such an initiator is a 2,2-bis(halomethyl)-1,3-dihalopropane (e.g. 2,2-bis(chloromethyl)-1,3-dichloropropane or 2,2-bis(bromomethyl)-1,3-dibromopropane), and a preferred example is where one of R_{35,} R₃₆ and R₃₇ is phenyl substituted with from one to five C₁₋₆ alkyl substituents, each of which may independently be further substituted with a X₁ group (e.g. α,α'-dibromoxylene, hexakis(α-chloro- or α-bromomethyl)benzene). Preferred initiators include 1-phenylethyl chloride and 1-phenylethyl bromide, chloroform, carbon tetrachloride, 2-chloropropionitrile and C₁₋₇ alkyl esters of a 2-halo-C₁₋₇ carboxylic acid (such as 2-chloropropionic acid, 2-bromopropionic acid, 2-chloroisobutyric chloroisobutyric acid, 2-bromo-isobutyric acid and the like). Another example of a suitable initiator is dimethyl-2-chloro-2,4,4-trimethylglutarate.

In the catalytic system of this other aspect of the invention, the multi-coordinated metal complex may be for instance any of the first, second, third, fourth and fifth embodiments respectively, of such complexes detailed hereinabove.

In yet another aspect, the present invention also provides a supported catalyst, preferably for use in a heterogeneous catalytic reaction, comprising:
(a) a catalytic system comprising a catalytically active reaction product according to any of claims 12 to 41, and
(b) a supporting amount of a carrier suitable for supporting said catalytic system (a).

The catalytic system (a) included in the supported catalyst of this aspect of the invention may, in addition to the reaction product of a multcoordinated metal complex and an acid, comprise one or more second catalyst components such as Lewis acid co-catalysts (b₁), catalyst activators (b₂) and initiators having a radically transferable atom or group (b₃) already discussed in the previous aspect of the invention.

In such a supported catalyst, said carrier (b) may be selected from the group consisting of porous inorganic solids (including silica, zirconia and alumino-silica), such as amorphous or paracrystalline materials, crystalline molecular sieves and modified layered materials including one or more inorganic oxides, and organic polymer resins such as polystyrene resins and derivatives thereof.

Porous inorganic solids that may be used with the supported catalysts of the invention have an open microstructure that allows molecules access to the relatively large surface areas of these materials that enhance their catalytic and sorptive activity. These porous materials can be sorted into three broad categories using the details of their microstructure as a basis for classification. These categories are the amorphous and paracrystalline supports, the crystalline molecular sieves and modified layered materials. The detailed differences in the microstructures of these materials manifest themselves as important differences in the catalytic and sorptive behavior of the materials, as well as in differences in various observable properties used to characterize them, such as their surface area, the sizes of pores and the variability in those sizes, the presence or absence of X-ray diffraction patterns and the details in such patterns, and the appearance of the materials when their microstructure is studied by transmission electron microscopy and electron diffraction methods. Amorphous and paracrystalline materials represent an important class of porous inorganic solids that have been used for many years in industrial applications. Typical examples of these materials are the amorphous silicas commonly used in catalyst formulations and the paracrystalline transitional aluminas used as solid acid catalysts and petroleum reforming catalyst supports. The term "amorphous" is used here to indicate a material with no long range order and can be somewhat misleading, since almost all materials are ordered to some degree, at least on the local scale. An alternate term that has been used to describe these materials is "X-ray indifferent". The microstructure of the silicas consists of 100-250 Angstrom particles of dense amorphous silica (Kirk-Othmer Encyclopedia of Chemical Technology, 3rd. ed., vol. 20, 766-781 (1982)), with the porosity resulting from voids between the particles.

Paracrystalline materials such as the transitional aluminas also have a wide distribution of pore sizes, but better defined X-ray diffraction patterns usually consisting of a few broad peaks. The microstructure of these materials consists of tiny crystalline regions of condensed alumina phases and the porosity of the materials results from irregular voids between these regions (K. Wefers and Chanakya Misra, "Oxides and Hydroxides of Aluminum", Technical Paper No 19 Revised, Alcoa Research Laboratories, 54-59 (1987)). Since, in the case of either material, there is no long range order controlling the sizes of pores in the material, the variability in pore size is typically quite high. The sizes of pores in these materials fall into a regime called the mesoporous range,, including, for example, pores within the range of about 15 to about 200 Angstroms.

In sharp contrast to these structurally ill-defined solids are materials whose pore size distribution is very narrow because it is controlled by the precisely repeating crystalline nature of the materials' microstructure. These materials are called "molecular sieves" the most important examples of which are zeolites. Zeolites, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials are known as "molecular sieves" and are utilized in a variety of ways to take advantage of these properties. Such molecular sieves, both natural and synthetic, include a wide variety of positive ion-containing crystalline silicates. These silicates can be described as a rigid three-dimensional framework of SiO₄ and Periodic Table Group IIIB element oxide, e.g., AlO₄, in which the tetrahedra are crosslinked by the sharing of oxygen atoms whereby the ratio of the total Group IIIB element, e.g., aluminum, and Group IVB element, e.g., silicon, atoms to oxygen atoms is 1:2. The electrovalence of the tetrahedra containing the Group IIIB element, e.g., aluminum, is balanced by the inclusion in the crystal of a cation, for example, an alkali metal or an alkaline earth metal cation. This can be expressed wherein the ratio of the Group IIIB element, e.g., aluminum, to the number of various cations, such as Ca, Sr, Na, K or Li, is equal to 1. One type of cation may be exchanged either entirely or partially with another type of cation utilizing ion exchange techniques in a conventional manner. By means of such cation exchange, it has been possible to vary the properties of a given silicate by suitable selection of the cation. Many of these zeolites have come to be designated by letter or other convenient symbols, as illustrated by zeolites A (U.S. Pat. No. 2,882,243); X (U.S. Pat. No. 2,882,244); Y (U.S. Pat. No. 3,130,007); ZK-5 (U.S. Pat. No. 3,247,195); ZK-4 (U.S. Pat. No. 3,314,752); ZSM-5 (U.S. Pat. No. 3,702,886); ZSM-11 (U.S. Pat. No. 3,709,979); ZSM-12 (U.S. Pat. No. 3,832,449), ZSM-20 (U.S. Pat. No. 3,972,983); ZSM-35 (U.S. Pat. No. 4,016,245); ZSM-23 (U.S. Pat. No. 4,076,842); MCM-22 (U.S. Pat. No. 4,954,325); MCM-35 (U.S. Pat. No. 4,981,663); MCM-49 (U.S. Pat. No. 5,236,575); and PSH-3 (U.S. Pat. No. 4,439,409). The latter refers to a crystalline molecular sieve composition of matter named PSH-3 and its synthesis from a reaction mixture containing hexamethyleneimine, an organic compound which acts as directing agent for synthesis of a layered MCM-56. A similar composition, but with additional structural components, is taught in European Patent Application 293,032. Hexamethyleneimine is also taught for use in synthesis of crystalline molecular sieves MCM-22 in U.S. Pat. 4,954,325; MCM-35 in U.S. Pat. No. 4,981,663; MCM-49 in U.S. Pat. 5,236,575; and ZSM-12 in U.S. Pat. No. 5,021,141. A molecular sieve composition SSZ-25 is taught in U.S. Pat. No. 4,826,667 and European Patent Application 231,860, said zeolite being synthesized from a reaction mixture containing an adamantane quaternary ammonium ion. Molecular sieve material being selected from the group consisting of zeolites REY, USY, REUSY, dealuminated Y, ultrahydrophobic Y, silicon-enriched dealuminated Y, ZSM-20, Beta, L, silicoaluminophosphates SAPO-5, SAPO-37, SAPO-40, MCM-9, metalloaluminophosphate MAPO-36, aluminophosphate VPI-5 and mesoporous crystalline MCM-41 are suitable for including into a supported catalyst of this invention.

Certain layered materials, which contain layers capable of being spaced apart with a swelling agent, may be pillared to provide materials having a large degree of porosity. Examples of such layered materials include clays. Such clays may be swollen with water, whereby the layers of the clay are spaced apart by water molecules. Other layered materials are not swellable with water, but may be swollen with certain organic swelling agents such as amines and quaternary ammonium compounds. Examples of such non-water swellable layered materials are described in U.S. Pat. No. 4,859,648 and include layered silicates, magadiite, kenyaite, trititanates and perovskites. Another example of a non-water swellable layered material, which can be swollen with certain organic swelling agents, is a vacancy-containing titanometallate material, as described in U.S. Pat. No. 4,831,006. Once a layered material is swollen, the material may be pillared by interposing a thermally stable substance, such as silica, between the spaced apart layers. The aforementioned U.S. Pat. Nos. 4,831,006 and 4,859,648 describe methods for pillaring the non-water swellable layered materials described therein and are incorporated herein by reference for definition of pillaring and pillared materials. Other patents teaching pillaring of layered materials and the pillared products include U.S. Pat. Nos. 4,216,188; 4,248,739; 4,176,090; and 4,367,163; and European Patent Application 205,711. The X-ray diffraction patterns of pillared layered materials can vary considerably, depending on the degree that swelling and pillaring disrupt the otherwise usually well-ordered layered microstructure. The regularity of the microstructure in some pillared layered materials is so badly disrupted that only one peak in the low angle region on the X-ray diffraction pattern is observed, at a d-spacing corresponding to the interlayer repeat in the pillared material. Less disrupted materials may show several peaks in this region that are generally orders of this fundamental repeat. X-ray reflections from the crystalline structure of the layers are also sometimes observed. The pore size distribution in these pillared layered materials is narrower than those in amorphous and paracrystalline materials but broader than that in crystalline framework materials.

In yet another aspect the present invention provides a method according to claim 47 of performing an
olefin metathesis (the latter being as explained in the background of the invention or as defined in http://www.ilpi.com/organomet/olmetathesis.html), in particular the ring-opening metathesis polymerisation of cyclic olefins, or an acetylene metathesis (the latter being as defined in http://www.ilpi.com/organomet/acmetathesis.html, a reaction in which all carbon-carbon triple bonds in a mixture of alkynes are cut and then rearranged in a statistical fashion, and involving a metalla-cyclobutadiene intermediate) or a reaction involving the transfer of an atom or group to an ethylenically or acetylenically unsaturated compound or another reactive substrate such as, but not limited to, saturated hydrocarbons, aldehydes, ketones, alcohols, alkyl halides and the like. That is, this aspect of the invention relates to methods of performing the above listed reactions in the presence of a catalytic component comprising the said reaction product.

An atom or group transfer reaction usually comprises the step of reacting the said ethylenically or acetylenically unsaturated compound or other reactive substrate with a second reactive substrate under suitable reaction conditions and in the presence of a suitable catalytic component, the second reactive substrate being a suitable donor for the atom or group to be transferred.

More specifically, the said atom or group transfer reactions (which will be detailed below) may be, but without limitation, selected from the group consisting of:
- atom or group transfer radical polymerisation of one or more radically (co)polymerisable monomers, especially mono- and diethylenically unsaturated monomers;
- atom transfer radical addition (the latter being as explained in the background of the invention), e.g. the addition of a polyhalomethane having the formula CXₙH₄₋ₙ, wherein X is halogen and n is an integer from 2 to 4, onto an ethylenically unsaturated compound to produce the corresponding saturated polyhalogenated adduct, including for instance the addition of carbon tetrachloride or chloroform onto an α-olefin;
- vinylation reaction, i.e. the reaction of a mono- or di-alkyne (e.g. phenylacetylene or 1,7-octadiyne) with a monocarboxylic acid (e.g. formic acid or acetic acid) or dicarboxylic acid to produce alk-1-enyl esters or enol esters or Markovnikov adducts or anti- Markovnikov adducts or mixtures thereof;
- cyclopropanation of an α-ethylenically unsaturated compound for producing an organic compound having one or more cyclopropane structural units;
- quinoline synthesis through oxidative cyclisation of 2-aminobenzyl alcohol with ketones;
- epoxidation of α-ethylenically unsaturated compounds for producing epoxides;
- oxidation of organic compounds including the oxidation of saturated hydrocarbons (such as, but not limited to, methane) for producing alcohols, or sulfides for producing sulfoxides and sulfones, or phosphines for producing phosphonates, or alcohols and aldehydes for producing carboxylic acids;
- cyclopropenation of an alkyne for producing an organic compound having one or more cyclopropene structural units;
- hydrocyanation of α-ethylenically unsaturated compounds for producing saturated nitriles, or alkynes for producing unsaturated nitriles, or α,β-unsaturated aldehydes or ketones for producing β-cyano carbonyl compounds;
- hydrosilylation of olefins for producing saturated silanes, or alkynes for producing unsaturated silanes, or ketones for producing silyl ethers, or trimethylsilylcyanation of aldehydes for producing cyanohydrin trimethylsilyl ethers;
- aziridination of imines or alkenes for producing organic compounds having one or more aziridine structural units;
- hydroamidation of olefins for producing saturated amides;
- hydrogenation of olefins for producing alkanes, or ketones for producing alcohols;
- aminolysis of olefins for producing saturated primary or secondary amines;
- isomerisation of alcohols, preferably allylic alcohols, for producing aldehydes;
- Grignard cross-coupling of alkyl or aryl halides for producing alkanes or arylalkanes;
- hydroboration of olefins for producing alkylboranes and trialkylboranes;
- hydride reduction of aldehydes and ketones for producing alcohols;
- aldol condensation of saturated carboxyl compounds (aldehydes or ketones) for producing α,β-unsaturated carboxyl compounds or β-hydroxycarbonyl compounds, and intra-molecular aldol condensation of dialdehydes or diones for producing cyclic α,β-unsaturated carboxyl compounds (aldehydes or ketones);
- Michael addition of a ketone or a β-dicarbonyl compound onto an α,β-unsaturated carboxyl compound for producing saturated polycarboxyl compounds;
- Robinson annulation, i.e. Michael addition followed by an intramolecular aldol condensation, of a ketone onto an α,β-unsaturated carboxyl compound for producing saturated polycyclic carboxyl compounds being suitable intermediates for steroids and other natural products containing six-membered rings;
- Heck reactions, i.e. the reaction of an aryl halide or a 1-hetero-2,4-cyclopentadiene (or a benzo-fused derivative thereof) with an α-ethylenically unsaturated compound for producing arylalkenes or heteroarylalkenes;
- codimerisation of alkenes for producing higher saturated hydrocarbons or alkynes for producing higher alkenes;
- hydroxylation of olefins for producing alcohols;
- hydroamination of olefins and alkynes,
- alkylation, preferably allylic alkylation, of ketones for producing alkylated ketones, preferably allylic ketones; and
- Diels-Alder reactions such as, but not limited to, the cycloaddition of a conjugated diene onto an α-ethylenically unsaturated compound for producing optionally substituted cyclohexenes, or the cycloaddition of furan onto an α-ethylenically unsaturated compound for producing optionally substituted 7-oxanorbornenes.

Each of the above organic synthesis reactions, which will be described in more detail hereinafter, is known *per se.* For further details on each type of reaction, reference may be made for instance to K. Vollhardt and N. Schore, Organic chemistry, structure and function (1999) by W.H. Freeman (3rd edition) and to B. Cornils and A. Herrmann, Applied homogeneous catalysis with organometallic compounds (2000) by Wiley.

Each organic synthesis reaction of this sixth aspect of the invention may be conducted in a continuous, semi-continuous, or batch manner and may involve a liquid and/or gas recycling operation as desired. The manner or order of addition of the reactants, catalyst, and solvent are usually not critical. Each organic synthesis reactions may be carried out in a liquid reaction medium that contains a solvent for the active catalyst, preferably one in which the reactants, including catalyst, are substantially soluble at the reaction temperature.

In a first embodiment of this sixth aspect of the invention, the said reaction is an olefin metathesis reaction for transforming a first olefin into at least one second olefin or into a linear olefin oligomer or polymer or into a cyclo-olefin. The invention thus relates to a method for performing an olefin metathesis reaction comprising contacting at least one first olefin with the catalytic component, optionally supported on a suitable carrier such as decribed hereinabove with reference to the fifth aspect of the invention. The high activity of the metal complexes of this invention cause these compounds to coordinate with, and catalyze metathesis reactions between, many types of olefins. Exemplary olefin metathesis reactions enabled by the metal complexes of the present invention include, but are not limited to, RCM of acyclic dienes, cross metathesis reactions, de-polymerization of olefinic polymers and, more preferaby, ROMP of strained cyclic olefins. In particular, the catalytic components of this invention may catalyze ROMP of unsubstituted, monosubstituted and disubstituted strained mono-, bi- and polycyclic olefins with a ring size of at least 3, preferably 3 to 5, atoms; examples thereof include norbornene, cyclobutene, norbornadiene, cyclopentene, dicyclopentadiene, cycloheptene, cyclooctene, 7-oxanorbornene, 7-oxanorbornadiene, cyclooctadiene, cyciododecene, mono- and disubstituted derivatives thereof, especially derivatives wherein the substituent may be C₁₋₇ alkyl, cyano, diphenylphosphine, trimethylsilyl, methylaminomethyl, carboxylic acid or ester, trifluoromethyl, maleic ester, maleimido and the like, such as disclosed in U.S. Patent No. 6,235,856, the content of which is incorporated herein in its entirety. The invention also contemplates ROMP of mixtures of two or more such monomers in any proportions. Further examples include water-soluble cyclic olefins such as exo-N-(N',N',N'-trimethylammonio)ethyl-bicyclo[2.2.1] hept-5-ene-2,3-dicarboximide chloride or exo-N-(N',N',N'-trimethylammonio)ethyl-bicyclo-7-oxabicyclo[2.2.1]hept-5-ene-2,3-dicarboximide chloride. As is well known to the skilled person, olefins such as cyclohexenes which have little or no ring strain cannot be polymerized because there is no thermodynamic preference for polymer versus monomer.

ROMP of the invention may be carried out in an inert atmosphere for instance by dissolving a catalytic amount of the catalytic component in a suitable solvent and then adding one or more of the said strained cyclic olefins, optionally dissolved in the same or another solvent, to the catalyst solution, preferably under agitation. Because a ROMP system is typically a living polymerisation process, two or more different strained cyclic olefins may be polymerised in subsequent steps for making diblock and triblock copolymers, thus permitting to tailor the properties of the resulting material, provided that the ratio of chain initiation and chain propagation is suitably selected. Solvents that may be used for performing ROMP include all kinds of organic solvents such as protic solvents, polar aprotic solvents and non-polar solvents, as well as supercritical solvents such as carbon dioxide (while performing ROMP under supercritical conditions) and aqueous solvents, which are inert with respect to the strained cyclic olefin and the catalytic component under the polymerization conditions used. More specific examples of suitable organic solvents include ethers (e.g. dibutyl ether, tetrahydrofuran, dioxane, ethylene glycol monomethyl or dimethyl ether, ethylene glycol monoethyl or diethyl ether, diethylene glycol diethyl ether or triethylene glycol dimethyl ether), halogenated hydrocarbons (e.g. methylene chloride, chloroform, 1,2-dichloroethane, 1,1,1-trichloroethane or 1,1,2,2-tetrachloroethane), carboxylic acid esters and lactones (e.g. ethyl acetate, methyl propionate, ethyl benzoate, 2-methoxyethyl acetate, γ-butyrolactone, δ-valerolactone or pivalolactone), carboxylic acid amides and lactams (e.g. N,N-dimethylformamide, N,N-diethylformamide, N,N-dimethylacetamide, tetramethylurea, hexamethyl-phosphoric acid triamide, γ-butyrolactam, ε-caprolactam, N-methylpyrrolidone, N-acetylpyrrolidone or N-methylcaprolactam), sulfoxides (e.g. dimethyl sulfoxide), sulfones (e.g. dimethyl sulfone, diethyl sulfone, trimethylene sulfone or tetramethylene sulfone), aliphatic and aromatic hydrocarbons (e.g. petroleum ether, pentane, hexane, cyclohexane, methylcyclohexane, benzene, chlorobenzene, o-dichlorobenzene, 1,2,4-trichlorobenzene, nitrobenzene, toluene or xylene), and nitriles (e.g. acetonitrile, propionitrile, benzonitrile or phenylacetonitrile).

When water or an aqueous mixture is selected as the solvent, it is preferable to use a cationic metal complex species as the catalytic component, the said cationic species being associated with an anion A as described hereinabove.

The solubility of the polymer formed by ROMP will depend upon the choice of the strained cyclic olefin, the choice of the solvent and the molecular weight and concentration of the polymer obtained. When the strained cyclic olefin is polyunsaturated (e.g. dicyclopentadiene or norbornadiene), the polymer obtained may often be insoluble, whatever the solvent used. Polymerisation temperatures may range from about 0°C to about 120°C, preferably 20°C to 85°C, also depending upon the strained cyclic olefin and the solvent. The duration of polymerisation may be at least about 1 minute, preferably at least 5 minutes, and more preferably at least 30 minutes; the duration of polymerisation may be at most about 24 hours (although longer times may be used at the expense of economic conditions), preferably at most about 600 minutes, and even below 60 minutes. The molar ratio of the strained cyclic olefin to the catalytic component of the invention is not critical and, depending upon the olefin to be polymerised, may be at least about 100, preferably at least 250, more preferably at least 500. The said molar ratio is usually at most about 1,000,000, preferably at most 300,000 and more preferably at most 50,000. Before the polymer formed solidifies in the reactor or mold or, at will, when a desired molecular weight of the polymer has been achieved (as may be controlled for instance by monitoring reactor temperature and/or reaction mixture viscosity), an oxidation inhibitor and/or a terminating or chain-transfer agent may be added to the reaction mixture, if needed. The choice of the terminating or chain-transfer agent used is not critical to this invention, provided that the said terminating agent reacts with the catalytic component and produces another species which is inactive, i.e. not able to further propagate the polymerisation reaction, under the prevailing conditions (e.g. temperature). For instance, adding a molar excess (with respect to the catalytic component) of a carbonyl compound to the reaction mixture is able to produced a metal oxo and an olefin (or polymer) capped with the former carbonyl functionality; the cleaved polymer can then be separated from the catalyst by precipitation with methanol. Another way of cleaving the polymer from the catalyst may be by the addition of a vinylalkylether. Alternatively, reaction with several equivalents of a chain-transfer agent such as a diene is another way of cleaving the polymer chain, which method does not deactivate the catalytic component, permitting additional monomer to be polymerised, however possibly at the risk of broadening molecular weight distribution.

Because the metal complexes of this invention are stable in the presence of various functional groups, they may be used to catalyze a wide variety of olefins under a wide variety of process conditions. In particular the olefinic compound to be converted by a metathesis reaction may include one or more, preferably at most 2, functional atoms or groups, being for instance selected from the group consisting of hydroxyl, thiol (mercapto), ketone, aldehyde, ester (carboxylate), thioester, cyano, cyanato, epoxy, silyl, silyloxy, silanyl, siloxazanyl, boronato, boryl, stannyl, disulfide, carbonate, imine, carboxyl, amine, amide, carboxyl, isocyanate, thioisocyanate, carbodiimide, ether (preferably C₁₋₂₀ alkoxy or aryloxy), thioether (preferably C₁₋₂₀ thioalkoxy or thioaryloxy), nitro, nitroso, halogen (preferably chloro), ammonium, phosphonate, phosphoryl, phosphino, phosphanyl, C₁₋₂₀ alkylsulfanyl, arylsulfanyl, C₁₋₂₀ alkylsulfonyl, arylsulfonyl, C₁₋₂₀ alkylsulfinyl, arylsulfinyl, sulfonamido and sulfonate (preferably toluenesulfonate, methanesulfonate or trifluoromethanesulfonate). The said olefin functional atom or group may be either part of a substituting group of the olefin or part of the carbon chain of the olefin.

The metal complexes of this invention are also useful components for catalyzing, at relatively low temperatures (from about 20°C to 80°C), in the presence or absence of a solvent, the ring-closing metathesis of acyclic dienes such as, for instance, diallylic compounds (diallyl ether, diallyl thioether, diallyl phtalate, diallylamino compounds such as diallylamine, diallylamino phosphonates, diallyl glycine esters), 1,7-octadiene, substituted 1,6-heptadienes and the like.

The metal complexes of this invention may also be used as catalytic components for the preparation of telechelic polymers, i.e. macromolecules with one or more reactive endgroups which are useful materials for chain extension processes, block copolymer synthesis, reaction injection moulding, and polymer network formation. An example thereof is hydroxyl-telechelic polybutadiene which may be obtained from 1,5-cycooctadiene, 1,4-diacetoxy-cis-2-butene and vinyl acetate. For most applications, a highly functionalized polymer, i.e. a polymer with at least two functional groups per chain, is required. The reaction scheme for a telechelic polymer synthesis via ring opening metathesis polymerisation is well known to those skilled in the art: in such a scheme, acyclic olefins act as chain-transfer agents in order to regulate the molecular weight of the telechelic polymer produced. When α,ω-bifunctional olefins are used as chain-transfer agents, truly bi-functional telechelic polymers can be synthesized.

According to the sixth aspect of the invention, olefin coupling may be performed by cross-metathesis comprising the step of contacting a first olefinic compound with such a metal complex in the presence of a second olefin or functionalized olefin. The said first olefinic compound may be a diolefin or a cyclic mono-olefin with a ring size of at least 3 atoms, and the said metathesis cross-coupling is preferably performed under conditions suitable for transforming said cyclic mono-olefin into a linear olefin oligomer or polymer, or said diolefin into a mixture of a cyclic mono-olefin and an aliphatic alpha-olefin.

Depending upon the selection of the starting substrates for the olefin metathesis reaction and the desired organic molecule to be produced, the olefin metathesis reaction can yield a very wide range of end-products including biologically active compounds. For instance the reaction may be for transforming a mixture of two dissimilar olefins, at least one of which is an alpha-olefin, selected from (i) cyclodienes containing from 5 to 12 carbon atoms and (ii) olefins having the formula:

XHC=CH-(CH₂)ᵣ-(CH=CH)ₐ-(CHX')_{c}-(CH₂)ₜ-X" (IV),

into an unsaturated biologically active compound having the formula:

H(CH₂)_{z}-(CH=CH)ₐ-(CH₂)ₘ-(CH=CH)_{b}-(CH₂)ₚX" (V),

wherein
a is an integer from 0 to 2; b is selected from 1 and 2; c is selected from 0 and 1; m and p are such that the hydrocarbon chain in formula (V) contains from 10 to 18 carbon atoms; r and t are such that the combined total of carbon atoms in the hydrocarbon chains of the two dissimilar olefins of formula (IV) is from 12 to 40; z is an integer from 1 to 10, and X, X' and X" are atoms or groups each independently selected from hydrogen, halogen, methyl, acetyl, -CHO and -OR₁₂, wherein R₁₂ is selected from hydrogen and an alcohol protecting group selected from the group consisting of tetrahydropyranyl, tetrahydrofuranyl, tert-butyl, trityl, ethoxyethyl and SiR₁₃R₁₄R₁₅ wherein R₁₃, R₁₄ and R₁₅ are each independently selected from C₁₋₇ alkyl groups and aryl groups.

The said unsaturated biologically active compound having the formula (V) may be a pheromone or pheromone precursor, an insecticide or a insecticide precursor, a pharmaceutically active compound or a pharmaceutical intermediate, a fragrance or a fragrance precursor. A few examples of the said unsaturated biologically active compounds include 1-chloro-5-decene, 8,10-dodecadienol, 3,8,10-dodecatrienol, 5-decenyl acetate, 11-tetradecenylacetate, 1,5,9-tetradeca-triene and 7,11-hexadecadienyl acetate. The latter is a pheronome commercially available under the trade name Gossyplure, useful in pest control by effectively disrupting the mating and reproductive cycles of specifically targeted insect species, which may be produced from 1,5,9-tetradecatriene, the latter being obtainable from cyclooctadiene and 1-hexene according to the present invention.

When performing the olefin metathesis reaction of the invention, although in most cases the said reaction proceeds very quickly, it may be advantageous for a few specific olefins, in order to improve the reaction rate and/or yield, to further contact the olefin with a Lewis acid co-catalyst (b₁) and/or a catalyst activator (b₂). The Lewis acid co-catalyst (b₁) may be selected from the group consisting of boron trihalides; trialkylboron; triarylboron; organoaluminum compounds; magnesium halides; aluminum halides; titanium or vanadium halides, preferably titanium tetrachloride; antimony and bismuth pentahalides. For instance the Lewis acid co-catalyst (b₁) may be an organoaluminum compound selected from the group consisting of tri-n-alkylaluminums; dialkyl-aluminum hydrides, trialkenylaluminums, alkylaluminum alkoxides, dialkylaluminum alkoxides, dialkylaluminum aryloxides and dialkylaluminum halides. The catalyst activator (b₂) may be for instance a diazo compound such as, but not limited to, ethyldiazoacetate and trimethylsilyldiazomethane.

At the opposite, ring-opening metathesis polymerization (ROMP) reactions using the catalytic components of the invention may proceed so quickly for olefinic monomers such as dicyclopentadiene or oligomers thereof (i.e. Diels-Alder adducts formed with about 1 to 20 cyclopentadiene units) or mixtures thereof with strained monocyclic or polycyclic fused olefins (e.g. as defined in U. S. Patent No. 6,235,856, the content of which is incorporated herein by reference) that polymerization control could become a problem in the absence of appropriate measures. This kind of problem is likely to occur during the molding of thermoset polymers wherein a liquid olefin monomer and a catalyst are mixed and poured, cast or injected into a mold and wherein on completion of polymerization (i.e. "curing" of the article) the molded part is removed from the mold before any post cure processing that may be required, such as in the Reaction Injection Molding ("RIM") technique. It is well known that the ability to control reaction rates, i.e. the pot life of the reaction mixture, becomes more important in the molding of larger parts using this technique. Using the catalytic components of this invention, extending the pot life and/or controlling the rate of an olefin metathesis polymerisation reaction may be effected in different ways, such as increasing the catalyst/olefin ratio and/or adding a polymerization retardant to the reaction mixture. Moreover this can be achieved by an improved embodiment comprising:
(a) a first step of contacting the olefin metathesis catalytic component (optionally supported) of the invention with an olefin in a reactor at a first temperature at which the said olefin metathesis catalytic component is substantially unreactive (inactive), and
(b) a second step of bringing the reactor temperature (e.g. heating the contents of said reactor) up to a second temperature above the said first temperature, at which said catalytic component is active, until completion of polymerisation.

In a more specific version of this improved embodiment, heat activation occurs in bursts rather than continuously, e.g. by repeating the sequence of steps (a) and (b).

Within the said controlled polymerization method, it should be understood that the non-reactivity of the catalytic component in the first step depends not only on the first temperature but also on the nature of the olefin(s) used in the said RIM technique and on the olefin/catalytic component ratio. Preferably the first temperature is about 20°C but, for specific olefins or specific olefin/catalytic component ratios, it may even be suitable to cool the reaction mixture below room temperature, e.g. down to about 0°C. The second temperature is preferably above 40°C and may be up to about 90°C.

ROMP using the catalytic components of this invention readily achieve linear or crosslinked polymers of the above-mentioned strained cyclic olefins, such as polynorbornenes and polydicyclopentadienes, with well controlled characteristics, i.e. average molecular weight and molecular weight distribution (polydispersity). In particular, norbornene polymers with an average molecular weight ranging from about 200,000 to 2,000,000 and a molecular weight distribution (polydispersity) from about 1.1 to 2.2 may be prepared. Polymerisation, in particular when performed in a mold such as in the RIM technique, may occur in the presence of one or more formulation auxiliaries, such as antistatics, antioxidants, ceramics, light stabilizers, plasticizers, dyes, pigments, fillers, reinforcing fibers, lubricants, adhesion promoters, viscosity-enhancing agents and demolding agents, all said auxilaries being well known in the art.

Depending upon the specific reaction involved in this sixth aspect of this invention, and especially when the said reaction is ROMP of strained cyclic olefins, reaction may also advantageously be performed under visible light or ultra-violet light irradiation, e.g. using a source of visible light or ultra-violet light being able to deliver sufficient energy to the reaction system.

In another embodiment of the sixth aspect of the present invention, the catalytic component is used for the atom transfer radical addition (ATRA) reaction of a polyhalogenated alkane CXCl₃, wherein X is hydrogen, C₁₋₇ alkyl, phenyl or halogen, onto an olefin or diolefin. Such reaction is preferably performed in the presence of an organic solvent, in a molar excess of the polyhalogenated alkane, and within a temperature range between about 30° and 100°C. Suitable examples of the polyhalogenated alkanes are carbon tetrachloride, chloroform, trichlorophenylmethane and carbon tetrabromide. Examples of suitable olefins for this radical addition reaction include internal and cyclic olefins as well as terminal olefins having the formula RR'C=CH₂, wherein R and R' may be each independently selected from hydrogen, C₁₋₇ alkyl, phenyl and carboxylic acid or ester, e.g. vinylaromatic monomers such as styrene or vinyltoluene, α,β-ethylenically unsaturated acid esters such as C₁₋₇ alkyl acrylates and methacrylates, acrylonitrile and the like.

In another embodiment of the sixth aspect of the present invention, the catalytic component is used for the atom or group transfer radical polymerization (ATRP) of one or more radically (co)polymerizable monomers. It is critical to the success of the living/controlled radical polymerisation contemplated in this embodiment to achieve rapid exchange between growing radicals present at low stationary concentrations (in a range of from about 10⁻⁸ mole/l to 10⁻⁶ mole/I) and dormant chains present at higher concentrations (typically in a range of from about 10⁻⁴ mole/I to 1 mole/I). It may therefore be desirable to match the respective amounts of the catalytic component of the invention and of the radically (co)polymerizable monomer(s) in such a way that these concentration ranges are achieved. If the concentration of growing radicals exceeds about 10⁻⁶ mole/I, there may be too many active species in the reaction, which may lead to an undesirable increase in the rate of side reactions (e.g. radical-radical quenching, radical abstraction from species other than the catalyst system, and do on). If the concentration of growing radicals is less than about 10⁻⁸ mole/I, the polymerisation rate may be undesirably slow. Similarly, if the concentration of dormant chains is less than about 10⁻⁴ mole/I, the molecular weight of the polymer produced may increase dramatically, thus leading to a potential loss of control of its polydispersity. On the other hand, if the concentration of dormant species is greater than 1 mole/I, the molecular weight of the reaction product may likely become too small and result in the properties of an oligomer with no more than about 10 monomeric units. In bulk, a concentration of dormant chains of about 10⁻² mole/I provides a polymer having a molecular weight of about 100,000 g/mole.

The various catalytic components of the present invention are suitable for the radical polymerisation of any radically polymerizable, ethylenically or acetylenically unsaturated compound, including acrylic acid, methacrylic acid, acrylic acid esters, methacrylic acid esters, acrylic acid amides, methacrylic acid amides, imides (such as N-cyclohexylmaleimide and N-phenylmaleimide), styrenes, dienes or mixtures thereof. By providing the said compounds in a single step or in a multi-steps procedure, they are able to provide controlled copolymers having various structures, including block, random, gradient, star-shaped, graft, comb-shaped, hyperbranched and dendritic (co)polymers of various monomer compositions and, consequently, having tailored properties such as heat resistance, scratch resistance, solvent resistance, etc.

More specifically, monomers suitable for ATRP include those of the formula R₃₁R₃₂C=C R₃₃R₃₄ wherein:
- R₃₁ and R₃₂ are independently selected from the group consisting of hydrogen, halogen, CN, CF₃, C₁₋₂₀ alkyl (preferably C₁₋₆ alkyl), α,β-unsaturated C₂₋₂₀ alkynyl (preferably acetylenyl), α,β-unsaturated C₂₋₂₀ alkenyl (preferably vinyl) optionally substituted (preferably at the α position) with a halogen, C₃₋₈ cycloalkyl, phenyl optionally bearing 1 to 5 substituents,
- R₃₃ and R₃₄ are independently selected from the group consisting of hydrogen, halogen (preferably fluorine or chlorine), C₁₋₆ alkyl and COOR₃₅ (where R₃₅ is selected from hydrogen, an alkali metal, or C₁₋₆ alkyl), and
- at least two of R₃₁ , R₃₂ , R₃₃ and R₃₄ are hydrogen or halogen.

Accordingly, vinyl heterocyclic monomers suitable for ATRP in this embodiment of the sixth aspect of the invention include, but are not limited to, 2-vinyl pyridine, 6-vinyl pyridine, 2-vinyl pyrrole, 5-vinyl pyrrole, 2-vinyl oxazole, 5-vinyl oxazole, 2-vinyl thiazole, 5-vinyl thiazole, 2-vinyl imidazole, 5-vinyl imidazole, 3-vinyl pyrazole, 5-vinyl pyrazole, 3-vinyl pyridazine, 6-vinyl pyridazine, 3-vinyl isoxazole, 3-vinyl isothiazoles, 2-vinyl pyrimidine, 4-vinyl pyrimidine, 6-vinyl pyrimidine, and any vinyl pyrazine, the most preferred being 2-vinyl pyridine.

Other preferred monomers include:
- (meth)acrylic esters of C₁₋₂₀ alcohols,
- acrylonitrile,
- cyanoacrylic esters of C₁₋₂₀ alcohols,
- didehydromalonate diesters of C₁₋₇ alcohols,
- vinyl ketones, and
- styrenes optionally bearing a C₁₋₇ alkyl group on the vinyl moiety (preferably at the α carbon atom) and/or bearing from 1 to 5 substituents on the phenyl ring, said substituents being selected from the group consisting of C₁₋₇ alkyl, C₁₋₇ alkenyl (preferably vinyl or allyl), C₁₋₇ alkynyl (preferably acetylenyl), C₁₋₇ alkoxy, halogen, nitro, carboxy, C₁₋₇ alkoxycarbonyl, hydroxy protected with a C₁₋₇ acyl group, cyano and phenyl.

The most preferred monomers for ATRP are methyl acrylate, methyl methacrylate, butyl acrylate, 2-ethylhexyl acrylate, acrylonitrile, maleimide and styrene.

In this embodiment, the catalytic component of the invention is more preferably used in combination with one or more initiators having a radically transferable atom or group, since an ATRP catalytic system is based on the reversible formation of growing radicals in a redox reaction between the metal component and an initiator. Suitable initiators may be selected from the group consisting of compounds having the general formula R₃₅R₃₆R₃₇CX₁, wherein:
- X₁ is selected from the group consisting of halogen, OR₃₈ (wherein R₃₈ is selected from the group consisting of C₁₋₂₀ alkyl, polyhalo C₁₋₂₀ alkyl, C₂₋₂₀ alkynyl (preferably acetylenyl), C₂₋₂₀ alkenyl (preferably vinyl or allyl), phenyl optionally substituted with 1 to 5 substituents selected from the group consisting of halogen and C₁₋₇ alkyl, and phenyl-substituted C₁₋₇ alkyl), SR₃₉, OC(=O)R₃₉, OP(=O)R₃₉, OP(=O)(OR₃₉)₂, OP(=O)OR₃₉, O--N(R₃₉)₂ and S--C(=S)N(R₃₉)₂, wherein R₃₉ is aryl or C₁₋₂₀ alkyl, or where an N(R₃₉)₂ group is present in the said X₁, the two R₃₉ groups may be joined together to form a 5-, 6- or 7-membered heterocyclic ring (in accordance with the definition above), and
- R₃₅, R₃₆ and R₃₇ are each independently selected from the group consisting of hydrogen, halogen, C₁₋₂₀ alkyl (preferably C₁₋₇ alkyl), C₃₋₁₀ cycloalkyl, C(=O)R₄₀, (wherein R₄₀ is selected from the group consisting of C₁₋₂₀ alkyl, C₁₋₂₀ alkoxy, aryloxy or heteroaryloxy), C(=O)NR₄₁R₄₂ (wherein R₄₁ and R₄₂ are independently selected from the group consisting of hydrogen and C₁₋₂₀ alkyl or R₄₁ and R₄₂ may be joined together to form a 5-, 6- or 7-membered heterocyclic ring (in accordance with the definition above), COCI, OH, CN, C₂₋₂₀ alkenyl (preferably vinyl), C₂₋₂₀ alkynyl, oxiranyl, glycidyl, aryl, heteroaryl, arylalkyl and aryl-substituted C₂₋₂₀ alkenyl.

In the latter initiators X₁ is preferably bromo which provides both a higher reaction rate and a lower polymer polydispersity.

When an alkyl, cycloalkyl, or alkyl-substituted aryl group is selected for one of R₃₅, R₃₆ and R₃₇, the alkyl group may be further substituted with a group X₁ such as defined above, in particular a halogen atom. Thus, it is possible for the initiator to serve as a starting molecule for branched, comb-shaped or star-shaped (co)polymers of virtually any type or geometry. One example of such an initiator is a 2,2-bis(halomethyl)-1,3-dihalopropane (e.g. 2,2-bis(chloromethyl)-1,3-dichloropropane or 2,2-bis(bromomethyl)-1,3-dibromopropane), and a preferred example is where one of R₃₅, R₃₆ and R₃₇ is phenyl substituted with from 1 to 5 C₁₋₇ alkyl substituents, each of which may independently be further substituted with a group X₁, e.g. α,α'-dibromoxylene, hexakis(α-chloro- or α-bromomethyl)benzene.

Preferred initiators include 1-phenylethyl chloride and 1-phenylethyl bromide, chloroform, carbon tetrachloride, 2-chloropropionitrile and C₁₋₇ alkyl esters of a 2-halo-C₁₋₇ saturated monocarboxylic acid (such as 2-chloropropionic acid, 2-bromopropionic acid, 2-chloroisobutyric acid, 2-bromoisobutyric acid and the like). Another example of a suitable initiator is dimethyl-2-chloro-2,4,4-trimethylglutarate.

Any transition metal complex which can participate in a redox cycle with the initiator and dormant polymer chain, but which does not form a direct carbon-metal bond with the polymer chain, such as a complex of ruthenium, osmium, iron, molybdenum, tungsten, titanium, rhenium, technetium, lanthanum, copper, chromium, manganese, rhodium, vanadium, zinc, gold, silver, nickel or cobalt, is suitable for use in this embodiment of the invention. The amounts and relative molar proportions of the initiator and the transition metal complex of the invention are those which are typically effective to conduct ATRP. The molar proportion of the transition metal complex with respect to the initiator may be from 0.0001:1 to 10:1, preferably from 0.1:1 to 5:1, more preferably from 0.3:1 to 2:1, and most preferably from 0.9:1 to 1.1:1.

ATRP according to the invention may be conducted in the absence of a solvent, i.e. in bulk. However, when a solvent is used, suitable solvents include ethers, cyclic ethers, alkanes, cycloalkanes, aromatic hydrocarbons, halogenated hydrocarbons, acetonitrile, dimethylformamide and mixtures thereof , and supercritical solvents (such as CO₂). ATRP may also be conducted in accordance with known suspension, emulsion or precipitation methods. Suitable ethers include diethyl ether, ethyl propyl ether, dipropyl ether, methyl t-butyl ether, di-t-butyl ether, glyme (dimethoxyethane) diglyme (diethylene glycol dimethyl ether), etc. Suitable cyclic ethers include tetrahydrofuran and dioxane. Suitable alkanes include pentane, hexane, cyclohexane, octane and dodecane. Suitable aromatic hydrocarbons include benzene, toluene, o-xylene, m-xylene, p-xylene and cumene. Suitable halogenated hydrocarbons include dichloromethane, 1,2-dichloroethane and benzene substituted with 1 to 6 fluorine and/or chlorine atoms, although one should ensure that the selected halogenated hydrocarbon does not act as an initiator under the reaction conditions.

ATRP may also be conducted in the gas phase (e.g. by passing the gaseous monomer(s) over a bed of the catalytic system), in a sealed vessel or in an autoclave. (Co)polymerization may be conducted at a temperature from about 0°C to 160°C, preferably from about 60°C to 120°C. Typically, the average reaction time will be from about 30 minutes to 48 hours, more preferably from 1 to 24 hours. (Co)polymerization may be conducted at a pressure from about 0.1 to 100 atmospheres, preferably from 1 to 10 atmospheres.

According to another embodiment, ATRP may also be conducted in emulsion or suspension in a suspending medium for suspending the monomer(s) and while using the metal complex of the invention in combination with a surfactant, so as to form a (co)polymer emulsion or suspension. The suspending medium usually is an inorganic liquid, preferably water. When water or an aqueous mixture is selected as the suspending medium, it is preferable to use a cationic metal complex species as the catalytic component, the said cationic species being associated with an anion A as described hereinabove. In this embodiment of the invention, the weight ratio of the organic phase to the suspending medium is usually between 1:100 and 100:1, preferably between 1:10 and 10:1. If desired, the suspending medium may be buffered. Preferably the surfactant will be selected in order to control the stability of the emulsion, i.e. to form a stable emulsion.

In order to conduct polymerization in a heterogeneous medium (where the monomer/polymer is insoluble, or only slightly soluble, in the suspension medium, i.e. water or CO₂), the metal catalytic component should be at least partially soluble in the monomer/polymer. Thus, only when ligands are properly selected to allow the catalyst to meet this requirement, such as ligands containing long alkyl chains to increase catalyst solubility in hydrophobic monomers targeted for polymerization, is a successful, controlled ATRP polymerization obtained in the water-borne systems of this embodiment. From the above description of ligands coordinating the metal in the catalytically active metal complexes of the invention, those skilled in the art will be able to make a suitable selection.

A key component in the preparation of the stable emulsions of the present embodiment is the use of the surfactant to stabilize the initial monomer suspension/emulsion and growing polymer particles and to prevent unwanted coagulation/flocculation of the particles. In order to conduct ATRP in emulsion however, care should be taken to choose a surfactant which does not interfere with the catalytic component or dormant chain end. Suitable surfactants for this purpose include non-ionic, anionic, and cationic surfactants, with cationic and non-ionic surfactants being preferred in non-buffered solutions. Particularly preferred non-ionic surfactants include polyethylene glycol, polyoxyethylene oleyl ethers and polyoxythylene sorbitan monoalkyls. A preferred cationic surfactant is dodecyltrimethyl ammonium bromide. Regardless of the surfactant used, efficient stirring is preferred to obtain good dispersions or latexes.

The surfactant is usually present in a concentration of about 0.01% to 50% by weight based on the total weight of all components introduced into the polymerisation reactor, i.e. suspending medium, monomer(s), surfactant and catalytic system.

High solubility in the suspension medium is not a prerequisite for the initiator as may be demonstrated by the use of the poorly water soluble ethyl 2-bromoisobutyrate, to initiate emulsion polymerization. While any order of addition of the initiator and other reaction components can be used, however if the initiator is added to a pre-emulsified reaction mixture, stable latexes are usually obtained. Suitable initiators have been described herein-above in the solvent embodiment of the ATRP process. Initiators can also be macromolecules that contain radically transferable atoms or groups. A special type of such macroinitiators may be water-soluble or even amphiphilic and may, after initiation of the reaction, be incorporated into the polymer particle and may stabilize the growing particle due to the hydrophilic segment of the macroinitiator.

After completing the (co)polymerization step of the ATRP process of this invention, the polymer formed may be isolated by known procedures, such as, but not limited to, precipitating in a suitable solvent, filtering the precipitated polymer, then washing and drying the filtered polymer. Precipitation can be typically conducted using a suitable alkane or cycloalkane solvent, such as pentane hexane, heptane, cyclohexane or mineral spirits, or using an alcohol, such as methanol, ethanol or isopropanol, or any mixture of suitable solvents. The precipitated (co)polymer can be filtered by gravity or by vacuum filtration, e.g. using a Buchner funnel and an aspirator. The polymer can then be washed with the solvent used to precipitate the polymer, if desired. The steps of precipitating, filtering and washing may be repeated, as desired. Once isolated, the (co)polymer may be dried by drawing air through the (co)polymer, by vacuum. The dried (co)polymer can then be analyzed and/or characterized e.g. by size exclusion chromatography or NMR spectroscopy.

(Co)polymers produced by the catalytic ATRP process of the invention may be useful in general as molding materials (e.g. polystyrene) and as barrier or surface materials (e.g. polymethyl methacrylate). However, since they typically have more uniform properties (in particular molecular weight distribution) than polymers produced by conventional radical polymerization, they will be most suitable for use for specialized applications. For example, block copolymers of polystyrene (PSt) and polyacrylate (PA), e.g. PSt-PA-PSt triblock copolymers, are useful thermoplastic elastomers. Polymethylmethacrylate/acrylate triblock copolymers (e.g. PMMA-PA-PMMA) are useful, fully acrylic, thermoplastic elastomers. Homo- and copolymers of styrene, (meth)acrylates and/or acrylonitrile are useful plastics, elastomers and adhesives. Either block or random copolymers of styrene and a (meth)acrylate or acrylonitrile are useful thermoplastic elastomers having high solvent resistance. Furthermore, block copolymers in which blocks alternate between polar monomers and non-polar monomers produced by the present invention are useful amphiphilic surfactants or dispersants for making highly uniform polymer blends. Star-shaped (co)polymers, e.g. styrene-butadiene star block copolymers, are useful as having high impact resistance.

(Co)polymers produced by the catalytic ATRP process of the present invention typically have a number average molecular weight from about 5,000 to 1,000,000, preferably from about 10,000 to 250,000, and more preferably from about 25,000 to 150,000.

Because ATRP is a living polymerization process, it can be started and stopped practically at will. Further, the polymer product retains the functional group X₁ necessary to initiate a further polymerization. Thus, in a specific embodiment, once a first monomer is consumed in the initial polymerizing step, a second monomer can then be added to form a second block on the growing polymer chain in a second polymerizing step. Further additional polymerizations with the same or different monomer(s) can be performed to prepare multi-block copolymers. Furthermore, since ATRP is also a radical polymerization, these blocks can be prepared in essentially any order.

(Co)polymers produced by the catalytic ATRP process of the present invention have a very low polydispersity index, i.e. the ratio M_{w}/Mₙ of their weight average molecular weight to their number average molecular weight is typically from about 1.1 to 2.4, preferably from 1.15 to 2.0, more preferably from 1.2 to 1.6.

Because the living (co)polymer chains retain an initiator fragment including X₁ as a terminal group, or in one embodiment as a substituent in a monomeric unit of the polymer chain, they may be considered as end-functional or in-chain functional (co)polymers. Such (co)polymers may thus be converted into (co)polymers having other functional groups (e.g. halogen can be converted into hydroxy or amino by known methods, and nitrile or carboxylic ester can be hydrolyzed to a carboxylic acid by known methods) for further reactions, including crosslinking, chain extension with reactive monomers (e.g. to form long-chain polyamides, polyurethanes and/or polyesters), reactive injection molding, and the like.

In order to facilitate the use of metal complexes of the invention in the above-mentioned heterogeneous catalytic reactions, the present invention further provides silyl derivatives of such complexes, being suitable for covalent bonding to a carrier, especially those complexes wherein the multidentate ligand is a bidentate or tridentate Schiff base, e.g. one having the general formula (IA) or (IB) referred to in figure 1, or a tetradentate ligand comprising two Schiff bases such as one having the general formula (IIA) or (IIB) or (IIC) referred to in figure 2 or the general formula (IIIA) or (IIIB) referred to in figure 3. In such silyl derivatives, R' and/or R" of the said general formula is replaced or substituted with a group having the formula:

-R₂₀-(CH₂)ₙ-D-Si-R₂₁R₂₂R₂₃ (VIII),

wherein:
- R₂₀ is a radical selected from the group consisting of C₁₋₇ alkylene, arylene, heteroarylene and C₃₋₁₀ cycloalkylene, the said radical being optionally substituted with one or more R₂₄ substituents each independently selected from the group consisting of C₁₋₂₀ alkyl, C₂₋₂₀ alkenyl, C₂₋₂₀ alkynyl, C₁₋₂₀ carboxylate, C₁₋₂₀ alkoxy, C₂₋₂₀ alkenyloxy, C₂₋₂₀ alkynyloxy, C₂₋₂₀ alkoxycarbonyl, C₁₋₂₀ alkylsulfonyl, C₁₋₂₀ alkynylsulfinyl, C₁₋₂₀ alkylthio, aryloxy and aryl;
- D is a divalent atom or radical selected from the group consisting of oxygen, sulphur, silicon, arylene, methylene, CHR₂₄, C(R₂₄)₂, NH, NR₂₄ and PR₂₄;
- R₂₁, R₂₂ and R₂₃ are each independently selected from the group consisting of hydrogen, halogen and R₂₄; and
- n is an integer from 1 to 20;
provided that at least one of R₂₁, R₂₂ and R₂₃ is selected from the group consisting of C₁₋₂₀ alkoxy, C₂₋₂₀ alkenyloxy, C₂₋₂₀ alkynyloxy, C₂₋₂₀ alkoxycarbonyl, C₁₋₂₀ alkylsulfonyl, C₁₋₂₀ alkynylsulfinyl, C₁₋₂₀ alkylthio and aryloxy.

More preferred within the above group are silyl derivatives wherein R' is replaced or substituted with a 3-(triethoxysilyl)propyl or 2-(triethoxysilyl)ethyl group. Alternatively suitable derivatives include shaped organosiloxane copolycondensation products such as disclosed in EP-A-484,755.

In another embodiment, the invention provides a supported catalyst, especially for use in the above-mentioned catalytic reactions, comprising the product of covalent bonding of (a) a silyl derivative of a metal complex such as defined hereinabove, and (b) a carrier including one or more inorganic oxides or an organic polymeric material. Preferably the said inorganic carrier is selected from silica, alumino-silica, zirconia, natural and synthetic zeolites and mixtures thereof, or the said organic polymeric carrier is a polystyrene resin or a derivative thereof wherein the aromatic ring is substituted with one or more groups selected from C₁₋₇ alkyl, C₃₋₁₀ cycloalkyl, aryl and heteroaryl. More detailed examples of suitable carriers (b) for this purpose were already disclosed with respect to the fifth aspect of the invention.

The catalytic component of the sixth aspect of this invention is also useful in the cyclopropanation of ethylenically unsaturated compounds, or the intramolecular cyclopropanation of α-diazoketones or α-diazo-β-ketoesters for producing compounds having one or more cyclopropane structural units in the hydrocarbon chain. This embodiment of the invention is thus useful in one or more manufacturing steps of the following natural and synthetic cyclopropyl-containing compounds. Cyclopropyl-containing compounds may be found in naturally occurring terpenes, steroids, amino-acids, fatty acids, alkaloids and nucleic acids. For instance, chrysanthemic acid derivatives (such as pyrethrines) produced in plants are precursors to potent insecticides. The invention is also applicable to making synthetic pyrethroid insecticides such as deltamethrin, as well as sirenine, aristolon, sesquicarene and cyclopropyl derivatives being intermediates in the synthesis of the steroid hirsutene or the antibiotic sarkomycine. Cyclopropyl-containing non-natural compounds also have biological activity, such as Cipro, a powerful anti-anthrax drug, or cyclopropane amino-acids (e.g. 2,3-methanophenylalanine, the anti-Parkinson drug 2,3-methano-*m*-tyrosine, coronatine and coronamic acid). Polycyclopropane fatty acid derivatives isolated from fungi, U-106305 (a cholesteryl ester transfer protein inhibitor) and FR-900848 (a nucleoside analogue), are also candidates for such synthetic production. Ethylenically unsaturated compounds that may be cyclopropanated according to this invention into the correspondingly cyclopropyl-containing compounds are not particularly limited but include, without restriction, compounds having terminal ethylenic unsaturation such as styrene (which, in the presence of ethyl diazoacetate, may be transformed into ethyl-2-phenylcyclopropanecarboxylate) and substituted derivatives thereof (e.g. 4-chlorostyrene, α-methylstyrene and vinylstyrene), 2-vinylnaphtalene, 1,1-diphenylethylene, 1-decene, functional α-olefins wherein the functional group is preferably adjacent to the ethylenic unsaturation and is preferably a protected alcohol such as in protected allylic alcohols such as acyclic allylic silyl ethers (which may be transformed into cyclopropylcarbinyl silyl ethers) or a carboxy group such as in acrylic and methacrylic acids (as well as esters, thioesters, amides or anhydrides thereof), cinnamate esters, alkenylboronic esters (such as 2-methylethenyl-4,5-bis[methoxy-diphenylmethyl]-1,3,2-dioxaborolanes or deriva-tives thereof wherein the methyl group is protected by a protecting group such as, but not limited to, *ter*-butyldimethylsiloxy, *ter*-butyldiphenylsiloxy, benzyloxy, methoxymethoxy or benzoyloxy, which may be transformed into the corresponding cyclopropylboronic esters), 2-phenylsulfonyl-1,3-dienes and cycloolefins such as cyclooctene. Such reaction preferably takes place in the presence of a diazo compound such as, but not limited to, ethyl diazoacetate, cinnamyl diazoacetate, dicyclohexylmethyl diazoacetate, vinyl diazoacetate, menthyl diazoacetate or 1-diazo-6-methyl-5-hepten-2-one, at moderate temperatures usually ranging from about 0°C to 80°C, preferably 20 to 60°C, the reaction time ranging from about 1 to 12 hours, and in a relatively low boiling solvent such as methylene chloride, tetrahydrofuran, ethanol, isopropanol, tert-butanol, L-menthol or water, or mixtures thereof. The diazo compound may be added as such or, in order to eliminate the handling risks associated with its explosive nature, may be generated *in situ* by reacting an acetoammonium salt with sodium nitrite in the presence of the ethylenically unsaturated compound. When water or an aqueous mixture is selected as the reaction solvent, it is preferable to use a cationic metal complex species as the catalytic component, the said cationic species being associated with an anion A as described hereinabove. Preferably the molar ratio of the ethylenically unsaturated compound to the catalytic component is in a range from 200 to 2,000, more preferably from 250 to 1,500. The molar ratio of the ethylenically unsaturated compound with respect to the diazo compound is conventional for this kind of reaction, i.e. a molar excess of the former compound. The cyclopropanation of ethylenically unsaturated compounds may optionally be carried out in the presence of a tertiary aliphatic amine, such as triethylamine or tri-n-butylamine, or a heterocyclic amine such as pyridine or lutidine as a co-catalyst. The intramolecular cyclopropanation of α-diazo carbonyl compounds such as α-diazo ketones or α-diazo-β-ketoesters may also be performed acccording to similar reaction conditions (temperature, reaction time, substrate/ catalyst ratio) and may result in bicyclic molecules wherein the cyclopropyl group may be fused to another cycloaliphatic group, e.g. a cyclopentanone such as in the synthesis of intermediates of hirsutene or sarkomycin, or a cyclopentyl group when starting from acetylenic α-diazo ketones. However, it should be noted that, in accordance with the teachings of Padwa in Molecules (2001) 6:1-12, the cyclisation of an acetylenic α-diazo ketone in the presence of a catalytic component of this invention may also lead to the formation of other polycyclic ring systems such as, but not limited to, cyclopentanone fused to a furan, an alkenyl-substituted indenone, a cyclopropyl-substituted indenone, a cyclopentazulenone or a cyclopentadiene fused to indenone.

The catalytic component of the sixth aspect of this invention is also useful in the cyclopropenation of alkynes for producing compounds having one or more cyclopropene structural units in the hydrocarbon chain. This applies in particular to alkynes having a C₂₋₇ alkynyl group such as, but not limited to, 1-hexyne, 3,3-dimethyl-1-butyne, phenylacetylene, cyclohexylacetylene, methoxy-methylacetylene and acetoxymethylacetylene which may be converted in good yields into ethylcyclopropene-3-carboxylates in the presence of a diazo compound such as, but not limited to, ethyl diazoacetate, cinnamyl diazoacetate, dicyclohexylmethyl diazoacetate, vinyl diazoacetate, 1-diazo-6-methyl-5-hepten-2-one or menthyl diazoacetate. It is also useful in the intramolecular cyclopropenation of acetylenic α-diazo ketones, leading for instance to cyclopropenyl-containing compounds such as cyclopropenyl substituted indenones.

The catalytic component of the sixth aspect of this invention is also useful in quinoline synthesis through oxidative cyclisation of 2-aminobenzyl alcohol with ketones (i.e. the so-called Friedlaender reaction). Such reaction preferably takes place with a molar excess of the said ketone, under basic conditions (such as in the presence of an alkali hydroxide), at moderate temperatures usually ranging from about 20 to about 100°C and optionally in the presence of a solvent. Preferably the ratio of the 2-aminobenzyl alcohol to the catalytic component is in a range from 100 to 2,000, preferably from 200 to about 1,000. A number of alkylarylketones, alkyl heteroarylketones, dialkylketones and benzo-fused cyclic ketones may be used in this process of the invention, including C₁₋₇alkylketones wherein the second hydrocarbon attached to the oxo group may be methyl, pentyl, isopropyl, phenethyl, phenyl, toluyl, anisyl, nitrophenyl, hydroxyphenyl, fluorophenyl, trifluoromethylphenyl, cyanophenyl, naphtyl, furanyl, thiophenyl, pyridyl, and the like. Exemplary ketones which may be cyclised to quinolines according to this embodiment of the invention include, but are not limited to, acetophenone, 3-methylacetophenone, cyclohexanone, 4-phenylcyclohexanone and propiophenone. Other suitable ketones for this purpose are as disclosed by Cho et al. in Chem. Commun. (2001) 2576-2577. Unexpectedly, some ketones such as cyclohexanone may be converted into the corresponding quinoline with a yield significantly higher, under equivalent reaction conditions, than achieved by the ruthenium catalyst used in the latter publication.

The catalytic component of the sixth aspect of this invention is also useful in the intramolecular epoxidation, including the asymmetric epoxidation, of ethylenically unsaturated compounds, i.e. alkenes, for producing the corresponding epoxides (i.e. oxacyclopropyl-containing compounds). Such alkenes include for instance, but without limitation, styrene and analogues thereof (such as α-methylstyrene, p-chorostyrene, p-trifluoromethylstyrene and the like) or cholesterol acetate. Illustrative olefinic starting reactants useful in the asymmetric epoxidation of this invention include those which can be terminally or internally unsaturated and be of straight chain, branched chain, or cyclic structure. Such olefinic reactants may contain from 3 to about 40 carbon atoms and may contain one or more ethylenically unsaturated groups. Moreover, such olefinic reactants may contain groups or substituents which do not essentially adversely interfere with the asymmetric epoxidation process such as carbonyl, carbonyloxy, oxy, hydroxy, oxycarbonyl, halogen, alkoxy, aryl, haloalkyl, and the like. Illustrative olefinic unsaturated compounds include substituted and unsubstituted alpha-olefins, internal olefins, alkyl alkenoates, alkenyl alkanoates, alkenyl alkyl ethers, alkenols, and the like, e.g. propylene, 1-butene, 1-pentene, 1-hexene, 1-octene, 1-decene, 1-dodecene, 1-octadecene, 2-butene, isoamylene, 2-pentene, 2-hexene, 3-hexene, 2-heptene, cyclohexene, 2-ethylhexene, 3-phenyl-1-propene, 1,4-hexadiene, 1,7-octadiene, 1,5,9-dodecatriene, 3-cyclohexyl-1-butene, allyl alcohol, hex-1-en-4-ol, oct-1-en-4-ol, vinyl acetate, allyl acetate, aryloates such as vinyl benzoate and the like, 3-butenyl acetate, vinyl propionate, allyl propionate, allyl butyrate, methyl methacrylate, 3-butenyl acetate, vinyl ethyl ether, vinyl methyl ether, allyl ethyl ether, n-propyl-7-octenoate, substituted and unsubstituted chromenes, 2,2-dimethylcyclochromene, 3-butenenitrile, 5-hexenamide, indene, 1,2-dihydronaphthalene, 2-vinylnaphtalene, norbornene, cis-stilbene, trans-stilbene, p-isobutylstyrene, 2-vinyl-6-methoxy-naphthylene, 3-ethenylphenyl phenylketone, 4-ethylphenyl-2-thienylketone, 4-ethenyl-2-fluoro-biphenyl, 4-(1m,3-dihydro-1-oxo-2H-isoindol-2-yl) styrene, 2-ethyl-5-benzoylthiophene , 3-ethenyl-phenyl phenylether, isobutyl-4-propenylbenzene, phenyl vinyl ether, 2-cyclohenenyl-1,1-dioxolane, vinyl chloride, benzopyrane and benzofurane type compounds, and substituted aryl ethylenes such as described in U.S. Pat. No. 4,329,507, incorporated herein by reference in its entirety. Epoxidation of the invention may be applied to the synthesis of biologically active molecules such as cis-stilbene oxide (a substrate for microsomal and cytosolic epoxide hydrolase) and isoprostane.

Such epoxidation reaction preferably takes place in the presence of an at least stoechiometric amount (with respect to the ethylenically unsaturated compound) of an oxygen atom source or oxygen-transfer reagent being relatively unreactive toward olefins in the absence of the catalytic system under the prevailing (e.g. temperature and pressure) conditions. The said oxygen atom source or oxygen-transfer reagent may be, but without limitation, selected from the group consisting of H₂O₂ (hydrogen peroxide), NaOCl, iodosylmesitylene, NalO₄, NBu₄lO₄, potassium peroxymonosulfate, magnesium monoperoxyphthalate, 2,6-dichloropyridine N-oxide and hexacyanoferrate ion. Mixtures of such oxygen atom sources or oxygen-transfer reagents may also be used. Such epoxidation reaction preferably takes place under conditions and for such a time as is needed to epoxidize the olefinic unsaturated compound. Such conditions include, but without limitation:
- reaction temperatures usually ranging from about -20° C to about 120° C, preferably from 0° C to 90° C, more preferably from 20 to about 40°C, and/or
- reaction pressures ranging from about 0.1 to about 70 bars, and/or
- conducting the reaction in the presence of a solvent for the catalytic system, preferably a relatively low boiling organic solvent selected from the group consisting of saturated alcohols, amines, alkanes, ethers, esters, aromatics and the like, and/or
- a molar ratio of the ethylenically unsaturated compound to the catalytic component in a range from about 200 to about 20,000, preferably from 500 to 10,000, and/or
- a molar excess of the oxygen-transfer reagent with respect to the olefinic unsaturated compound.

The catalytic component of the sixth aspect of this invention is also useful in the oxidation of hydrocarbons into alcohols such as, but not limited to, the oxidation of methane (which is known to be more difficult to oxidize than other alkanes) into methanol. Although this process is effective for a wide variety of hydrocarbons, it is particularly effective for the oxidation of straight chain and branched chain alkanes and cycloalkanes with 1 to 15 carbon atoms, and arylalkanes such as toluene, xylene and ethylbenzene. The preferred aliphatic hydrocarbons have 1 to 10 carbon atoms, including ethane, propane, butane, isobutane, hexanes, and heptanes; and the preferred cyclic hydrocarbons have 5 to 10 carbon atoms such as cyclopentane, cyclohexane, cycloheptane, cyclooctane and adamantane. This invention is also applicable to a broad range of hydrocarbons containing various substituents to enhance the rate of oxidation. Oxidation according to this invention may be carried out in a liquid phase, mixed solvent system such as water/acetone, water/acetonitrile and/or acetic acid, which is inert to the conditions of the reaction and to oxidation by molecular oxygen. The temperature can range between 20 and 60 °C. The pressure may range from 5 to 20 atmospheres. Depending upon whether the hydrocarbon is a solid, liquid or gas, it is either dissolved in the mixed solvent system or is bubbled through the solvent together with air or oxygen before adding the catalytic component of the invention. A concentration ranging from 10⁻³ to 10⁻⁶ moles of the catalytic component in solution is usually sufficient to achieve the desired oxidation. The reaction time preferably ranges from 30 minutes to 30 hours, more preferably from 1 to 5 hours. According to another embodiment, the catalytic component of the sixth aspect of this invention is also useful in the oxidation of allylic and benzylic alcohols into carbonyl compounds.

The sixth aspect of the present invention also relates to other atom or group transfer reactions such as asymmetric syntheses in which a prochiral or chiral compound is reacted in the presence of an optically active, metal-ligand complex catalyst, in enantiomerically active form, to produce an optically active compound. These reactions, which are useful for the production of numerous classes of products, e.g. sulfoxides, aziridines, enol esters, nitriles, silanes, silyl ethers, alkanes, phosphonates, alkylboranes, hydroxycarbonyl compounds, β-cyano carbonyl compounds, carboxyl compounds, arylalkenes, heteroarylalkenes, cyclohexenes, 7-oxanorbornenes, aldehydes, alcohols, primary or secondary amines, amides and the like, have been listed hereinbefore and will be detailed below.

For instance, the catalytic oxidation of sulfides (into sulfoxides and sulfones), phosphines (into phosphonates), and alcohols or aldehydes into carboxylic acids can be carried out in accordance with conventional oxidation procedures known in the art. For example, but without limitation, optically active carboxylic acids can be prepared by reacting a racemic aldehyde and an oxygen atom source in the presence of an optically active metal complex catalytic system as described herein. A number of sulfoxides finding application in the pharmaceutical industry, such as a quinolone sulfoxide described by Matsugi et al. in Tetrahedron (2001) 57:2739 (a platelet adhesion inhibitor), or a pyrazolotriazine sulfoxide described by Naito et al. in Yakugaku Zasshi (2001) 121:989 (a drug for the treatment of hyperurecemia and ischemic reperfusion injury), or methylphenyl sulfoxide (from methylphenyl thioether) may be made by using such a process step.

Catalytic hydrocyanation (or cyanohydration) of α-ethylenically unsaturated compounds for producing saturated nitriles, or alkynes for producing unsaturated nitriles, or α,β-unsaturated aldehydes or ketones for producing β-cyano carbonyl compounds can be carried out in accordance with conventional procedures known in the art. For example, 1-phenyl propenone may be transformed into 4-oxo-4-phenyl-butanenitrile, or optically active nitrile compounds can be prepared by reacting a prochiral olefin and hydrogen cyanide in the presence of an optically active metal complex catalytic system as described herein.

Catalytic hydrosilylation of olefins for producing saturated silanes, or alkynes for producing unsaturated silanes, or ketones for producing silyl ethers, or trialkylsilylcyanation of aldehydes (e.g. benzaldehyde) for producing cyanohydrin trialkylsilyl ethers (which may afterwards be hydrolysed into cyanohydrins) can be carried out in accordance with conventional procedures known in the art. For example, optically active silanes or silyl ethers can be prepared by reacting a prochiral olefin or ketone or aldehyde together with a suitable silyl compound under conventional hydrosilylation conditions in the presence of an optically active metal complex catalytic system described herein.

Catalytic aziridination of imines or alkenes for producing organic compounds having one or more aziridine structural units can be carried out in accordance with conventional procedures known in the art. For example, prochiral olefins can be converted to optically active aziridines under conventional aziridanation conditions in the presence of an optically active metal complex catalytic system as described herein.

Catalytic hydroamidation of olefins for producing saturated amides can be carried out in accordance with conventional procedures known in the art. For example, optically active amides can be prepared by reacting a prochiral olefin, carbon monoxide, and a primary or secondary amine or ammonia under conventional hydroamidation conditions in the presence of an optically active metal complex catalytic system as described herein.

Catalytic hydrogenation of olefins into alkanes, or ketones into alcohols can be carried out in accordance with conventional procedures known in the art. For example, a ketone can be converted to an optically active alcohol under conventional hydrogenation conditions in the presence of an optically active metal complex catalytic system as described herein. Substrates that can be hydrogenated in accordance with this embodiment of the invention include, but are not limited to, α-(acylamino) acrylic acids (thus enantioselectively providing chiral amino-acids), α-acetamidocinnamic acid, α-benzamidocinnamic acid, dehydroamino acid derivatives and methyl esters thereof, imines, β-ketoesters (such as methyl acetylacetate) and ketones.

Catalytic aminolysis of olefins for producing saturated primary or secondary amines can be carried out in accordance with conventional procedures known in the art. For example, optically active amines can be prepared by reacting a prochiral olefin with a primary or secondary amine under conventional aminolysis conditions in the presence of an optically active metal complex catalytic system as described herein.

Catalytic isomerization of alcohols, preferably allylic alcohols, for producing aldehydes can be carried out in accordance with conventional procedures known in the art. For example, allylic alcohols can be isomerized under conventional isomerization conditions to produce optically active aldehydes in the presence of an optically active metal complex catalytic system described herein.

Catalytic Grignard cross coupling of alkyl or aryl halides for producing alkanes or arylalkanes can be carried out in accordance with conventional procedures known in the art. For example, optically active alkanes or arylalkanes can be prepared by reacting a chiral Grignard reagent with an alkyl or aryl halide under conventional Grignard cross coupling conditions in the presence of an optically active metal complex catalytic system as described herein.

Catalytic hydroboration of olefins (such as, but not limited to, 4-methyl-1-pentene) for producing alkylboranes and trialkylboranes (which may then be oxidised or hydrolysed into alcohols) can be carried out in accordance with conventional procedures known in the art. For example, optically active alkyl boranes or alcohols can be prepared by reacting a prochiral olefin and a borane under conventional hydroboration conditions in the presence of an optically active metal complex catalytic system as described herein.

Catalytic hydride reduction of aldehydes and ketones for producing alcohols can be carried out in accordance with conventional procedures known in the art, i.e. by treating the said aldehyde or ketone with a hydride reagent such as sodium borohydride or alithium aluminum hydride. For example, pentanal may be reduced into 1-pentanol, cyclobutanone into cyclobutanol, and cyclohexane-1,4-dione into 1,4-cyclohexanediol.

Catalytic aldol condensation of saturated carboxyl compounds (aldehydes or ketones) for producing α,β-unsaturated carboxyl compounds or β-hydroxycarbonyl compounds, and intra-molecular aldol condensation of dialdehydes or diones for producing cyclic α,β-unsaturated carboxyl compounds (aldehydes or ketones) can be carried out in accordance with conventional procedures known in the art. For example, optically active aldols can be prepared by reacting a prochiral ketone or aldehyde and a protected enol such as a silyl enol ether under conventional aldol condensation conditions in the presence of an optically active metal complex catalytic system as described herein.

Catalytic codimerization of alkenes for producing higher saturated hydrocarbons or alkynes for producing higher alkenes can be carried out in accordance with conventional procedures known in the art. For example, optically active hydrocarbons can be prepared by reacting a prochiral alkene and another alkene under codimerization conditions in the presence of an optically active metal complex catalytic system as described herein.

Catalytic alkylation, preferably allylic alkylation, of ketones for producing alkylated ketones, preferably allylic ketones, can be carried out in accordance with conventional procedures known in the art in the presence of a metal complex catalytic system as described herein. Similarly, 1,3-diphenyl-2-propenyl acetate may be alkylated with a nucleophile such as CH₂(CO₂CH₃)₂ in the presence of the catalytic component of the invention.

Catalytic Diels-Alder reactions such as, but not limited to, the cycloaddition of a conjugated diene onto an α-ethylenically unsaturated compound for producing optionally substituted cyclohexenes, or the cycloaddition of furan onto an α-ethylenically unsaturated compound for producing optionally substituted 7-oxanorbornenes can be carried out in accordance with conventional procedures known in the art in the presence of a metal complex catalytic system as described herein.

Catalytic Michael addition of a ketone or a β-dicarbonyl compound onto an α,β-unsaturated carboxyl compound for producing saturated polycarboxyl compounds can be carried out in accordance with conventional procedures known in the art in the presence of a metal complex catalytic system as described herein, i.e. for example an enolate ion may undergo conjugate addition to an α,β-unsaturated aldehyde or ketone, such as for example the addition of acrolein onto 2,4-pentanedione (acetylacetone) or 2-methylcyclohexanone. With some Michael acceptors, such as 3-buten-2-one, the products of the initial addition are capable of a subsequent intramolecular aldol condensation, the so-called Robinson annulation, e.g. the addition of 3-buten-2-one onto 2-methylcyclohexanone.

Catalytic Heck reactions can be carried out in accordance with conventional procedures known in the art in the presence of a metal complex catalytic system as described herein. The standard Heck reaction, especially with the metal of the catalytic component being palladium, involves the reaction of an aryl or heteroaryl halide, e.g. 3-bromoquinoline, with an alkene, commonly an acrylate. An oxidative variant of the Heck reaction proceeds from certain heterocyclic compounds such as indoles, furans and thiophenes such as, but not limited to, N-acetyl-3-methylindole. A reductive variant of the Heck reaction proceeds from certain 3-acylpyridines, 4-acylpyridines and acylindoles, e.g. the reaction of 3-acetylpyridine with triethoxysilylethylene.

Catalytic hydroamination of olefins and alkynes for producing amines can be carried out in accordance with conventional procedures known in the art in the presence of a metal complex catalytic system as described herein. This type or reaction is useful namely for the direct amination of common feedstocks such as ethylene with ammonia for producing ethylamine, and for the addition of aromatic and aliphatic amines to dienes, and for the addition of aromatic amines to vinylarenes.

The permissible prochiral and chiral starting material reactants encompassed by the processes of this invention are, of course, chosen depending on the particular synthesis and product desired. Such starting materials are well known in the art and can be used in conventional amounts in accordance with conventional methods. Illustrative starting material reactants include, for example, aldehydes (e.g. for intramolecular hydroacylation, aldol condensation, and oxidation into acids), prochiral olefins (e.g. for epoxidation, hydrocyanation, hydrosilylation, aziridination, hydroamidation, aminolysis, cyclopropanation, hydroboration, Diels-Alder reaction, hydroamination and codimerization), ketones (e.g. for hydrogenation, hydrosilylation, aldol condensation, Robinson annulation, transfer hydrogenation and allylic alkylation), alkynes (e.g. for cyclopropenation and hydroamination), epoxides (e.g. for hydrocyanation or nucleophilic ring opening reaction), alcohols (e.g. for carbonylation), aryl halides (e.g. for decarbonylation and Heck reactions), and chiral Grignard reagents (e.g. for Grignard cross coupling).

The present invention will now be further explained by reference to the following set of examples which should be understood as merely illustrating various embodiments of the invention without limiting the scope thereof.

### EXAMPLES 1-A to 1-E - preparation of Schiff base ligands

Schiff base ligands were prepared and purified as follows. Condensation of a salicylaldehyde (10 mmole) with a suitably substituted aniline was carried out with stirring in 40 ml methanol at reflux temperature during 4 hours. After cooling at -18°C for 24 hours, the crystals formed were filtered and washed with cold ethanol, then dried in vacuo at 40°C during 4 hours to afford with the following yields the desired salicylaldimine ligands. Each ligand (formula given hereunder) was characterized by means of proton nuclear magnetic resonance (hereinafter referred as NMR, performed at 300 MHz with C₆D₆ at 25°C), carbon NMR (performed at 75 MHz with C₆D₆) and infrared spectrophotometry (IR, performed with CCl₄), as follows:
- N-(2,6-diisopropylphenyl)-2-hydroxy-3-tertbutyl-1-phenylmethaneimine (Schiff base 1-A) obtained (yellow-orange oil, 2.9 g, yield 87 %) from 1.71 ml 3-*tert*-butyl-2-hydroxy-benzaldehyde and 1.88 ml 2,6-diisopropylaniline. 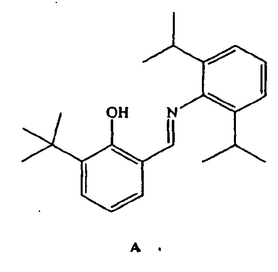 ¹H-NMR: δ 12.24 (s, 1 H), 9.19 (s, 1H), 7.34-6.67 (m, 6H), 2.96 (sept, 2H), 1.56 (s, 9H) and 1.31 (d, 12H) ppm; ¹³C-NMR: δ 167.4, 160.3, 146.0, 138.8, 137.5, 130.4, 125.3, 123.0, 118.6, 118.3, 109.2, 34.9, 28.0 and 23.2 ppm; IR: 3451 (OH), 3056, 2962 (tBu), 2927, 2870, 1626 (C=N), 1579, 1494, 1437, 1396, 1385, 1359, 1318, 1278, 1109, 1060, 906, 844, 813, 781, 759, 741, 701, 560 and 462 cm⁻¹.
- N-(4-bromo-2,6-dimethyl)-2-hydroxy-3-tertbutyl-1-phenylmethaneimine (Schiff base 1-B) obtained (yellow oil, 2.8 g, yield 79 %) from 1.71 ml 3-*tert*-butyl-2-hydroxybenzaldehyde and 2 g 4-bromo-2,6-dimethylaniline. 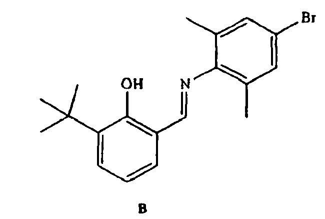 ¹H-NMR: δ 12.35 (s, 1H), 8.3 (s, 1H), 7.45 (d, 1H), 7.25 (s, 2H), 7.18 (d, 1H), 6.9 (t, 1H), 2.15 (s, 6H) and 1.6 (s, 9H) ppm; ¹³C-NMR: δ 168.2, 161.3, 147.3, 139.0, 138.0, 130.9, 126.3, 123.9, 118.7, 118.0, 110.1, 35.1, 28.5, 23.8 ppm; IR: 3450 (OH), 3057, 2964 (tBu), 2928, 2888, 1627 (C=N), 1578, 1496, 1438, 1386, 1360, 1320, 1280, 1212, 1174, 1109, 1097 1061, 989.7, 844, 813, 800, 782, 759, 739, 701, 623, 560, 534, 462 and 418 cm⁻¹.
- N-(4-bromo-2,6-dimethylphenyl)-2-hydroxy-1-phenylmethaneimine (Schiff base 1-C) obtained (yellow powder, 2.83 g, yield 93 %) from 1.065 ml salicylaldehyde and 2 g 4-bromo-2,6-dimethylaniline. 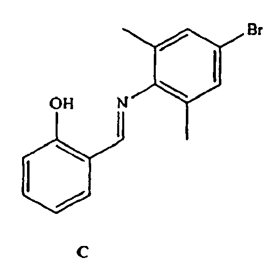 ¹H-NMR: δ 12.85 (S, 1H), 8.32 (s, 1H), 7.30-7.15 (m, 6H) and 2.21 (s, 6H) ppm; ¹³C-NMR: δ167.0, 160.9, 148.3, 138.9, 133.4, 132.1, 130.8, 130.3, 119.0, 117.6, 117.2 and 19.0 ppm; IR: 3350, 3065, 3031, 2942, 2930, 2860, 1620 (C=N), 1570, 1526, 1489, 1461 and 1109 cm⁻¹.
- N-(4-bromo-2,6-dimethylphenyl)-2-hydroxy-4-nitro-1-phenylmethaneimine (Schiff base 1-D) obtained as a dark yellow powder from 1.67 g 4-hydroxy-3-nitrobenzaldehyde and 2 g 4-bromo-2,6-dimethylaniline. 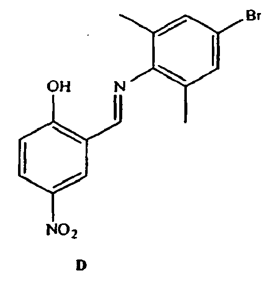 ¹H-NMR: □ 13.96 (s, 1H), 8.41 (s, 1H), 8.35 (d, 1H), 8.30 (d, 1H), 7.28 (s, 2H), 7.13 (d, 1 H) and 2.19 (s, 6H) ppm; ¹³C-NMR: δ 166.4, 165.5, 145.6, 139.8, 132.0, 130.4, 128.7, 128.5, 118.6, 118.3, 117.1, and 18.1 ppm; IR: 3459 (OH), 3086, 3059, 2987, 2967, 2932, 1619 (C=N), 1581, 1523, 1480, 1458, 1340 (NO₂), 1300, 1177, 1095, 983, 937, 853, 832, 798, 772, 751, 731, 716, 659, 633, 567 and 464 cm⁻¹.
- N-(2,6-diisopropylphenyl)-2-hydroxy-4-nitro-1-phenylmethaneimine (Schiff base 1-E) obtained as a yellow powder from 1.065 ml salicylaldehyde and 1.88 ml 2,6-diisopropylaniline. 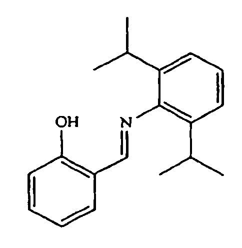 ¹H-NMR: □ 13.16 (s, 1 H), 8.34 (s, 1 H), 7.46 (d, 1 H), 7.40 (t, 1H), 7.22 (bs, 3H), 7.10 (d, 1 H), 6.99 (t, 1H), 3.20 (sept, 2H) and 1.20 (d, 12H) ppm; ¹³C-NMR: δ 166.4, 161.0, 145.9, 138.4, 133.0, 132.0, 125.3, 123.0, 118.8, 118.4, 117.1, 27.9 and 23.3 ppm; IR 3330 (OH), 3080, 3055, 2982, 2970, 2930, 1608 (C=N), 1581, 1520, 1477, 1454, 1323, 1301, 1170, 1090, 980, 935, 850, 835, 796, 770 and 751 cm⁻¹.

### EXAMPLES 2 to 8 - preparation of Schiff base substituted ruthenium complexes

Ruthenium complexes with Schiff bases from examples 1-A to 1-E were prepared in three steps and purified as follows. In a first step, to a solution in THF (15 ml) of the appropriate Schiff base (3 mmole), a solution of thallium ethoxide in THF (5 ml) was added dropwise at room temperature. Immediately after addition, a pale yellow solid was formed and the reaction mixture was stirred for 2 hours at 20°C.

To a solution of the said salicylaldimine thallium salt in THF (5 ml) was added a solution of [RuCl₂(p-cymene)]₂ in THF (5 ml), then the reaction mixture was stirred at room temperature (20°C) for 6 hours. The thallium chloride by-product was removed via filtration. After evaporation of the solvent, the residue was recrystallized at 0°C from a dichloromethane/pentane mixture. The resulting product was then dissolved in dry ether (15 ml) and cooled down to 0°C.

In a third and last step, to the said ether solution was slowly added a solution of methyllithium (2.3 ml, 1.4 M in ether) or phenylmagnesium chloride (1.75 ml, 2 M in THF) or pentafluorophenylmagnesium chloride (7 ml, 0.5 M in ether), respectively. The reaction mixture was then slowly warmed up the room temperature and was stirred for 4 hours. The salt formed was filtered and the solvent removed. After recrystallization from ether/pentane, complexes having the following formulae were obtained with yields ranging from 60 to 70% and characterized by means of proton nuclear magnetic resonance (hereinafter referred as NMR, performed at 300 MHz with C₆D₆ at 25°C), carbon NMR (performed at 75 MHz with C₆D₆) and infrared spectrophotometry (IR, performed with CCl₄), as follows:
- example 2 (obtained from Schiff base 1-C and methyllithium): 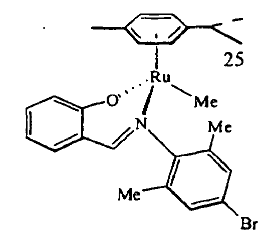 ¹H-NMR: 9.81 (s, 1H), 7.10-6.80 (m, 6 H), 1.33 (s, 6H); 5.48 (d, 1 H), 5.34 (d, 1 H), 4.48 (d, 1 H), 4.36 (d, 1 H), 2.90 (sept, 1 H), 2.16 (s, 3H), 1.26 (d, 6H) and 0.10 (s, 3H) ppm; IR (KBr) 3051, 2957, 2923, 2853, 1920, 1670, 1596, 1564, 1516, 1462, 1447, 1372, 758 cm⁻¹
- example 3 (obtained from Schiff base 1-E and methyllithium): 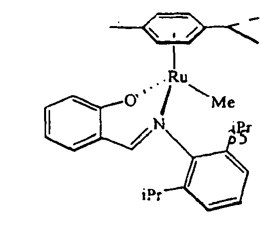 ¹H-NMR: 9.70 (s, 1 H), 7.3-7.0 (m, 7 H), 3.00 (sept, 2H), 1.12 (d, 12 H); 5.43 (d, 1 H), 5.30 (d, 1 H), 4.47 (d, 1 H), 4.33 (d, 1 H), 3.10 (sept, 1 H), 2.11 (s, 3H), 1.22 (d, 6H) and 0.22 (s, 3H) ppm; IR: 3052, 2980, 2970, 2924, 1602, 1583, 1514, 1470, 1451, 1333, 1300, 1087, 976, 930, 850, 830, 796 and 750 cm⁻¹
- example 4 (obtained from Schiff base 1-B and methyllithium): 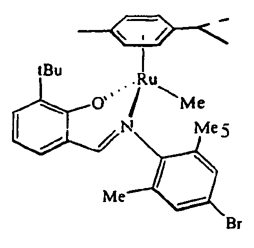 ¹H-NMR: 9.27 (s, 1H), 7.2-7.75 (m, 6 H), 3.00 (sept, 2H), 1.12 (d, J = 6.5 Hz, 12 H); 5.43, 4.70 4.44, 4.37, (d, 4H), 3.14 (sept, 1 H), 2.08 (s, 3H), 1.30 (d, 6H) and 0.13 (s, 3H) ppm; IR: 3052, 2980, 2970, 2924, 1602, 1583, 1514, 1470, 1451, 1333, 1300, 1087, 976, 930, 850, 830, 796 and 750 cm⁻¹.
- example 5 (obtained from Schiff base 1-A and phenylmagnesium chloride): 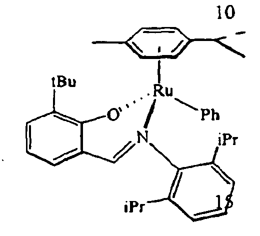 ¹H-NMR: 9.70 (s, 1H), 7.3-7.0 (m, 7 H), 3.00 (sept, 2H), 1.12 (d, J =6.5 Hz, 12 H); 5.43 (d, 2H), 5.30(d, 2H), 3.10 (sept, 1H), 2.11 (s, 3H), 1.22 (d, 6H) and 0.22 (s, 3H) ppm; IR: 3052, 2980, 2970, 2924, 1602, 1583, 1514, 1470, 1451, 1333, 1300, 1087, 976, 930, 850, 830, 796 and 750 cm⁻¹
- example 6 (obtained from Schiff base 1-A in the second step) 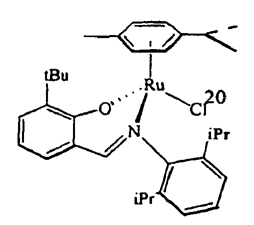 ¹H-NMR 7.69 (s, 1H), 7.07-6.12 (m, 6H), 2.99 (sept, 2H), 1.40 (s, 9H), 1.28 (d, 12H); 5.10, 4.55, 4.46, 4.39 (d, 1 H), 2.72 (sept, 1 H), 1.60 (s, 6H) and 1.09 (d, 6H) ppm; ¹³C-NMR: 161.4, 152.9, 138.96, 133.36, 130.85, 125.74, 123.43, 118.7, 114.42, 35.34, 28.42, 26.52, 23.47, 104.14, 93.64, 86.38, 83.74, 80.69, 78.61, 30.20, 22.40 and 17.86 ppm; IR: 3050, 3032, 2956, 2923, 2853, 1920, 1672, 1594, 1536, 1467, 1447, 1376, 1347 and 757 cm⁻¹.
- example 7 (obtained from Schiff base 1-A and methyllithium) 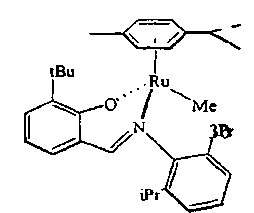 ¹H-NMR: δ 7,693 (s, 1H); 7,047-6,15 (m, 6H); 2,723 (sept, 2H); 1,404 (s, 9H); 1,31 (d, 12H); 4,95 (d, 1H); 4,55 (d, 1H); 4,48 (d, 1 H); 4,42 (d, 1 H); 2,426 (sept, 1 H); 1,596 (s, 3H); 1,050 (d, 6H) and 0,04 (s, 3H) ppm; ¹³C-NMR: δ 163,047; 152,815; 137,314; 134,565; 131,872; 127,227; 124,148; 122,06; 115,546; 35,619; 26,934; 103,04; 91,77; 88,36; 85,972; 79,485; 77,121; 30,837; 21,986 and 18,01 ppm.
- example 8 (obtained from Schiff base 1-A and pentafluorophenylmagnesium chloride) 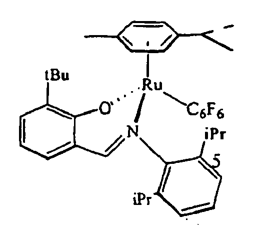 ¹H-NMR: 7.71 (s, 1 H), 6.99-6.14 (m, 6H), 2.77 (sept, 2H), 1.46 (s, 9H), 1.30 (d, 12H); 5.20, 4.72, 4.58, 4.36 (all d, 1 H), 2.61 (sept, 1H), 1.62 (s, 6H) and 1.10 (d, 6H) ppm; ¹³C-NMR 161.4, 152.9, 138.96, 133.36, 130.85, 125.74, 123.43, 118.7, 114.42, 35.34, 23.42, 26.52, 23.47, 104.14, 93.64, 86.38, 83.74, 80.69, 78.61, 30.20, 22.40, 17.86, 109.94(d), 136.51 (d), 133.19 (d) and 147.73 (d) ppm; IR: 3050, 3032, 2956, 2923, 2853, 1920, 1648, 1605, 1537, 1503, 1465, 1433, 1410, 1376, 1347, 1263, 1078, 1030, 801, 749, 720 and 566 cm⁻¹.

### EXAMPLES 9 and 10 - preparation of bimetallic Schiff base substituted ruthenium complexes

A ruthenium precursor [RuCl₂L³]₂.wherein L³ is norbornadiene (example 9) or cyclooctadiene (example 10) was dissolved in methylene chloride (15 ml), to which was added 3 ml of the thallium salt of the Schiff base 1-A (10 ml, 0.3 m) and the reaction mixture was stirred for 10 hours. After thallium chloride filtration and solvent removal, the residue was washed with methylene chloride and characterized by means of proton nuclear magnetic resonance (hereinafter referred as NMR, performed at 300 MHz with C₆D₆ at 25°C), carbon NMR (performed at 75 MHz with C₆D₆) and infrared spectrophotometry (IR, performed with CCl₄), as follows:
- example 9: 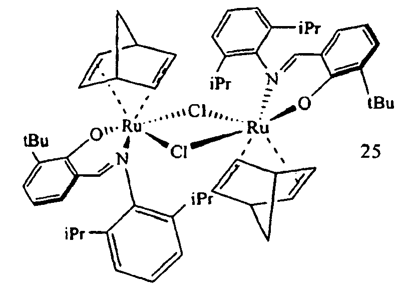 ¹H-NMR: 7.70 (s, 1H), 7.14-6.66 (m, 6H), 2.70 (sept, 2H), 1.34 (s, 9H), 1.27 (d, 12H); 6.59 (d, 1H), 6.47 (d, 1 H), 4.1 (s, 1 H), 3.98 (s, 1H), 3.91 (s, 1H), 3.86 (s, 1 H) and 1.82 (s, 2H) ppm; ¹³C-NMR δ 160.98, 151.36, 140.15, 135.14, 130.91, 126.68, 123.88, 120.52, 113.92, 34.49, 31.17, 27.84, 24.59, 145.88, 140.15, 139.84, 135.14, 72.7, 54.94 and 50.10 ppm; IR: 3098, 3025, 3032, 2956, 2923, 2853, 1920, 1672, 1594, 1536, 1467, 1409, 1310, 1240, 1180, 1160, 1085, 1035, 1000, 941, 863, 805 and 757 cm⁻¹.
- example 10: ¹H-NMR 7.69 (s, 1H), 7.07-6.12 (m, 6H), 2.99 (sept, 2H), 1.40 (s, 9H), 1.28 (d, 12H); 5.10, 4.55, 4.46, 4.39 (all d, 1H), 2.72 (sept, 1H), 1.60 (s, 6H), 1.09 (d, 6H); ¹³C-NMR: 161.4, 152.9, 138.96, 133.36, 130.85, 125.74, 123.43, 118.7, 114.42, 35.34, 28.42, 26.52, 23.47, 104.14, 93.64, 86.38, 83.74, 80.69, 78.61, 30.20, 22.40 and 17.86 ppm; IR: 3050, 3032, 2956, 2923, 2853, 1920, 1672, 1594, 1536, 1467, 1447, 1376, 1347 and 757 cm⁻¹.

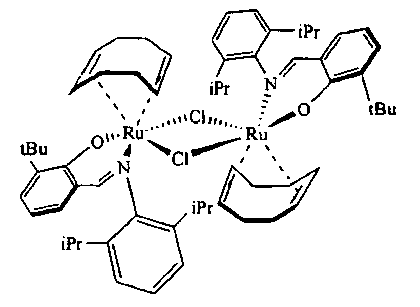

### EXAMPLE 11 - manufacture of multicoordinated Schiff base ruthenium complexes

This example illustrates an alternative route of manufacture for the Schiff base substituted ruthenium complexes represented by formulae (VII.a) to (VII.f) in example 6 and figure 1 of WO 03/062253 (i.e. having a carbene ligand with a fused aromatic ring system having the formula (VI) shown in figure 3 of WO 03/062253). This alternative method is schematically shown in figure 5, wherein the following abbreviations are used:
- Ph stands for phenyl,
- Cy stands for cyclohexyl,
- Me stands for methyl,
- *i*Pr stands for isopropyl, and
- *t*Bu stands for ter-butyl.

The scheme is self-understandable and shows a method which proceeds in five steps and achieves the desired Schiff base substituted ruthenium complexes with better yields than the method disclosed in examples 1-6 and figure 1 of WO 03/062253.

### EXAMPLE 12 (comparative) - preparation of a Schiff-base-substituted ruthenium complex for use in the ring opening polymerisation of cyclooctadiene without acid activation

A Schiff base substituted ruthenium complex similar to the compound 70 shown in figure 5 (i.e. with R₁ = NO₂, R₂ = methyl and R₃ = bromo), with the only exception that the carbene ligand with a fused aromatic ring system is replaced with a =CHC₆H₅ carbene ligand, was manufactured according to the procedure of example 11.

Ring opening metathesis polymerisation of cyclooctadiene was performed during 17 hours at 60°C in tetrahydrofuran (THF) as a solvent, while using this Schiff base substituted ruthenium complex as a catalyst in a molar ratio cyclooctadiene/catalyst equal to 500/1. A polymer having a number average molecular weight of 59,000 and a polydispersity of 1.4 was obtained in 96% yield.

### EXAMPLE 13 - ring opening polymerisation of cyclooctadiene with acid activation of a Schiff base ruthenium complex

The Schiff base substituted ruthenium complex obtained in example 12 was dissolved in THF. Then the catalyst solution was cooled down to -78°C by using an acetone bath and liquid nitrogen. Then 6 equivalents of a hydrogen chloride acid solution in THF was added to the cooled catalyst solution and the mixture was stirred for about 1 hour until room temperature was reached, and stirring was continued for 1 additional hour.

Ring opening metathesis polymerisation of cyclooctadiene was performed during 2 hours at room temperature in THF as a solvent, while using this acid-modified Schiff base substituted ruthenium complex as a catalyst in a molar ratio cyclooctadiene/catalyst equal to 500/1. A polymer having a number average molecular weight of 57,500 and a polydispersity of 1.4 was obtained in 100% yield. It is remarkable that, due to acid activation of the ruthenium complex catalyst, a polymer of very similar characteristics as the one of example 12 may be obtained while decreasing the reaction temperature from 60°C to room temperature and while simultaneously dividing the reaction time by a factor equal to 51.

### EXAMPLE 14 (comparative) - metathesis of 1,9 - decadiene without acid activation of a Schiff base ruthenium complex

The acyclic diene metathesis (ADMET) of 1,9 - decadiene was performed in bulk under partial vacuum during 17 hours at 60°C, while using this Schiff base substituted ruthenium complex of example 12 as a catalyst in a molar ratio 1,9-decadiene/catalyst equal to 500/1. No polymer was obtained after this reaction time, i.e. said ruthenium complex does not catalyze ADMET under such conditions.

### EXAMPLE 15 - metathesis of 1,9 - decadiene with acid activation of a Schiff base ruthenium complex

The Schiff base substituted ruthenium complex obtained in example 12 was dissolved in THF. Then the catalyst solution was cooled down to -78°C by using an acetone bath and liquid nitrogen. Then 6 equivalents of a hydrogen chloride acid solution in THF was added to the cooled catalyst solution and the mixture was stirred for about 1 hour until room temperature was reached, and stirring was continued for 1 additional hour.

The acyclic diene metathesis (ADMET) of 1,9 - decadiene was performed in bulk under partial vacuum during 2 hours at 60°C, while using this acid-modified Schiff base substituted ruthenium complex as a catalyst in a molar ratio 1,9-decadiene/catalyst equal to 500/1.

A polymer having a number average molecular weight of 5,700 and a polydispersity of 1.2 was obtained. It is remarkable that, due to acid activation of the ruthenium complex catalyst, a polymer may be obtained while decreasing the reaction time from 17 hours to 2 hours, all other reactions conditions being equivalent, where no polymer was obtained in the absence of acid activation of the metal complex.

### EXAMPLE 16 - ring opening polymerisation of dicyclopentadiene with acid activation of a Schiff base ruthenium complex

The Schiff base substituted ruthenium complex obtained in example 12 was dissolved in THF. Then the catalyst solution was cooled down to -78°C by using an acetone bath and liquid nitrogen. Then 6 equivalents of a hydrogen chloride acid solution in THF was added to the cooled catalyst solution and the mixture was stirred for about 1 hour until room temperature was reached, and stirring was continued for 1 additional hour.

Ring opening metathesis polymerisation of dicyclopentadiene was performed in bulk, using standard reaction-injection moulding (RIM) conditions, during 5 minutes at room temperature, while using this acid-modified Schiff base substituted ruthenium complex as a catalyst in a molar ratio dicyclopentadiene/catalyst equal to 50,000/1. A transparent (" glass grade ") polymer was obtained.

### EXAMPLE 17 - ring opening polymerisation of other monomers with acid activation of a Schiff base ruthenium complex

The Schiff base substituted ruthenium complex obtained in example 12 was dissolved in THF. Then the catalyst solution was cooled down to -78°C by using an acetone bath and liquid nitrogen. Then 6 equivalents of a hydrogen chloride acid solution in THF was added to the cooled catalyst solution and the mixture was stirred for about 1 hour until room temperature was reached, and stirring was continued for 1 additional hour. Ring opening metathesis polymerisation of various monomers was performed during 5 minutes at room temperature in THF as a solvent, while using this acid-modified Schiff base substituted ruthenium complex as a catalyst in the following monomer/catalyst molar ratios:

| | |
|---|---|
| Ethylidene-norbornene: | 50,000/1 |
| Cyclooctene: | 150,000/1 |
| Ethyltetracyclododecene: | 50,000/1 |

In each case full conversion of the monomer into the corresponding polymer was obtained within less than 5 minutes.

### EXAMPLE 18 - acid activation of multicoordinated ruthenium complexes

The monometallic Schiff base substituted ruthenium complexes of examples 2 to 8 and the bimetallic Schiff base substituted ruthenium complexes of examples 9 and 10 were activated by hydrogen chloride under the experimental conditions of example 13, i.e. with a molar ratio of said acid to said ruthenium complex equal to 6/1. The so modified ruthenium complexes were then tested in the ring opening polymerisation of various strained cyclic olefins under experimental conditions similar to those of examples 13, 16 and 17. Improved polymer yields were obtained within shorter reaction times and/or at lower reaction temperatures as compared to the starting non acid-activated ruthenium complex.

### EXAMPLE 19 - study of the reaction of an acid with a Schiff-base-substituted ruthenium complex

Air stable ruthenium complexes coordinated with Schiff base ligands are efficient catalysts for ROMP of strained cyclic olefins such as dicyclopentadiene. As shown in the previous examples, more effective catalysts can be generated *in situ* after addition of a significant molar excess of a strong acid to said Schiff-base-substituted ruthenium complexes. In order to more precisely understand the reaction between said acid and a Schiff-base-substituted ruthenium complex, and to characterize the intermediate and final ruthenium species involved in this reaction, acid activation of the Schiff-base-substituted ruthenium complex prepared in example 12 was monitored by ¹H NMR spectroscopy. ¹H NMR spectra were performed on solutions of the Schiff-base-substituted ruthenium complex, before and after acid activation at different time intervals, in deuterated chloroform. Acid activation of the free Schiff base ligand under the same conditions was also investigated by the same technique for comparison purposes.

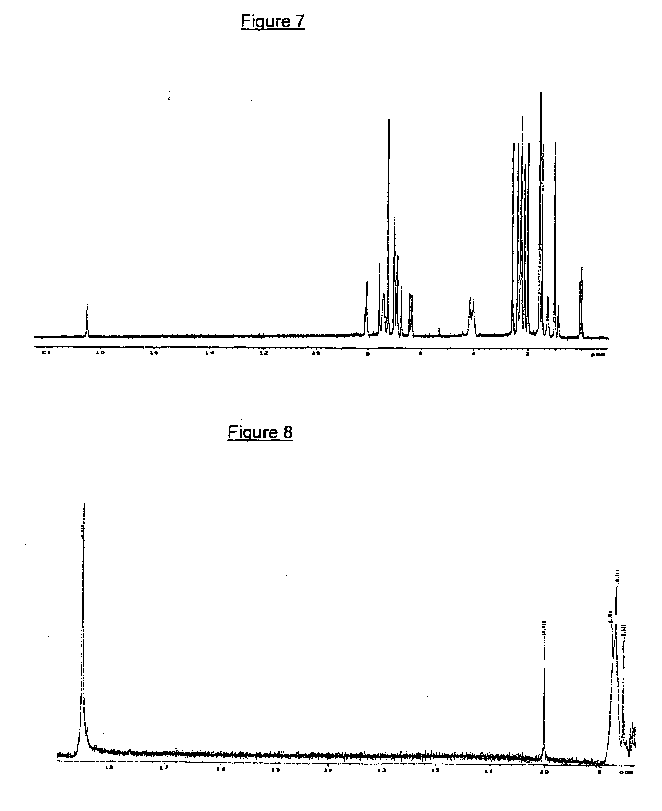

Figure 7 shows the ¹H NMR spectrum in deuterated chloroform, in the range of chemical shifts from 0 to 9 ppm, of the Schiff-base-substituted ruthenium complex obtained in example 12, before any acid activation. This spectrum shows several groups of signals due to protons from coordinated ligands. We observe two singlets at δ 1.03 and 1.48 ppm due to methyl groups of the Schiff base ligand, then between δ 2.0 and 2.8 ppm there are 6 singlets characteristic for methyl groups of the dihydroimidazolylidene ligand. The next signals between δ 3.9 and 4.3 ppm are assigned to the methylene protons of the dihydroimidazolylidene ligand. Between δ 6.2 and 8.2 ppm there is the multiplet due to phenyl protons of all ligands and protons connected to the carbon atom involved in the imine group. Finally at δ 18.51 ppm (not shown in figure 7) we observe a singlet characterizing the proton of the benzylidene ligand.

Continuous ¹H NMR monitoring allowed us to observe the results of acid-ruthenium complex reaction. For instance, figure 8 shows the ¹H NMR spectrum in deuterated chloroform, in the range of chemical shifts from 8 to 19 ppm, of the product resulting from 5 minutes of acid activation of the same Schiff-base-substituted ruthenium complex (prepared in example 12) with 10 molar equivalents of DCl (deuterium chloride) at 20°C. In this spectrum, a characteristic broad proton signal for the protonated Schiff base ligand still connected to ruthenium was detected at δ 8.72 ppm, as well as signals at δ 10.01 and 8.56 ppm which are typical of nitrosalicylaldehyde. Thus, the spectrum clearly demonstrates that acid activation after 5 minutes under these conditions (acid/complex molar ratio equal to 10:1) results in both (i) the protonation of the Schiff base ligand together with partial decoordination of the nitrogen atom of the Schiff base and (ii) the partial decoordination of the oxygen atom of the Schiff base ligand from the metal center following cleavage of the imine bond. However, such an interaction of deuterium chloride with the nitrogen atom of the Schiff base ligand can be clearly observed only during the first few minutes of reaction preceding the appearance and increase in intensity of signals at δ 10.01 and 8.56 ppm due to formation of nitrosalicylaldehyde. Protonation of the Schiff base ligand results in the presence of an intermediate species which can be depicted by the following formula:

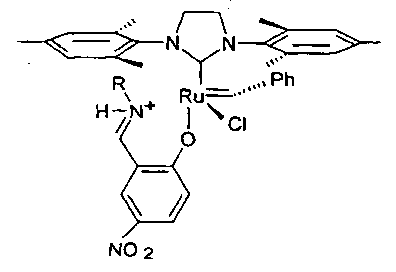

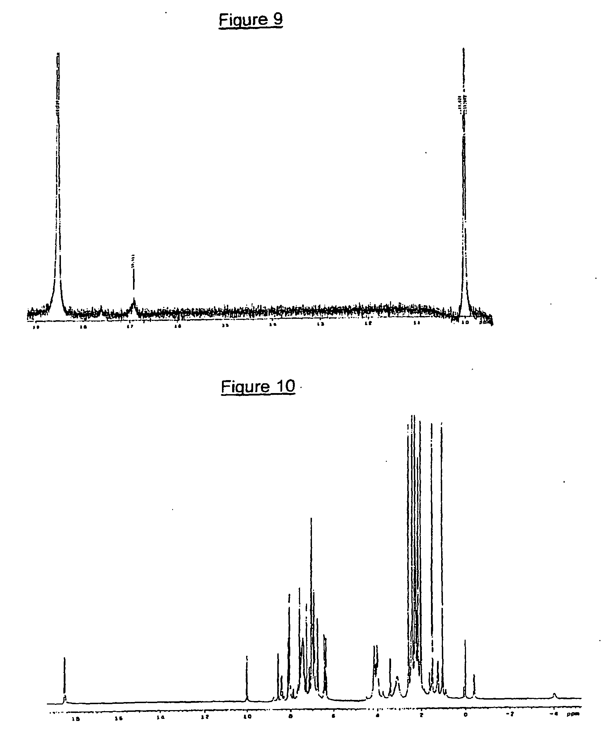

Figure 9 shows the ¹H NMR spectrum in deuterated chloroform, in the range of chemical shifts from 10 to 19 ppm, of the product resulting from 50 minutes of acid activation of the same Schiff-base-substituted ruthenium complex (prepared in example 12) with 10 molar equivalents of DCI (deuterium chloride) at 20°C. In this spectrum, we detect the formation of at least one new ruthenium carbene complex which is characterized by week broad signals from benzylidene ligand coordinated to the metal center at δ 16.91 and 17.62 ppm, respectively. This at least one new ruthenium carbene complex was probably forming during the previous step of Schiff base decoordination, i.e. was probably present at an early stage (between 5 and 50 minutes reaction) but in a concentration too low to be detectable by NMR. Without wishing to be bound by theory, we believe that this at least one new ruthenium carbene complex probably constitutes the active catalytic species which contributes in promoting olefin metathesis reactions and can be depicted by the following formula (wherein Ph is phenyl):

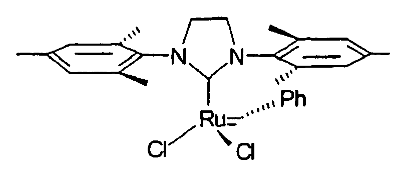

This species should therefore be named [dichloro][phenylmethylidene][1,3-dimesityl-imidazolidin-2-ylidene]ruthenium. It should be noticed that, as will be shown now, this species is very unstable and reactive, being subject to fast decomposition.

Figure 10 shows the ¹H NMR spectrum in deuterated chloroform, in the range of chemical shifts from - 5 to + 19 ppm, of the product resulting from 90 minutes of acid activation of the same Schiff-base-substituted ruthenium complex (prepared in example 12) with 10 molar equivalents of DCl (deuterium chloride) at 20°C. In this spectrum, we detect the presence of new chemical shifts at - 0.2 and - 4.0 ppm respectively. Without wishing to be bound by theory, we believe that these signals can probably be assigned to at least one new ruthenium monohydride complex which can be depicted by the following formula: 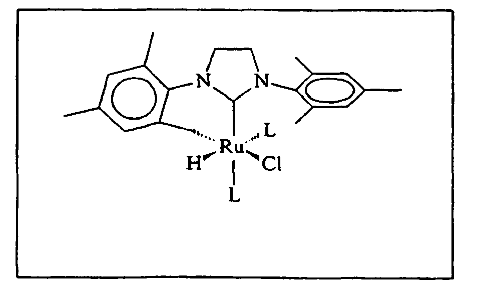 wherein L is water (H₂O).

From figure 10, it can be estimated that conversion of the acid activation reaction was around 60 % after 90 minutes.

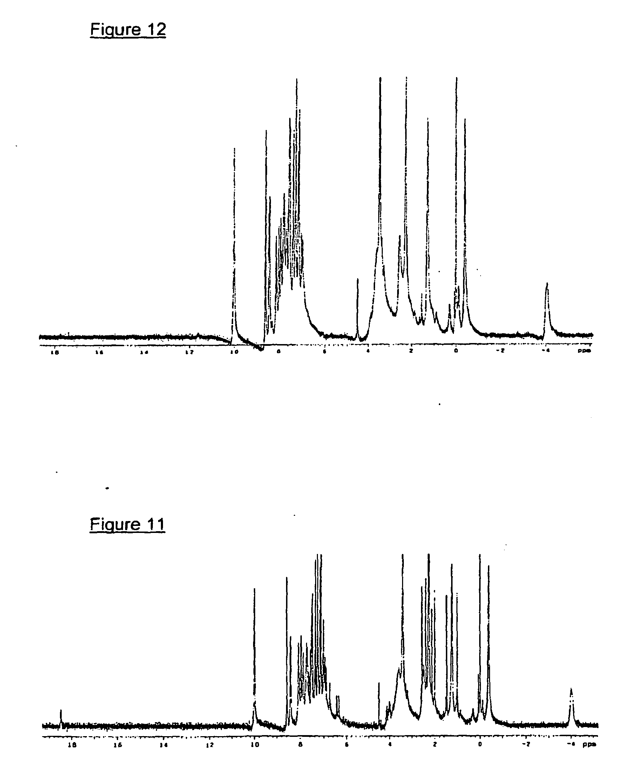

Figure 11 and 12 show the ¹H NMR spectra in deuterated chloroform, in the range of chemical shifts from - 5 to + 19 ppm, of products resulting from 24 hours and 91 hours, respectively, of acid activation of the same Schiff-base-substituted ruthenium complex (prepared in example 12) with 10 molar equivalents of DCI (deuterium chloride) at 20°C. After 24 hours of reaction time, signals due to protons of the starting Schiff-base-substituted ruthenium complex were still present together with signals from 5-nitrosalicylaldehyde (at δ 10.01 ppm, δ 11.61 ppm, δ 8.6 - 8.4 ppm and δ 7.13 ppm), from protonated 4-bromo-2,6-dimethylaniline (at δ 2.56 ppm and 7.27 ppm) and signals assigned to the new ruthenium monohydride complex (at δ - 4 ppm, δ - 0.2 ppm, δ 1.2 ppm, δ 2.1 ppm and δ 3.2 ppm, and the multiplet between 6.5 and 8 ppm). From figure 11, it can be estimated that conversion of the acid activation reaction was around 90 % after 24 hours.

After 91 hours of reaction time, all signals due to protons of the starting Schiff-base-substituted ruthenium complex have disappeared. Only signals from the protons of the new ruthenium monohydride complex, from 5-nitrosalicylaldehyde and from protonated 4-bromo-2,6-dimethylaniline could still be observed.

### EXAMPLE 20 - cyclooctene polymerization in the presence of an acid-activated Schiff-base-substituted ruthenium complex

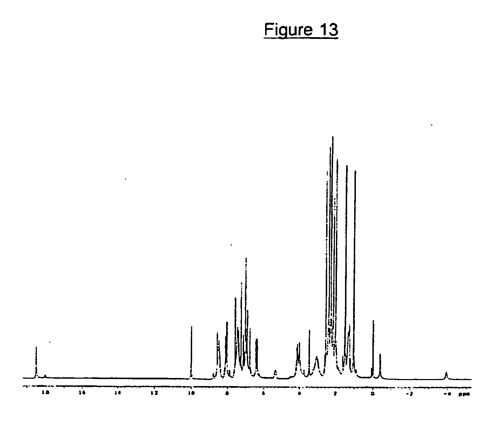

After 90 minutes of acid activation under the conditions of example 19 (i.e. acid activation at 20 °C, in an acid/complex molar ratio equal to 10, of the starting Schiff-base-substituted ruthenium complex prepared according to example 12), 100 molar equivalents of cyclooctene (with respect to the Schiff-base-substituted ruthenium complex) were added to the NMR tube. This resulted in a very fast polymerization of monomer, with the polymer being immediately apparent at the top of the tube. Figure 13 shows the ¹H NMR spectrum in deuterated chloroform, in the range of chemical shifts from - 5 to + 19 ppm, of the mixture present in the tube after 2 hours (i.e. 90 minutes acid activation and 30 minutes polymerisation). Signals from olefinic protons of polycyclooctene at 5.4 ppm were detected and can easily be seen in figure 13. This experiment also lets us follow the formation of a propagating species which gives a signal at δ 18.0 ppm.

### EXAMPLES 21 to 27 - dicyclopentadiene polymerization in the presence of an acid-activated Schiff-base-substituted ruthenium complex (first set-up)

In order to study the effect of various parameters on the result of dicyclopentadiene polymerization in the presence of a certain acid-activated Schiff-base-substituted ruthenium complex, the following procedure was set up, based on the complex obtained in example 12. Ring opening metathesis polymerisation of dicyclopentadiene was performed in a 16 ml polypropylene vessel, while using this complex as a catalyst in a molar ratio dicyclopentadiene/catalyst equal to 30,000/1, unless specified otherwise. First, catalyst (dissolved in 0.1 ml methylene chloride) and chlorhydric acid (in the acid/catalyst molar ratio r₁ specified in the table below) were introduced into the reactor at room temperature, optionally together with an additive (in an additive/catalyst molar ratio r₂ specified in the table below), and then after a certain activation time tₐ (expressed in minutes in the table below), dicyclopentadiene was in turn introduced into the reactor at room temperature in the above-specified molar ratio until the volume of all reactants reached 10 ml, and reaction was allowed to proceed for a certain time tᵣ (expressed in minutes in the table below), after which temperature quickly decreases. The polymerisation reaction was extremely exothermic and the maximum temperature Tₘₐₓ (expressed in °C in the table below) was duly recorded by means of a thermocouple. In a few embodiments of this experimental set-up (examples 24 and 25), dynamic mechanical analysis (hereinafter referred as DMA) was performed on the resulting polydicyclopentadiene in order to assess its glass transition temperature T_{g}. Results of DMA were as follows:

| | |
|---|---|
| Example 24: | 149.9 °C |
| Example 25: | 151.6 °C |

These DMA data show that Tₘₐₓ is in good accordance with T_{g}.

The following table 1 indicates the maximum temperature Tₘₐₓ obtained while changing various reaction parameters.

**TABLE 1**

| Example | r₁ | r₂ | tₐ | tᵣ | Tₘₐₓ |
|---|---|---|---|---|---|
| 21 | 10 | 0 | 0 | 7.4 | 129 |
| 22 | 10 | 0 | 1 | 1.8 | 151 |
| 23 | 10 | 0.25 | 1 | 4.0 | 153 |
| 24 | 20 | 5 | 1 | 8.7 | 150 |
| 25 | 30 | 5 | 1 | 4.1 | 153 |
| 26 | 30 | 10 | * | 9.3 | 142 |
| 27 | 20 | 0 | * | 4.2 | 155 |

In all above examples, the dicyclopentadiene monomer used includes 0.2 % by weight vinylnorbornene (hereinafter referred as VNB) acting as a chain transfer agent. The additive used in example 23 is a ruthenium dimer represented by the following formula:

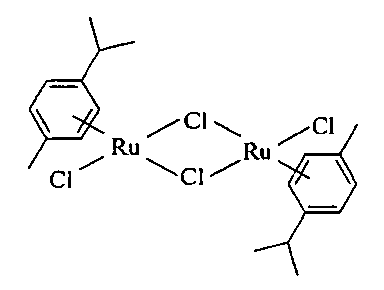

The additive used in examples 24 and 25 is azobis(isobutyronitrile) (herein-after abbreviated as AIBN) represented by the formula:

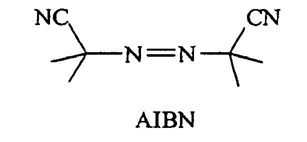

The additive used in example 26 is phosphorus tribromide PBr₃.

The data presented in table 1 show that, provided that a short activation time is left for reacting catalyst and chlorhydric acid before addition of the monomer to be polymerized, polydicyclopentadiene with a glass transition temperature T_{g} above 140 °C can reproducibly be obtained according to this invention.

### EXAMPLES 28 to 31 - dicyclopentadiene polymerization in the presence of an acid-activated Schiff-base-substituted ruthenium complex (second set-up)

The experimental procedure of examples 21 to 27 was repeated, except that in this second set-up, a 100 ml polypropylene vessel was used, the catalyst (obtained from example 12) was dissolved in 1 ml methylene chloride, dicyclopentadiene was introduced into the reactor at room temperature until the volume of all reactants reached 80 ml, the activation time was fixed to 1 minute, and the dicyclopentadiene/catalyst molar ratio R was also used as a reaction parameter.

The following table 2 indicates the maximum temperature Tₘₐₓ obtained while changing various reaction parameters. The additive used in examples 30 and 31 is AIBN. The data presented in table 1 show that polydicyclopentadiene with a maximum exothermic temperature (previously checked to be consistent with glass transition temperature T_{g}) above 140 °C and up to 166 °C can reproducibly be obtained according to this invention even at higher dicyclopentadiene/catalyst molar ratios than in the first set-up.

**TABLE 2**

| Example | R | r₁ | r₂ | tᵣ | Tₘₐₓ |
|---|---|---|---|---|---|
| 28 | 30,000 | 10 | 0 | 5.6 | 193 |
| 29 | 60,000 | 30 | 0 | 14.6 | 143 |
| 30 | 60,000 | 30 | 20 | 19.4 | 160 |
| 31 | 60,000 | 30 | 30 | 16.5 | 166 |

### EXAMPLES 32 to 42 - dicyclopentadiene polymerization in the presence of an acid-activated Schiff-base-substituted ruthenium complex and in a solvent (third set-up)

Ring opening metathesis polymerisation of dicyclopentadiene was performed while using the complex obtained in example 12 (dissolved in 1 ml of a solvent S, either tetrahydrofuran or methylene chloride, respectively indicated as THF or MC in the following table 3) as a catalyst, in a molar ratio dicyclopentadiene/catalyst indicated as R. First, chlorhydric acid (in the acid/catalyst molar ratio r₁ specified in the table below) and VNB (0.2 % by weight with respect to dicyclopentadiene) were added to 80 ml dicyclopentadiene at room temperature. Then this mixture was added to the catalyst solution, optionally together with AIBN as an additive (in an additive/catalyst molar ratio r₂ specified in the table below). Reaction was allowed to proceed for a certain time tᵣ (expressed in minutes in the table below), after which the reactor was cooled down. Dynamic mechanical analysis (hereinafter referred as DMA) was performed on the resulting polydicyclopentadiene in order to assess its glass transition temperature T_{g}.

The following table 3 indicates the temperature measured by DMA (expressed in °C) while changing various reaction parameters.

**TABLE 3**

| Example | R | r₁ | r₂ | tᵣ | S | DMA |
|---|---|---|---|---|---|---|
| 32 | 60,000 | 30 | 30 | 58.6 | THF | 154.7 |
| 33 | 30,000 | 10 | 30 | 17.7 | CM | 165.2 |
| 34 | 30,000 | 30 | 0 | 7.7 | THF | 163.8 |
| 35 | 30,000 | 30 | 0 | 7.9 | CM | 170.2 |
| 36 | 30,000 | 10 | 30 | 46.9 | THF | 166.7 |
| 37 | 60,000 | 30 | 30 | 30.6 | CM | 158.2 |
| 38 | 30,000 | 30 | 30 | 18.0 | THF | 165.3 |
| 39 | 30,000 | 10 | 0 | 28.9 | CM | 167.4 |
| 40 | 60,000 | 30 | 0 | 56.0 | THF | 151.1 |
| 41 | 60,000 | | 0 | 21.9 | THF | 169.5 |
| 42 | 30,000 | 30 | 30 | 12.5 | CM | 159.6 |

| | | | | | | |
|---|---|---|---|---|---|---|
| (end of Table 3) | | | | | | |

The data presented in table 3 show that polydicyclopentadiene with a glass transition temperature T_{g} between about 150 °C and 170 °C can reproducibly be obtained according to various conditions in this set-up of the invention.

### EXAMPLE 43 - study of the reaction of an acid with a Schiff-base-substituted ruthenium complex

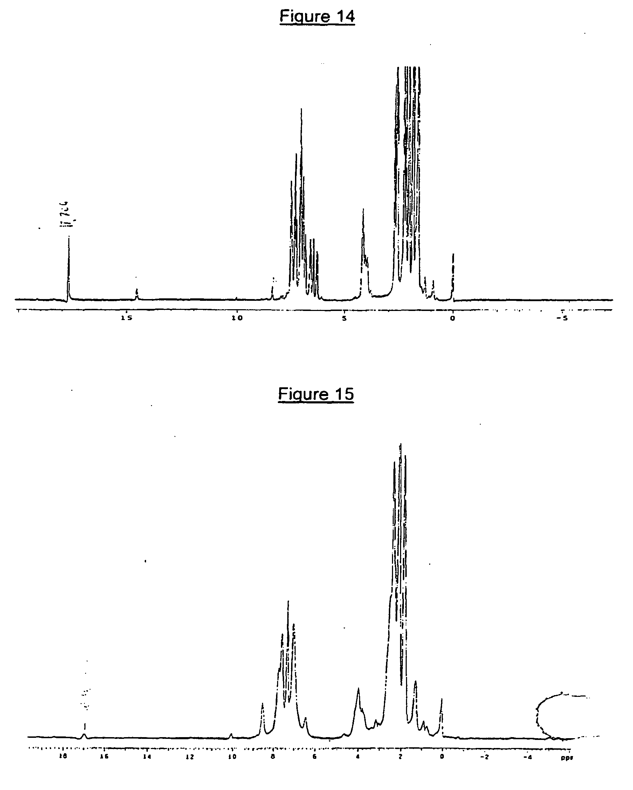

The study of example 19 was repeated but on a different Schiff-base-substituted ruthenium complex having the following formula: 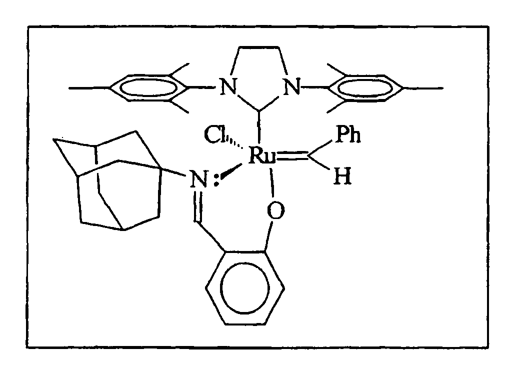 i.e. a ruthenium complex similar to that of example 12, except that the substituted phenyl group on the nitrogen atom of the Schiff base was replaced by a more sterically hindered adamantyl group. The corresponding Schiff base ligand is easily accessible from adamantylamine. The proton NMR spectrum of this complex in deuterated chloroform is shown in figure 14. We observe a group of signals between δ 1.5 and 2.8 ppm which are characteristic for methyl groups of the dihydroimidazolylidene ligand and protons of the adamantly group coordinated to nitrogen. Signals between δ 3.9 and 4.3 ppm are assigned to methylene protons of the dihydroimidazolylidene ligand. Between δ 6.2 and 8.2 ppm is the multiplet due to phenyl protons of all ligands and proton connected to the carbon atom involved in the imine bond. Finally at δ 17.7 ppm we observe a singlet characterizing the proton of the benzylidene ligand.

While performing acid activation under the same conditions as in example 19, except an acid activation of only 10 minutes, The proton NMR spectrum of the resulting product is shown in figure 15. This experiment allowed us to observe very fast and full (100 %) conversion of the starting complex after 10 minutes (disappearance of the signal at δ 17.7 ppm). During this period of time, the formation of a new complex was observed (signal at δ 16.91 ppm). Similarly to the experiment of example 19, the broad proton signal characteristic for a protonated Schiff base ligand still coordinated to ruthenium was also detected at δ 8.62 ppm.

## Claims

1. A method of modifying a multi-coordinated metal complex, a salt, a solvate or an enantiomer thereof, said multi-coordinated metal complex comprising (i) at least one multidentate Schiff base ligand comprising an imino group and being coordinated to the metal, in addition to the nitrogen atom of said imino group, through at least one further heteroatom selected from the group consisting of oxygen, sulfur and selenium, and (ii) one or more other ligands, wherein said metal is selected from the group consisting of ruthenium, osmium and iron, and wherein the number of carbon atoms in said at least one multidentate Schiff base ligand (i), between the nitrogen atom of said imino group and said coordinating heteroatom of said at least one multidentate Schiff base ligand (i), is from 2 to 4, and wherein at least one of said other ligands (ii) is a carbene ligand selected from the group consisting of N-heterocyclic carbenes, alkylidene ligands, vinylidene ligands, indenylidene ligands and allenylidene ligands, said method comprising bringing said multi-coordinated metal complex into contact with an acid under conditions such that said acid is able to at least partly cleave a bond between the metal and said at least one multidentate Schiff base ligand (i), and wherein said other ligands (ii) are selected such as to be unable of protonation by said acid under said conditions, and are not selected from the group consisting of phosphines, amines, arsines and stibines

2. A method according to claim 1, **characterized in that** said conditions include a molar ratio between said acid and said multi-coordinated metal complex being above 1.2 and below 40.

3. A method according to claim 1 or claim 2, **characterized in that** said conditions include a contact time from 5 seconds to 100 hours.

4. A method according to any of claims 1 to 3, **characterized in that** said conditions include a contact temperature from about -50 °C to about +80 °C.

5. A method according to any of claims 1 to 4, wherein the pKa of said acid is lower than the pKa of said multidentate Schiff base ligand (i).

6. A method according to any of claims 1 to 5, **characterized in that** at least one of said other ligands (ii) is a constraint steric hindrance ligand having a pKa of at least 15.

7. A method according to any of claims 1 to 6, **characterized in that** at least one of said other ligands (ii) is an anionic ligand.

8. A method according to any of claims 1 to 6, **characterized in that** at least one of said other ligands (ii) is a non-anionic ligand.

9. A method according to any of claims 1 to 8, **characterized in that** said acid is chlorhydric acid or bromhydric acid.

10. A method according to any of claims 1 to 9, **characterized in that** said conditions are able to protonate the multidentate Schiff base ligand and decoordinate the nitrogen atom of the imino group of said multidentate Schiff base ligand from the metal.

11. A method according to any of claims 1 to 10, **characterized in that** said conditions are able to decoordinate the further heteroatom of said multidentate Schiff base ligand from the metal.

12. The reaction product of:
(a) a multi-coordinated metal complex, a salt, a solvate or an enantiomer thereof, said multi-coordinated metal complex comprising (i) at least one multidentate Schiff base ligand comprising an imino group and being coordinated to the metal, in addition to the nitrogen atom of said imino group, through at least one further heteroatom selected from the group consisting of oxygen, sulfur and selenium, wherein the number of carbon atoms in said at least one multidentate Schiff base ligand (i), between the nitrogen atom of said imino group and said coordinating heteroatom of said at least one multidentate Schiff base ligand (i), is from 2 to 4, and (ii) one or more other ligands, wherein at least one of said other ligands (ii) is a carbene ligand selected from the group consisting of N-heterocyclic carbenes, alkylidene ligands, vinylidene ligands, indenylidene ligands and allenylidene ligands, wherein said metal is selected from the group consisting of ruthenium, osmium and iron, and
(b) an acid reacted in a molar ratio above about 1.2 with respect to said multi-coordinated metal complex (a),
provided that said other ligands (ii) are unable of protonation by said acid and are not selected from the group consisting of amines, phosphines, arsines and stibines

13. A product according to claim 12, **characterized in that** the pKa of said acid (b) is lower than the pKa of said at least one multidentate Schiff base ligand (i).

14. A product according to any of claims 12 to 13, **characterized in that** at least one of said other ligands (ii) of said multi-coordinated metal complex (a) is a constraint steric hindrance ligand having a pKa of at least 15.

15. A product according to any of claims 12 to 14, **characterized in that** at least one of said other ligands (ii) of said multi-coordinated metal complex (a) is an anionic ligand.

16. A product according to any of claims 12 to 14, **characterized in that** at least one of said other ligands (ii) of said multi-coordinated metal complex (a) is a non-anionic ligand.

17. A product according to any of claims 12 to 16, **characterized in that** said acid is chlorhydric acid or bromhydric acid.

18. A product according to any of claims 12 to 17, being a monometallic species represented by the general formula:
[M(L_{c})(L₂)(X)(SB⁺)]X⁻
wherein
- M is a metal selected from the group consisting of ruthenium, osmium and iron;
- SB⁺ is a protonated Schiff base ligand;
- L_{c} is a carbene ligand selected from the group consisting of alkylidene ligands, vinylidene ligands, indenylidene ligands and allenylidene ligands;
- L₂ is a non-anionic ligand;
- X is an anionic ligand; and
- X⁻ is an anion,
salts, solvates and enantiomers thereof.

19. A product according to any of claims 12 to 17, being a bimetallic species represented by the general formula:
-[M(L_{c})(SB⁺)(X₁)(X₂)(M')(X₃)(L)]X⁻
wherein
- M and M' are each a metal independently selected from the group consisting of ruthenium, osmium and iron;
- SB⁺ is a protonated Schiff base ligand;
- L_{c} is a carbene ligand selected from the group consisting of alkylidene ligands, vinylidene ligands, indenylidene ligands and allenylidene ligands;
- L is a non-anionic ligand;
- X₁, X₂ and X₃ are each independently selected from anionic ligands; and
- X⁻ is an anion,
salts, solvates and enantiomers thereof.

20. A product according to any of claims 12 to 17, being a cationic monometallic species being represented by the general formula (VI): 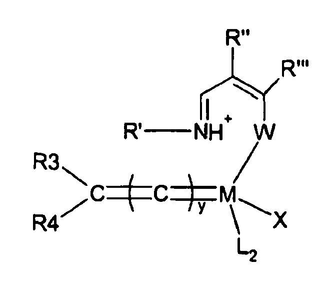 or a cationic monometallic species being represented by the general formula (VII): 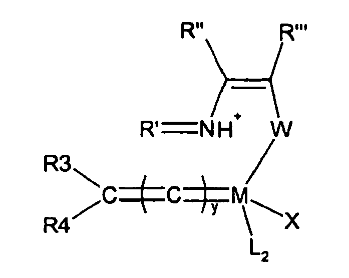 wherein
- M is a metal selected from the group consisting of ruthenium, osmium and iron;
- W is selected from the group consisting of oxygen, sulphur, selenium, NR"", PR"", AsR"" and SbR"";
- R", R"' and R"" are each a radical independently selected from the group consisting of hydrogen, C₁₋₆ alkyl, C₃₋₈ cycloalkyl, C₁₋₆ alkyl-C₁₋₆ alkoxysilyl, C₁₋₆ alkyl-aryloxysilyl, C₁₋₆ alkyl-C₃-₁₀ cycloalkoxysilyl, aryl and heteroaryl, or R" and R'" together form an aryl or heteroaryl radical, each said radical (when different from hydrogen) being optionally substituted with one or more, preferably 1 to 3, substituents R₅ each independently selected from the group consisting of halogen atoms, C₁₋₆ alkyl, C₁₋₆ alkoxy, aryl, alkylsulfonate, arylsulfonate, alkylphosphonate, arylphosphonate, C₁₋₆ alkyl-C₁₋₆ alkoxysilyl, C₁₋₆ alkyl-aryloxysilyl, C₁₋₆ alkyl-C₃₋₁₀ cycloalkoxysilyl, alkylammonium and arylammonium;
- R' is either as defined for R", R"' and R"" when included in a compound having the general formula (IA) or, when included in a compound having the general formula (IB), is selected from the group consisting of C₁₋₆ alkylene and C₃₋₈ cycloalkylene, the said alkylene or cycloalkylene group being optionally substituted with one or more substituents R₅:
- L₂ is a non-anionic ligand;
- X is an anionic ligand;
- R₃ and R₄ are each hydrogen or a radical selected from the group consisting of C₁-₂₀ alkyl, C₂₋₂₀ alkenyl, C₂₋₂₀ alkynyl, C₁-₂₀ carboxylate, C₁₋₂₀ alkoxy, C₂₋₂₀ alkenyloxy, C₂₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium and arylammonium;
- R' and one of R₃ and R₄ may be bonded to each other to form a bidentate ligand;
- R"' and R"" may be bonded to each other to form an aliphatic ring system including a heteroatom selected from the group consisting of nitrogen, phosphorous, arsenic and antimony;
- R₃ and R₄ together may form a fused aromatic ring system, and
- y represents the number of sp₂ carbon atoms between M and the carbon atom bearing R₃ and R₄ and is an integer from 0 to 3 inclusive,
salts, solvates and enantiomers thereof, and said cationic species being associated with an anion.

21. A product according to any of claims 12 to 17, being a cationic bimetallic species being represented by the general formula (X): 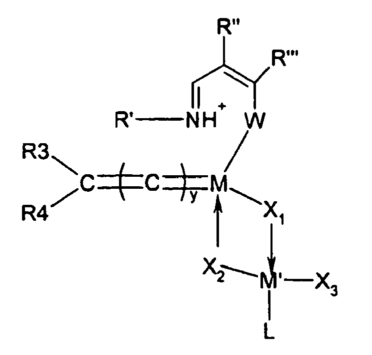 or a cationic bimetallic species being represented by the general formula (XI): 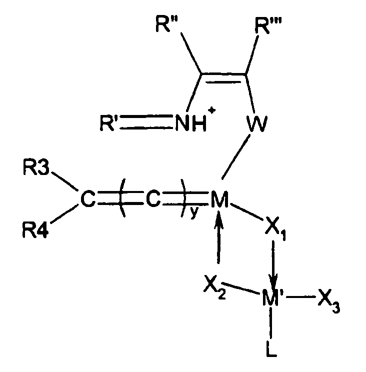 wherein
- M and M' are each a metal independently selected from the group consisting of ruthenium, osmium and iron;
- W is selected from the group consisting of oxygen, sulphur, selenium, NR"", PR"", AsR"" and SbR"";
- R", R"' and R"" are each a radical independently selected from the group consisting of hydrogen, C₁₋₆ alkyl, C₃₋₈ cycloalkyl, C₁₋₆ alkyl-C₁₋₆ alkoxysilyl, C₁₋₆ alkyl-aryloxysilyl, C₁₋₆ alkyl-C₃₋₁₀ cycloalkoxysilyl, aryl and heteroaryl, or R" and R'" together form an aryl or heteroaryl radical, each said radical (when different from hydrogen) being optionally substituted with one or more, preferably 1 to 3, substituents R₅ each independently selected from the group consisting of halogen atoms, C₁₋₆ alkyl, C₁₋₆ alkoxy, aryl, alkylsulfonate, arylsulfonate, alkylphosphonate, arylphosphonate, C₁₋₆ alkyl-C₁₋₆ alkoxysilyl, C₁₋₆ alkyl-aryloxysilyl, C₁₋₆ alkyl-C₃₋₁₀ cycloalkoxysilyl, alkylammonium and arylammonium;
- R' is either as defined for R", R"' and R"" when included in a compound having the general formula (IA) or, when included in a compound having the general formula (IB), is selected from the group consisting of C₁₋₆ alkylene and C₃₋₈ cycloalkylene, the said alkylene or cycloalkylene group being optionally substituted with one or more substituents R₅;
- R₃ and R₄ are each hydrogen or a radical selected from the group consisting of C₁₋₂₀ alkyl, C₂₋₂₀ alkenyl, C₂₋₂₀ alkynyl, C₁₋₂₀ carboxylate, C₁₋₂₀ alkoxy, C₂₋₂₀ alkenyloxy, C₂₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium and arylammonium;
- R' and one of R₃ and R₄ may be bonded to each other to form a bidentate ligand;
- R"' and R"" may be bonded to each other to form an aliphatic ring system including a heteroatom selected from the group consisting of nitrogen, phosphorous, arsenic and antimony;
- R₃ and R₄ together may form a fused aromatic ring system, and
- y represents the number of sp₂ carbon atoms between M and the carbon atom bearing R₃ and R₄ and is an integer from 0 to 3 inclusive,
- X₁, X₂ and X₃ are each independently selected from anionic ligands; and
- L is a non-anionic ligand,
including salts, solvates and enantiomers thereof.

22. A product according to any of claims 12 to 17, **characterized in that** at least one of said other ligands (ii) of said multi-coordinated metal complex (a) is a solvent S and said complex (a) is a cationic species associated with an anion A.

23. A product according to any of claims 12 to 17, **characterized in that** said multi-coordinated metal complex (a) is a bimetallic complex.

24. A product according to claim 23, **characterized in that** one metal of said bimetallic complex is penta-coordinated with said at least one multidentate Schiff base ligand (i) and with said one or more other ligands (ii), and the other metal is tetra-coordinated with one or more neutral ligands and one or more anionic ligands.

25. A product according to claim 23, **characterized in that** each metal of said bimetallic complex is hexa-coordinated with said at least one multidentate Schiff base ligand (i) and with said one or more other ligands (ii).

26. A product according to claim 23, **characterized in that** the two metals of the bimetallic complex are the same.

27. A product according to claim 23, **characterized in that** the two metals of the bimetallic complex are different.

28. A product according to any of claims 12 to 17, **characterized in that** said multi-coordinated metal complex (a) is a monometallic complex.

29. A product according to any of claims 12 to 17, **characterized in that** said multi-coordinated metal complex (a) is a penta-coordinated metal complex or a tetra-coordinated metal complex.

30. A product according to claim 29, **characterized in that** said at least one multidentate Schiff base ligand (i) is a bidentate ligand and said multi-coordinated metal complex (a) comprises two other ligands (ii).

31. A product according to claim 29, **characterized in that** said at least one multidentate Schiff base ligand (i) is a tridentate ligand and said multi-coordinated metal complex (a) comprises a single other ligand (ii).

32. A product according to claim 29 or claim 30, **characterized in that** said at least one multidentate Schiff base ligand (i) has one of the general formulae (IA) and (IB): 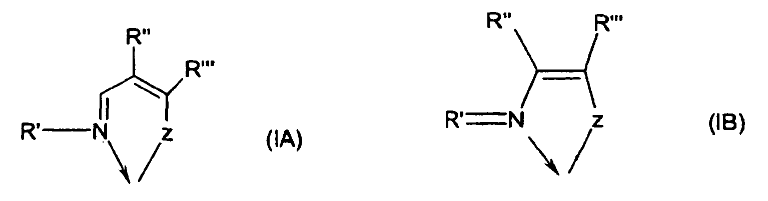 wherein:
- Z is selected from the group consisting of oxygen, sulfur and selenium;
- R" and R"' are each a radical independently selected from the group consisting of hydrogen, C₁₋₇ alkyl, C₃₋₁₀ cycloalkyl, C₁₋₆ alkyl-C₁₋₆ alkoxysilyl, C₁₋₆ alkyl-aryloxysilyl, C₁₋₆ alkyl-C₃₋₁₀ cycloalkoxysilyl, aryl and heteroaryl, or R" and R'" together form an aryl or heteroaryl radical, each said radical being optionally substituted with one or more, preferably 1 to 3, substituents R₅ each independently selected from the group consisting of halogen atoms, C₁₋₆ alkyl, C₁₋₆ alkoxy, aryl, alkylsulfonate, arylsulfonate, alkylphosphonate, arylphosphonate, C₁₋₆ alkyl-C₁₋₆ alkoxysilyl, C₁₋₆ alkyl-aryloxysilyl, C₁₋₆ alkyl-C₃₋₁₀ cycloalkoxysilyl, alkylammonium and arylammonium;
- R' is either as defined for R" and R"' when included in a compound having the general formula (IA) or, when included in a compound having the general formula (IB), is selected from the group consisting of C₁₋₇ alkylene and C₃₋₁₀ cycloalkylene, the said alkylene or cycloalkylene group being optionally substituted with one or more substituents R₅.

33. A product according to claim 29, **characterized in that** at least one of said other ligands (ii) of said multi-coordinated metal complex (a) is a derivative, wherein one or more hydrogen atoms is substituted with a group providing constraint steric hindrance, of a N-heterocyclic carbene selected from the group consisting of imidazol-2-ylidene, dihydroimidazol-2-ylidene, oxazol-2-ylidene, triazol-5-ylidene, thiazol-2-ylidene, bis(imida-zolin-2-ylidene), bis(imidazolidin-2-ylidene), pyrrolylidene, pyrazolylidene, dihydro-pyrrolylidene, pyrrolylidinylidene and benzo-fused derivatives thereof, or a non-ionic prophosphatrane superbase.

34. A product according to claim 29, **characterized in that** at least one of said other ligands (ii) of said multi-coordinated metal complex (a) is an anionic ligand selected from the group consisting of C₁₋₂₀ alkyl, C₁₋₂₀ alkenyl, C₁₋₂₀ alkynyl, C₁₋₂₀ carboxylate, C₁₋₂₀ alkoxy, C₁₋₂₀ alkenyloxy, C₁₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium, arylammonium, halogen atoms and cyano.

35. A product according to claim 29, **characterized in that** at least one of said other ligands (ii) of said multi-coordinated metal complex (a) is a carbene ligand represented by the general formula =[C=]_{y}CR₃R₄, wherein:
- y is an integer from 0 to 3 inclusive, and
- R₃ and R₄ are each hydrogen or a hydrocarbon radical selected from the group consisting of C₁₋₂₀ alkyl, C₁₋₂₀ alkenyl, C₁₋₂₀ alkynyl, C₁₋₂₀ carboxylate, C₁₋₂₀ alkoxy, C₁₋₂₀ alkenyloxy, C₁₋₂₀ alkynyloxy, aryl, aryloxy, C₁₋₂₀ alkoxycarbonyl, C₁₋₈ alkylthio, C₁₋₂₀ alkylsulfonyl, C₁₋₂₀ alkylsulfinyl C₁₋₂₀ alkylsulfonate, arylsulfonate, C₁₋₂₀ alkylphosphonate, arylphosphonate, C₁₋₂₀ alkylammonium and arylammonium; or R₃ and R₄ together may form a fused aromatic ring system.

36. A product according to claim 35, **characterized in that** R₃ and R₄ together form a fused aromatic ring system such that said other ligand (ii) of said multi-coordinated metal complex (a) is a phenylindenylidene ligand.

37. A product according to claim 29, **characterized in that** at least one of said other ligands (ii) of said multi-coordinated metal complex (a) is a non-anionic unsaturated ligand L¹ selected from the group consisting of aromatic and unsaturated cycloaliphatic groups, preferably aryl, heteroaryl and C₄₋₂₀ cycloalkenyl groups, the said aromatic or unsaturated cycloaliphatic group being optionally substituted with one or more C₁₋₇ alkyl groups or electron-withdrawing groups such as, but not limited to, halogen, nitro, cyano, (thio)carboxylic acid, (thio)carboxylic acid (thio)ester, (thio)carboxylic acid (thio)amide, (thio)carboxylic acid anhydride and (thio) carboxylic acid halide.

38. A product according to claim 29, **characterized in that** at least one of said other ligands (ii) of said multi-coordinated metal complex (a) is a non-anionic ligand L² selected from the group consisting of C₁₋₇ alkyl, C₃₋₁₀ cycloalkyl, arylalkyl and heterocyclic, the said group being optionally substituted with one or more preferably electron-withdrawing substituents such as, but not limited to, halogen, nitro, cyano, (thio)carboxylic acid, (thio)carboxylic acid (thio)ester, (thio)carboxylic acid (thio)amide, (thio)carboxylic acid anhydride and (thio) carboxylic acid halide.

39. A product according to claim 29, **characterized in that** said at least one multidentate Schiff base ligand (i) is a tetradentate ligand and said multi-coordinated metal complex (a) comprises one or two other ligands (ii) being non-anionic ligands L⁷ selected from the group consisting of aromatic and unsaturated cycloaliphatic groups, preferably aryl, heteroaryl and C₄₋₂₀ cycloalkenyl groups, wherein the said aromatic or unsaturated cycloaliphatic group is optionally substituted with one or more C₁₋₇ alkyl groups or electron-withdrawing groups such as, but not limited to, halogen, nitro, cyano, (thio)carboxylic acid, (thio)carboxylic acid (thio)ester, (thio)carboxylic acid (thio)amide, (thio)carboxylic acid anhydride and (thio) carboxylic acid halide.

40. A product according to claim 12, wherein said acid (b) is an acid generated *in situ* from a photoacid generator.

41. A product according to claim 12, comprising the product of at least partial cleavage of a bond between the metal and said at least one multidentate Schiff base ligand (i).

42. A catalytic system comprising:
(a) as the main catalytic species, a reaction product according to any of claims 12 to 41, and
(b) one or more second catalyst components being selected from the group consisting of Lewis acid co-catalysts (b₁), catalyst activators (b₂), and initiators having a radically transferable atom or group (b₃).

43. A catalytic system according to claim 42, wherein the second catalyst component includes a co-catalyst (b₁) selected from the group consisting of boron trihalides; phosphorus trihalides; trialkylboron compounds; triarylboron compounds; organoaluminum compounds; magnesium dihalides; aluminum trihalides; tin tetrachloride; titanium or vanadium trihalides or tetrahalides or tetraalkoxides; antimony and bismuth pentahalides.

44. A catalytic system according to claim 42, wherein the second catalyst component includes, as a catalyst activator (b₂), a diazo compound.

45. A catalytic system according to claim 42, wherein the second catalyst component includes, as an initiator having a radically transferable atom or group (b₃), a compound having the formula R₃₅R₃₆R₃₇CX₁, wherein:
- X₁ is selected from the group consisting of halogen, OR₃₈ (wherein R₃₈ is selected from C₁₋₂₀ alkyl, polyhaloC₁₋₂₀alkyl, C₂₋₂₀ alkynyl (preferably acetylenyl), C₂₋₂₀ alkenyl (preferably vinyl), phenyl optionally substituted with 1 to 5 halogen atoms or C₁₋₇ alkyl groups and phenyl-substituted C₁₋₇ alkyl), SR₃₉, OC(=O)R₃₉, OP(=O)R₃₉, OP(=O)(OR₃₉)₂, OP(=O)OR₃₉, ON(R₃₉)₂ and S-C(=S)N(R₃₉)₂, wherein R₃₉ is aryl or C₁₋₂₀ alkyl, or where an N(R₃₉)₂ group is present, the two R₃₉ groups may be joined to form a 5-, 6- or 7-membered heterocyclic ring (in accordance with the definition of heteroaryl above), and
- R₃₅, R₃₆ and R₃₇ are each independently selected from the group consisting of hydrogen, halogen, C₁₋₂₀ alkyl (preferably C₁₋₆ alkyl), C₃₋₈ cycloalkyl, C(=O)R₄₀, (wherein R₄₀ is selected from the group consisting of C₁₋₂₀ alkyl, C₁₋₂₀ alkoxy, aryloxy or heteroaryloxy), C(=O)NR₄₁R₄₂ (wherein R₄₁ and R₄₂ are independently selected from the group consisting of hydrogen and C₁₋₂₀ alkyl or R₄, and R₄₂ may be joined together to form an alkylene group of 2 to 5 carbon atoms), COCI, OH, CN, C₂₋₂₀ alkenyl (preferably vinyl), C₂₋₂₀ alkynyl, oxiranyl, glycidyl, aryl, heteroaryl, arylalkyl and aryl-substituted C₂₋₂₀ alkenyl.

46. A supported catalyst, comprising:
(a) a catalytically active reaction product according to any of claims 12 to 41, or a catalytic system according to claim 46, and
(b) a supporting amount of a carrier suitable for supporting said catalytically active product or catalytic system (a).

47. A method of performing an olefin or acetylene metathesis reaction or a reaction involving the transfer of an atom or group to an ethylenically or acetylenically unsaturated compound or another reactive substrate in the presence of a catalytic component, wherein the said catalytic component comprises a product according to any of claims 12 to 41 or a catalytic system according to any of claims 42 to 45.

48. A method according to claim 47, wherein said reaction involving the transfer of an atom or group to an olefin or another reactive substrate is selected from the group consisting of:
- atom or group transfer radical polymerisation of one or more radically (co)polymerisable monomers, especially mono- and diethylenically unsaturated monomers;
- atom transfer radical addition of a polyhalomethane having the formula CXₙH₄₋ₙ, wherein X is halogen and n is an integer from 2 to 4, onto an ethylenically unsaturated compound to produce the corresponding saturated polyhalogenated adduct;
- vinylation reaction of a mono- or di-alkyne with a mono- or di-carboxylic acid to produce alk-1-enyl esters or enol esters or Markovnikov adducts or anti- Markovnikov adducts or mixtures thereof;
- cyclopropanation of an α-ethylenically unsaturated compound for producing an organic compound having one or more cyclopropane structural units;
- quinoline synthesis through oxidative cyclisation of 2-aminobenzyl alcohol with ketones;
- epoxidation of α-ethylenically unsaturated compounds for producing epoxides;
- oxidation of organic compounds including the oxidation of saturated hydrocarbons for producing alcohols, or sulfides for producing sulfoxides and sulfones, or phosphines for producing phosphonates, or alcohols and aldehydes for producing carboxylic acids;
- cyclopropenation of an alkyne for producing an organic compound having one or more cyclopropene structural units;
- hydrocyanation of α-ethylenically unsaturated compounds for producing saturated nitriles, or alkynes for producing unsaturated nitriles, or α,β-unsaturated aldehydes or ketones for producing β-cyano carbonyl compounds;
- hydrosilylation of olefins for producing saturated silanes, or alkynes for producing unsaturated silanes, or ketones for producing silyl ethers, or trimethylsilylcyanation of aldehydes for producing cyanohydrin trimethylsilyl ethers;
- aziridination of imines or alkenes for producing organic compounds having one or more aziridine structural units;
- hydroamidation of olefins for producing saturated amides;
- hydrogenation of olefins for producing alkanes, or ketones for producing alcohols;
- aminolysis of olefins for producing saturated primary or secondary amines;
- isomerisation of alcohols, preferably allylic alcohols, for producing aldehydes;
- Grignard cross-coupling of alkyl or aryl halides for producing alkanes or arylalkanes;
- hydroboration of olefins for producing alkylboranes and trialkylboranes;
- hydride reduction of aldehydes and ketones for producing alcohols;
- aldol condensation of saturated carboxyl compounds for producing α,β-unsaturated carboxyl compounds or β-hydroxycarbonyl compounds, and intra-molecular aldol condensation of dialdehydes or diones for producing cyclic α,β-unsaturated carboxyl compounds;
- Michael addition of a ketone or a β-dicarbonyl compound onto an α,β-unsaturated carboxyl compound for producing saturated polycarboxyl compounds;
- Robinson annulation for producing saturated polycyclic carboxyl compounds;
- Heck reactions of an aryl halide or a 1-hetero-2,4-cyclopentadiene or a benzo-fused derivative thereof with an α-ethylenically unsaturated compound for producing arylalkenes or heteroarylalkenes;
- codimerisation of alkenes for producing higher saturated hydrocarbons or alkynes for producing higher alkenes;
- hydroxylation of olefins for producing alcohols;
- hydroamination of olefins and alkynes for producing amines;
- alkylation, preferably allylic alkylation, of ketones for producing alkylated ketones, preferably allylic ketones; and
- Diels-Alder reactions such as the cycloaddition of a conjugated diene onto an α-ethylenically unsaturated compound for producing optionally substituted cyclohexenes, or the cycloaddition of furan onto an α-ethylenically unsaturated compound for producing optionally substituted 7-oxanorbornenes.

49. A method according to claim 47, wherein the said metathesis reaction is the ring-opening metathesis polymerisation of strained cyclic olefins.

50. A method according to claim 47, wherein the catalytic component is supported on a carrier.

51. A method according to claim 50, wherein the said carrier is selected from the group consisting of porous inorganic solids, such as amorphous or paracrystalline materials, crystalline molecular sieves and modified layered materials including one or more inorganic oxides, and organic polymer resins.

## Patentansprüche

1. Verfahren zur Modifikation eines mehrfach koordinierten Metallkomplexes, eines Salzes, eines Solvats oder eines Enantiomers davon, wobei der mehrfach koordinierte Metallkomplex umfasst: (i) mindestens einen mehrzähnigen Schiff-Base-Liganden, umfassend eine Iminogruppe, und mit dem Metall koordiniert, zusätzlich zu dem Stickstoffatom der Iminogruppe, durch mindestens ein weiteres Heteroatom, ausgewählt aus der Gruppe, bestehend aus Sauerstoff, Schwefel und Selen, und (ii) einen oder mehrere weitere(n) Liganden, wobei das Metall aus der Gruppe, bestehend aus Ruthenium, Osmium und Eisen, ausgewählt ist und wobei die Anzahl an Kohlenstoffatomen in dem wenigstens einen mehrzähnigen Schiff-Base-Liganden (i) zwischen dem Stickstoffatom der Iminogruppe und dem koordinierenden Heteroatom des wenigstens einen mehrzähnigen Schiff-Base-Liganden (i) 2 bis 4 ist und wobei mindestens einer der weiteren Liganden (ii) ein Carben-Ligand ist, ausgewählt aus der Gruppe, bestehend aus N-heterocyclischen Carbenen, Alkylidenliganden, Vinylidenliganden, Indenylidenliganden und Allenylidenliganden, wobei das Verfahren umfasst: Inkontaktbringen des mehrfach koordinierten Metallkomplexes mit einer Säure unter solchen Bedingungen, dass die Säure fähig ist, eine Bindung zwischen dem Metall und dem mindestens einen mehrzähnigen Schiff-Base-Liganden (i) zumindest teilweise zu spalten, und wobei die weiteren Liganden (ii) so gewählt sind, dass sie unter den genannten Bedingungen nicht durch die Säure protoniert werden können, und nicht aus der Gruppe, bestehend aus Phosphinen, Aminen, Arsinen und Stibinen, ausgewählt sind.

2. Verfahren gemäß Anspruch 1, **dadurch gekennzeichnet, dass** die Bedingungen ein Molverhältnis zwischen der Säure und dem mehrfach koordinierten Metallkomplex umfassen, das über 1,2 und unter 40 liegt.

3. Verfahren gemäß Anspruch 1 oder Anspruch 2, **dadurch gekennzeichnet, dass** die Bedingungen eine Kontaktzeit von 5 Sekunden bis 100 Stunden umfassen.

4. Verfahren gemäß einem der Ansprüche 1 bis 3, **dadurch gekennzeichnet, dass** die Bedingungen eine Kontakttemperatur von etwa -50°C bis etwa +80°C umfassen.

5. Verfahren gemäß einem der Ansprüche 1 bis 4, wobei der pKa der Säure niedriger als der pKa des mehrzähnigen Schiff-Base-Liganden (i) ist.

6. Verfahren gemäß einem der Ansprüche 1 bis 5, **dadurch gekennzeichnet, dass** mindestens einer der weiteren Liganden (ii) ein eingeschränkter Ligand mit sterischer Hinderung mit einem pKa von wenigstens 15 ist.

7. Verfahren gemäß einem der Ansprüche 1 bis 6, **dadurch gekennzeichnet, dass** mindestens einer der weiteren Liganden (ii) ein anionischer Ligand ist.

8. Verfahren gemäß einem der Ansprüche 1 bis 6, **dadurch gekennzeichnet, dass** mindestens einer der weiteren Liganden (ii) ein nicht-anionischer Ligand ist.

9. Verfahren gemäß einem der Ansprüche 1 bis 8, **dadurch gekennzeichnet, dass** die Säure Chlorwasserstoffsäure oder Bromwasserstoffsäure ist.

10. Verfahren gemäß einem der Ansprüche 1 bis 9, **dadurch gekennzeichnet, dass** die Bedingungen geeignet sind, den mehrzähnigen Schiff-Base-Liganden zu protonieren und das Stickstoffatom der Iminogruppe des mehrzähnigen Schiff-Base-Liganden von dem Metall zu dekoordinieren.

11. Verfahren gemäß einem der Ansprüche 1 bis 10, **dadurch gekennzeichnet, dass** die Bedingungen geeignet sind, das weitere Heteroatom des mehrzähnigen Schiff-Base-Liganden von dem Metall zu dekoordinieren.

12. Reaktionsprodukt aus:
(a) einem mehrfach koordinierten Metallkomplex, einem Salz, einem Solvat oder einem Enantiomer davon, wobei der mehrfach koordinierte Metallkomplex umfasst: (i) mindestens einen mehrzähnigen Schiff-Base-Liganden, umfassend eine Iminogruppe, und mit dem Metall koordiniert, zusätzlich zum Stickstoffatom der Iminogruppe, durch mindestens ein weiteres Heteroatom, ausgewählt aus der Gruppe, bestehend aus Sauerstoff, Schwefel und Selen, wobei die Anzahl an Kohlenstoffatomen in dem mindestens einen mehrzähnigen Schiff-Base-Liganden (i) zwischen dem Stickstoffatom der Aminogruppe und dem koordinierenden Heteroatom des mindestens einen mehrzähnigen Schiff-Base-Liganden (i) 2 bis 4 ist, und (ii) einen oder mehrere andere(n) Liganden, wobei der mindestens eine andere Ligand (ii) ein Carbenligand ist, ausgewählt aus der Gruppe, bestehend aus N-heterocyclischen Carbenen, Alkylidenliganden, Vinylidenliganden, Indenylidenliganden und Allenylidenliganden, wobei das Metall aus der Gruppe, bestehend aus Ruthenium, Osmium und Eisen, ausgewählt ist, und
(b) einer Säure, umgesetzt in einem Molverhältnis von über etwa 1,2 bezüglich des mehrfach koordinierten Metallkomplexes (a),
mit der Maßgabe, dass die weiteren Liganden (ii) ungeeignet sind, durch die Säure protoniert zu werden, und nicht aus der Gruppe, bestehend aus Aminen, Phosphinen, Arsinen und Stibinen, ausgewählt sind.

13. Produkt gemäß Anspruch 12, **dadurch gekennzeichnet, dass** der pKa der Säure (b) niedriger als der pKa des mindestens einen mehrzähnigen Schiff-Base-Liganden (i) ist.

14. Produkt gemäß einem der Ansprüche 12 bis 13, **dadurch gekennzeichnet, dass** mindestens einer der weiteren Liganden (ii) des mehrfach koordinierten Metallkomplexes (a) ein eingeschränkter Ligand mit sterischer Hinderung mit einem pKa von wenigstens 15 ist.

15. Produkt gemäß einem der Ansprüche 12 bis 14, **dadurch gekennzeichnet, dass** mindestens einer der weiteren Liganden (ii) des mehrfach koordinierten Metallkomplexes (a) ein anionischer Ligand ist.

16. Produkt gemäß einem der Ansprüche 12 bis 14, **dadurch gekennzeichnet, dass** mindestens einer der weiteren Liganden (ii) des mehrfach koordinierten Metallkomplexes (a) ein nicht-anionischer Ligand ist.

17. Produkt gemäß einem der Ansprüche 12 bis 16, **dadurch gekennzeichnet, dass** die Säure Fluorwasserstoffsäure oder Bromwasserstoffsäure ist.

18. Produkt gemäß einem der Ansprüche 12 bis 17, welches eine monometallische Spezies ist, dargestellt durch die allgemeine Formel:
[M (L_{c}) (L₂) (X) (SB⁺)]X⁻
wobei
- M ein Metall, ausgewählt aus der Gruppe, bestehend aus Ruthenium, Osmium und Eisen, ist;
- SB⁺ ein protonierter Schiff-Base-Ligand ist;
- L_{c} ein Carbenligand ist, ausgewählt aus der Gruppe, bestehend aus Alkylidenliganden, Vinylidenliganden, Indenylidenliganden und Allenylidenliganden;
- L₂ ein nicht-anionischer Ligand ist;
- X ein anionischer Ligand ist und
- X⁻ ein Anion ist,
Salze, Solvate und Enantiomere davon.

19. Produkt gemäß einem der Ansprüche 12 bis 17, welches eine bimetallische Spezies ist, dargestellt durch die allgemeine Formel:
[M (L_{c}) (SB⁺) (X₁) (X₂) (M') (X₃) (L)]X⁻
wobei
- M und M' jeweils ein Metall sind, unabhängig ausgewählt aus der Gruppe, bestehend aus Ruthenium, Osmium und Eisen;
- SB⁺ ein protonierter Schiff-Base-Ligand ist;
- L_{c} ein Carbenligand ist, ausgewählt aus der Gruppe, bestehend aus Alkylidenliganden, Vinylidenliganden, Indenylidenliganden und Allenylidenliganden;
- L ein nicht-anionischer Ligand ist;
- X₁, X₂ und X₃ jeweils unabhängig aus anionischen Liganden ausgewählt sind und
- X⁻ ein Anion ist,
Salze, Solvate und Enantiomere davon.

20. Produkt gemäß einem der Ansprüche 12 bis 17, welches eine kationische monometallische Spezies, dargestellt durch die allgemeine Formel (VI): 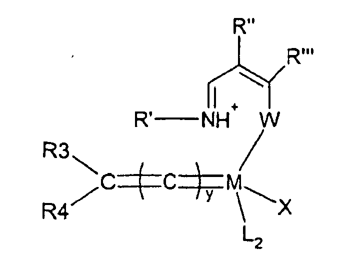 oder eine kationische monometallische Spezies ist, dargestellt durch die allgemeine Formel (VII): 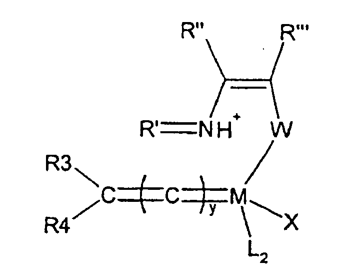 wobei
- M ein Metall ist, ausgewählt aus der Gruppe, bestehend aus Ruthenium, Osmium und Eisen;
- W ausgewählt ist aus der Gruppe, bestehend aus Sauerstoff, Schwefel, Selen, NR"", PR"", AsR"" und SbR"";
- R", R"' und R"" jeweils ein Rest sind, der unabhängig ausgewählt ist aus der Gruppe, bestehend aus Wasserstoff, C₁₋₆-Alkyl, C₃₋₈-Cycloalkyl, C₁₋₆-Alkyl-C₁₋₆-alkoxysilyl, C₁₋₆-Alkyl-aryloxysilyl, C₁₋₆-Alkyl-C₃₋₁₀-cycloalkylsilyl, Aryl und Heteroaryl, oder R" und R'" zusammen einen Aryl- oder Heteroarylrest bilden, wobei jeder Rest (wenn von Wasserstoff verschieden) gegebenenfalls mit einem oder mehreren, vorzugsweise 1 bis 3, Substituenten R₅, jeweils unabhängig ausgewählt aus der Gruppe, bestehend aus Halogenatomen, C₁₋₆-Alkyl, C₁₋₆-Alkoxy, Aryl, Alkylsulfonat, Arylsulfonat, Alkylphosphonat, Arylphosphonat, C₁₋₆-Alkyl-C₁₋₆-alkoxysilyl, C₁₋₆-Alkyl-aryloxysilyl, C₁₋₆-Alkyl-C₃₋₁₀-cycloalkoxysilyl, Alkylammonium und Arylammonium, substituiert ist;
- R' entweder wie für R", R'" und R"" definiert ist, wenn es in einer Verbindung mit der allgemeinen Formel (IA) enthalten ist, oder, wenn es in einer Verbindung mit der allgemeinen Formel (IB) enthalten ist, aus der Gruppe, bestehend aus C₁₋₆-Alkylen und C₃₋₈-Cycloalkylen, wobei die Alkylen- oder Cycloalkylengruppe gegebenenfalls mit einem oder mehreren Substituenten R₅ substituiert ist, ausgewählt ist;
- L₂ ein nicht-anionischer Ligand ist;
- X ein anionischer Ligand ist;
- R₃ und R₄ jeweils Wasserstoff oder ein Rest, ausgewählt aus der Gruppe, bestehend aus, C₁₋₂₀-Alkyl, C₂₋₂₀-Alkenyl, C₂₋₂₀-Alkinyl, C₁₋₂₀-Carboxylat, C₁₋₂₀-Alkoxy, C₂₋₂₀-Alkenyloxy, C₂₋₂₀-Alkinyloxy, Aryl, Aryloxy, C₁₋₂₀-Alkoxycarbonyl, C₁₋₈-Alkylthio, C₁₋₂₀-Alkylsulfonyl, C₁₋₂₀-Alkylsulfinyl, C₁₋₂₀-Alkylsulfonat, Arylsulfonat, C₁₋₂₀-Alkylphosphonat, Arylphosphonat, C₁₋₂₀-Alkylammonium und Arylammonium, sind;
- R' und eines von R₃ und R₄ unter Bildung eines zweizähnigen Liganden miteinander verbunden sein können;
- R'" und R"" unter Bildung eines aliphatischen Ringsystems, umfassend ein Heteroatom, ausgewählt aus der Gruppe, bestehend aus Stickstoff, Phosphor, Arsen und Antimon, aneinander gebunden sein können;
- R₃ und R₄ zusammen ein kondensiertes aromatisches Ringsystem bilden können und
- y die Anzahl von sp₂-Kohlenstoffatomen zwischen M und dem Kohlenstoff, welches R₃ und R₄ trägt, darstellt und eine ganze Zahl von 0 bis 3, einschließlich, ist,
Salze, Solvate und Enantiomere davon und wobei die kationische Spezies mit einem Anion assoziiert ist.

21. Produkt gemäß einem der Ansprüche 12 bis 17, welches eine kationische bimetallische Spezies, dargestellt durch die allgemeine Formel (X): 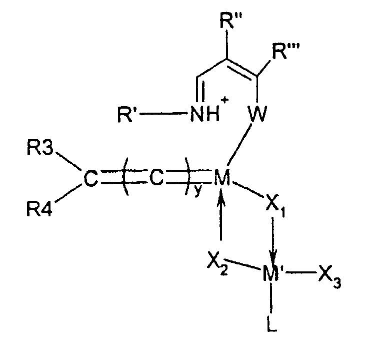 oder eine kationische bimetallische Spezies ist, dargestellt durch die allgemeine Formel (XI): 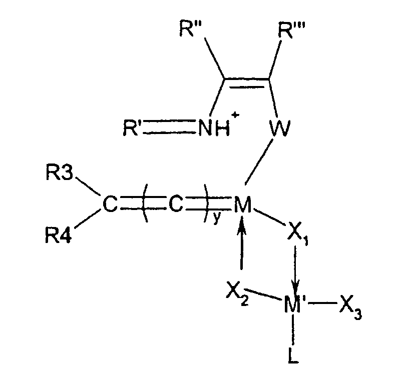 wobei
- M und M' jeweils ein Metall sind, unabhängig ausgewählt aus der Gruppe, bestehend aus Ruthenium, Osmium und Eisen;
- W ausgewählt ist aus der Gruppe, bestehend aus Sauerstoff, Schwefel, Selen, NR"", PR"", AsR"" und SbR"";
- R", R'" und R"" jeweils ein Rest sind, der unabhängig ausgewählt ist aus der Gruppe, bestehend aus Wasserstoff, C₁₋₆-Alkyl, C₃₋₈-Cycloalkyl, C₁₋₆-Alkyl-C₁₋₆-alkoxysilyl, C₁₋₆-Alkyl-aryloxysilyl, C₁₋₆-Alkyl-C₃₋₁₀-cycloalkylsilyl, Aryl und Heteroaryl, oder R" und R'" zusammen einen Aryl- oder Heteroarylrest bilden, wobei jeder Rest (wenn von Wasserstoff verschieden) gegebenenfalls mit einem oder mehreren, vorzugsweise 1 bis 3, Substituenten R₅, jeweils unabhängig ausgewählt aus der Gruppe, bestehend aus Halogenatomen, C₁₋₆-Alkyl, C₁₋₆-Alkoxy, Aryl, Alkylsulfonat, Arylsulfonat, Alkylphosphonat, Arylphosphonat, C₁₋₆-Alkyl-C₁₋₆-alkoxysilyl, C₁₋₆-Alkyl-aryloxysilyl, C₁₋₆-Alkyl-C₃-₁₀-cycloalkoxysilyl, Alkylammonium und Arylammonium, substituiert ist;
- R' entweder wie für R", R'" und R"" definiert ist, wenn es in einer Verbindung mit der allgemeinen Formel (IA) enthalten ist, oder, wenn es in einer Verbindung mit der allgemeinen Formel (IB) enthalten ist, aus der Gruppe, bestehend aus C₁₋₆-Alkylen und C₃₋₈-Cycloalkylen, wobei die Alkylen- oder Cycloalkylengruppe gegebenenfalls mit einem oder mehreren Substituenten R₅ substituiert ist, ausgewählt ist;
- R₃ und R₄ jeweils Wasserstoff oder ein Rest, ausgewählt aus der Gruppe, bestehend aus C₁₋₂₀-Alkyl, C₂₋₂₀-Alkenyl, C₂₋₂₀-Alkinyl, C₁₋₂₀-Carboxylat, C₁₋₂₀-Alkoxy, C₂₋₂₀-Alkenyloxy, C₂₋₂₀-Alkinyloxy, Aryl, Aryloxy, C₁₋₂₀-Alkoxycarbonyl, C₁₋₈-Alkylthio, C₁₋₂₀-Alkylsulfonyl, C₁₋₂₀-Alkylsulfinyl, C₁₋₂₀-Alkylsulfonat, Arylsulfonat, C₁₋₂₀-Alkylphosphonat, Arylphosphonat, C₁₋₂₀-Alkylammonium und Arylammonium, sind;
- R' und eines von R₃ und R₄ unter Bildung eines zweizähnigen Liganden miteinander verbunden sein können;
- R"' und R"" unter Bildung eines aliphatischen Ringsystems, umfassend ein Heteroatom, ausgewählt aus der Gruppe, bestehend aus Stickstoff, Phosphor, Arsen und Antimon, aneinander gebunden sein können;
- R₃ und R₄ zusammen ein kondensiertes aromatisches Ringsystem bilden können und
- y die Anzahl von sp₂-Kohlenstoffatomen zwischen M und dem Kohlenstoff, welches R₃ und R₄ trägt, darstellt und eine ganze Zahl von 0 bis 3, einschließlich, ist;
- X₁, X₂ und X₃ jeweils unabhängig ausgewählt sind aus anionischen Liganden und
- ein nicht-anionischer Ligand ist,
einschließlich Salze, Solvate und Enantiomere davon.

22. Produkt gemäß einem der Ansprüche 12 bis 17, **dadurch gekennzeichnet, dass** mindestens einer der weiteren Liganden (ii) des mehrfach koordinierten Metallkomplexes (a) ein Lösungsmittel S ist und der Komplex (a) eine kationische Spezies ist, die mit einem Anion A assoziiert ist.

23. Produkt gemäß einem der Ansprüche 12 bis 17, **dadurch gekennzeichnet, dass** der mehrfach koordinierte Metallkomplex (a) ein bimetallischer Komplex ist.

24. Produkt gemäß Anspruch 23, **dadurch gekennzeichnet, dass** ein Metall des bimetallischen Komplexes mit mindestens einem mehrzähnigen Schiff-Base-Liganden (i) und mit dem einen oder den mehreren weiteren Liganden (ii) pentakoordiniert ist und das andere Metall mit einem oder mehreren neutralen Liganden und einem oder mehren anionischen Liganden tetra-koordiniert ist.

25. Produkt gemäß Anspruch 23, **dadurch gekennzeichnet, dass** jedes Metall des bimetallischen Komplexes mit dem mindestens einen mehrzähnigen Schiff-Base-Liganden (i) und mit dem einen oder den mehreren weiteren Liganden (ii) hexa-koordiniert ist.

26. Produkt gemäß Anspruch 23, **dadurch gekennzeichnet, dass** die zwei Metalle des bimetallischen Komplexes die gleichen sind.

27. Produkt gemäß Anspruch 23, **dadurch gekennzeichnet, dass** die zwei Metalle des bimetallischen Komplexes unterschiedlich sind.

28. Produkt gemäß einem der Ansprüche 12 bis 17, **dadurch gekennzeichnet, dass** der mehrfach koordinierte Metallkomplex (a) ein monometallischer Komplex ist.

29. Produkt gemäß einem der Ansprüche 12 bis 17, **dadurch gekennzeichnet, dass** der mehrfach koordinierte Metallkomplex (a) ein penta-koordinierter Metallkomplex oder ein tetra-koordinierter Metallkomplex ist.

30. Produkt gemäß Anspruch 29, **dadurch gekennzeichnet, dass** der mindestens eine mehrzähnige Schiff-Base-Ligand (i) ein zweizähniger Ligand ist und der mehrfach koordinierte Metallkomplex (a) zwei weitere Liganden (ii) umfasst.

31. Produkt gemäß Anspruch 29, **dadurch gekennzeichnet, dass** der mindestens eine mehrzähnige Schiff-Basen-Ligand (i) ein dreizähniger Ligand ist und der mehrfach koordinierte Metallkomplex (a) einen einzelnen weiteren Liganden (ii) umfasst.

32. Produkt gemäß Anspruch 29 oder Anspruch 30, **dadurch gekennzeichnet, dass** der mindestens eine mehrzähnige Schiff-Basen-Ligand (i) eine der allgemeinen Formeln (IA) und (IB) hat: 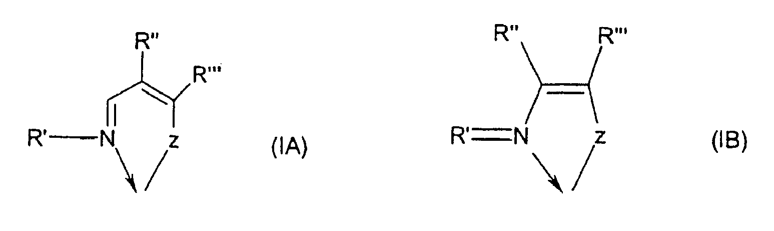 wobei:
- Z ausgewählt ist aus der Gruppe, bestehend aus Sauerstoff, Schwefel und Selen;
- R" und R'" jeweils ein Rest sind, unabhängig ausgewählt aus der Gruppe, bestehend aus Wasserstoff, C₁₋₇-Alkyl, C₃₋₁₀-Cycloalkyl, C₁₋₆-Alkyl-C₁₋₆-alkoxysilyl, C₁₋₆-Alkyl-aryloxysilyl, C₁₋₆-Alkyl-C₃₋₁₀-cycloalkoxysilyl, Aryl und Heteroaryl, oder R" und R"" zusammen einen Aryl- oder Heteroarylrest bilden, wobei jeder Rest gegebenenfalls mit einem oder mehreren, vorzugsweise 1 bis 3, Substituenten R₅, jeweils unabhängig ausgewählt aus der Gruppe, bestehend aus Halogenatomen, C₁₋₆-Alkyl, C₁₋₆-Alkoxyy, Aryl, Alkylsulfonat, Arylsulfonat, Alkylphosphonat, Arylphosphonat, C₁₋₆-Alkyl-C₁₋₆-alkoxysilyl, C₁₋₆-Alkyl-aryloxysilyl, C₁₋₆-Alkyl-C₃₋₁₀-cycloalkoxysilyl, Alkylammonium und Arylammonium, substituiert ist;
- R' entweder wie für R" und R"' definiert ist, wenn es in einer Verbindung mit der allgemeinen Formel (IA) enthalten ist, oder, wenn es in einer Verbindung mit der allgemeinen Formel (IB) enthalten ist, ausgewählt ist aus der Gruppe, bestehend aus C₁₋₇-Alkylen und C₃₋₁₀-Cycloalkylen, wobei die Alkylen- oder Cycloalkylengruppe gegebenenfalls mit einem oder mehreren Substituenten R₅ substituiert ist.

33. Produkt gemäß Anspruch 29, **dadurch gekennzeichnet, dass** mindestens einer der weiteren Liganden (ii) des mehrfach koordinierten Metallkomplexes (a) ein Derivat ist, bei dem ein oder mehrere Wasserstoffatome ersetzt sind durch eine Gruppe, die eine erzwungene sterische Hinderung bereitstellt, eines N-heterocyclischen Carbens, ausgewählt aus der Gruppe, bestehend aus Imidazol-2-yliden, Dihydroimidazol-2-yliden, Oxazol-2-yliden, Triazol-5-yliden, Thiazol-2-yliden, Bis(imidazolin-2-yliden), Bis(imidazolidin-2-yliden), Pyrrolyliden, Pyrazolyliden, Dihydropyrrolyliden, Pyrrolylidinyliden und benzo-kondensierten Derivaten davon, oder einer nicht-ionischen Prophosphatran-Superbase.

34. Produkt gemäß Anspruch 29, **dadurch gekennzeichnet, dass** wenigstens einer der weiteren Liganden (ii) des mehrfach koordinierten Metallkomplexes (a) ein anionischer Ligand ist, ausgewählt aus der Gruppe, bestehend aus C₁₋₂₀-Alkyl, C₁₋₂₀-Alkenyl, C₁₋₂₀-Alkinyl, C₁₋₂₀-Carboxylat, C₁₋₂₀-Alkoxy, C₁₋₂₀-Alkenyloxy, C₁₋₂₀-Alkinyloxy, Aryl, Aryloxy, C₁₋₂₀-Alkoxycarbonyl, C₁₋₈-Alkylthio, C₁₋₂₀-Alkylsulfonyl, C₁₋₂₀-Alkylsulfinyl, C₁₋₂₀-Alkylsulfonat, Arylsulfonat, C₁₋₂₀-Alkylphosphonat, Arylphosphonat, C₁₋₂₀-Alkylammonium, Arylammonium, Halogenatomen und Cyano.

35. Produkt gemäß Anspruch 29, **dadurch gekennzeichnet, dass** mindestens einer der weiteren Liganden (ii) des mehrfach koordinierten Metallkomplexes (a) ein Carbenligand ist, der durch die allgemeine Formel =[C=]_{y}CR₃R₄ dargestellt wird, wobei:
- y eine ganze Zahl von 0 bis 3, einschließlich, ist und
- R₃ und R₄ jeweils Wasserstoff oder ein Kohlenwasserstoffrest, ausgewählt aus der Gruppe, bestehend aus C₁₋₂₀-Alkyl, C₁₋₂₀-Alkenyl, C₁₋₂₀-Alkinyl, C₁₋₂₀-Carboxylat, C₁₋₂₀-Alkoxy, C₁₋₂₀-Alkenyloxy, C₁₋₂₀-Alkinyloxy, Aryl, Aryloxy, C₁₋₂₀-Alkoxycarbonyl, C₁₋₈-Alkylthio, C₁₋₂₀-Alkylsulfonyl, C₁₋₂₀-Alkylsulfinyl, C₁₋₂₀-Alkylsulfonat, Arylsulfonat, C₁₋₂₀-Alkylphosphonat, Arylphosphonat, C₁₋₂₀-Alkylammonium und Arylammonium, sind oder R₃ und R₄ zusammen ein kondensiertes aromatisches Ringsystem bilden können.

36. Produkt gemäß Anspruch 35, **dadurch gekennzeichnet, dass** R₃ und R₄ zusammen ein kondensiertes aromatisches Ringsystem bilden, derart, dass der weitere Ligand (ii) des mehrfach koordinierten Metallkomplexes (a) ein Phenylindenylidenligand ist.

37. Produkt gemäß Anspruch 29, **dadurch gekennzeichnet, dass** mindestens einer der weiteren Liganden (ii) des mehrfach koordinierten Metallkomplexes (a) ein nicht-anionischer ungesättigter Ligand L¹ ist, ausgewählt aus der Gruppe, bestehend aus aromatischen und ungesättigten cycloaliphatischen Gruppen, vorzugsweise Aryl-, Heteroaryl- und C₄₋₂₀-Cycloalkenylgruppen, wobei die aromatische oder ungesättigte cycloaliphatische Gruppe gegebenenfalls mit einer oder mehreren C₁₋₇-Alkylgruppen oder Elektronen anziehenden Gruppen, zum Beispiel, aber nicht beschränkt auf, Halogen, Nitro, Cyano, (Thio)carbonsäure, (Thio)carbonsäure-(thio)ester, (Thio)carbonsäure-(thio)amid, (Thio)carbonsäureanhydrid und (Thio)carbonsäurehalogenid, substituiert ist.

38. Produkt gemäß Anspruch 29, **dadurch gekennzeichnet, dass** mindestens einer der weiteren Liganden (ii) des mehrfach koordinierten Metallkomplexes (a) ein nicht-anionischer Ligand L², ausgewählt aus der Gruppe, bestehend aus C₁₋₇-Alkyl, C₃₋₁₀-Cycloalkyl, Arylalkyl und Heterocyclyl, ist, wobei die Gruppe gegebenenfalls mit einem oder mehreren vorzugsweise elektronenanziehenden Substituenten, zum Beispiel, aber nicht beschränkt auf, Halogen, Nitro, Cyano, (Thio)carbonsäure, (Thio)carbonsäure-(thio)ester, (Thio)carbonsäure-(thio)amid, (Thio)carbonsäureanhydrid und (Thio)carbonsäurehalogenid, substituiert ist.

39. Produkt gemäß Anspruch 29, **dadurch gekennzeichnet, dass** der mindestens eine mehrzähnige Schiff-Basen-Ligand (i) ein vierzähniger Ligand ist und der mehrfach koordinierte Metallkomplex (a) einen oder zwei andere Liganden (ii) umfasst, die nicht-anionische Liganden L⁷ sind, ausgewählt aus der Gruppe, bestehend aus aromatischen und ungesättigten cycloaliphatischen Gruppen, vorzugsweise Aryl-, Heteroaryl- und C₄₋₂₀-Cycloalkenylgruppen, wobei die aromatische oder ungesättigte cycloaliphatische Gruppe gegebenenfalls mit einer oder mehreren C₁₋₇-Alkylgruppen oder Elektronen anziehenden Gruppen, zum Beispiel, aber nicht beschränkt auf, Halogen, Nitro, Cyano, (Thio)carbonsäure, (Thio)carbonsäure-(thio)ester, (Thio)carbonsäure-(thio)amid, (Thio)carbonsäureanhydrid und (Thio)carbonsäurehalogenid, substituiert ist.

40. Produkt gemäß Anspruch 12, wobei die Säure (b) eine Säure ist, die in situ aus einem Photosäure-Erzeugungsmittel erzeugt wird.

41. Produkt gemäß Anspruch 12, umfassend das Produkt einer wenigstens partiellen Spaltung einer Bindung zwischen dem Metall und dem mindestens einen mehrzähnigen Schiff-Base-Liganden (i).

42. Katalytisches System, umfassend:
(a) als die katalytische Hauptspezies ein Reaktionsprodukt gemäß einem der Ansprüche 12 bis 41 und
(b) eine oder mehrere zweite Katalysatorkomponenten, ausgewählt aus der Gruppe, bestehend aus Lewis-Säure-CoKatalysatoren (b₁), Katalysatoraktivatoren (b₂) und Initiatoren, die ein(e) radikalisch übertragbare(s) Atom oder Gruppe haben, (b₃).

43. Katalytisches System gemäß Anspruch 42, wobei die zweite Katalysatorkomponente einen Co-Katalysator (b₁) umfasst, der aus der Gruppe, bestehend aus Bortrihalogeniden; Phosphortrihalogeniden; Trialkylborverbindungen; Triarylborverbindungen; Organoaluminiumverbindungen; Magnesiumdihalogeniden; Aluminiumtrihalogeniden; Zinntetrachlorid; Titan- oder Vanadiumtrihalogeniden oder -tetrahalogeniden oder -tetraalkoxiden; Antimon- und Wismutpentahalogeniden, ausgewählt ist.

44. Katalytisches System gemäß Anspruch 42, wobei die zweite Katalysatorkomponente als Katalysatoraktivator (b₂) eine Diazoverbindung umfasst.

45. Katalytisches System gemäß Anspruch 42, wobei die zweite Katalysatorkomponente als Initiator, der ein(e) radikalisch übertragbare(s) Atom oder Gruppe hat, (b₃), eine Verbindung mit der Formel R₃₅R₃₆R₃₇CX₁ umfasst, wobei:
- X₁ ausgewählt ist aus der Gruppe, bestehend aus Halogen, OR₃₈ (worin R₃₈ ausgewählt ist aus C₁₋₂₀-Alkyl, Polyhalogen-C₁₋₂₀-alkyl, C₂₋₂₀-Alkinyl (vorzugsweise Acetylenyl), C₂₋₂₀-Alkenyl (vorzugsweise Vinyl), Phenyl, gegebenenfalls substituiert mit 1 bis 5 Halogenatomen oder C₁₋₇-Alkylgruppen, und Phenyl-substituiertem C₁₋₇-Alkyl), SR₃₉, OC(=O)R₃₉, OP(=O)R₃₉, OP(=O)(OR₃₉)₂, OP(=O)OR₃₉, O-N(R₃₉)₂ und S-C(=S)N(R₃₉)₂, wobei R₃₉ Aryl oder C₁₋₂₀-Alkyl ist oder wobei eine N(R₃₉)₂-Gruppe vorhanden ist, wobei zwei R₃₉-Gruppen unter Bildung eines 5-, 6- oder 7-gliedrigen heterocyclischen Rings (gemäß der Definition von Heteroaryl oben) verknüpft sein können, und
- R₃₅, R₃₆ und R₃₇ jeweils unabhängig ausgewählt sind aus der Gruppe, bestehend aus Wasserstoff, Halogen, C₁₋₂₀-Alkyl (vorzugsweise C₁₋₆-Alkyl), C₃₋₈-Cycloalkyl, C(=O)R₄₀ (worin R₄₀ ausgewählt ist aus der Gruppe, bestehend aus C₁₋₂₀-Alkyl, C₁₋₂₀-Alkoxy, Aryloxy oder Heteroaryloxy), C(=O)NR₄₁R₄₂ (wobei R₄₁ und R₄₂ unabhängig ausgewählt sind aus der Gruppe, bestehend aus Wasserstoff und C₁₋₂₀-Alkyl, oder R₄₁ und R₄₂ unter Bildung einer Alkylengruppe mit 2 bis 5 Kohlenstoffatomen miteinander verknüpft sein können), COCl, OH, CN, C₂₋₂₀-Alkenyl (vorzugsweise Vinyl), C₂₋₂₀-Alkinyl, Oxiranyl, Glycidyl, Aryl, Heteroaryl, Arylalkyl und Aryl-substituiertem C₂₋₂₀-Alkenyl.

46. Trägerkatalysator, umfassend:
(a) ein katalytisch aktives Reaktionsprodukt gemäß einem der Ansprüche 12 bis 41 oder ein katalytisches System gemäß Anspruch 46 und
(b) eine tragende Menge eines Trägers, der zum Tragen des katalytisch aktiven Produkts oder des katalytischen Systems (a) geeignet ist.

47. Verfahren zur Durchführung einer Olefin- oder Acetylen-Metathesereaktion oder einer Reaktion, die den Transfer eines Atoms oder einer Gruppe auf eine ethylenisch oder acetylenisch ungesättigte Verbindung oder ein anderes reaktives Substrat in Gegenwart einer katalytischen Komponente umfasst, wobei die katalytische Komponente ein Produkt gemäß einem der Ansprüche 12 bis 41 oder ein katalytisches System gemäß einem der Ansprüche 42 bis 45 umfasst.

48. Verfahren gemäß Anspruch 47, wobei die Reaktion, die den Transfer eines Atoms oder einer Gruppe zu einem Olefin oder einem anderen reaktiven Substrat involviert, ausgewählt ist aus der Gruppe, bestehend aus:
- einer Atom- oder Gruppen-Transfer-Radikalpolymerisation eines oder mehrerer radikalisch (co)polymerisierbarer Monomere, speziell mono- und diethylenisch ungesättigter Monomere;
- Atomtransfer-Radikaladdition eines Polyhalogenmethans der Formel CXₙH₄₋ₙ, worin X Halogen ist und n eine ganze Zahl von 2 bis 4 ist, an eine ethylenisch ungesättigte Verbindung, um das entsprechende gesättigte polyhalogenierte Addukt zu produzieren;
- Vinylierungsreaktion eines Mono- oder Dialkyls mit einer Mono- oder Dicarbonsäure unter Herstellung von Alk-1-enylestern oder Enolestern oder Markovnikov-Addukten oder Anti-Markovnikov-Addukten oder Gemischen davon;
- Cyclopropanierung einer α-ethylenisch ungesättigten Verbindung zur Herstellung einer organischen Verbindung, die eine oder mehrere Cyclopropan-Struktureinheit(en) hat,
- Chinolinsynthese durch oxidative Cyclisierung von 2-Aminobenzylalkohol mit Ketonen;
- Epoxidierung von α-ethylenisch ungesättigten Verbindungen zur Herstellung von Epoxiden;
- Oxidation von organischen Verbindungen, einschließlich der Oxidation von gesättigten Kohlenwasserstoffen zur Herstellung von Alkoholen oder von Sulfiden zur Herstellung von Sulfoxiden und von Sulfonen oder Phosphinen zur Herstellung von Phosphonaten oder von Alkoholen und Aldehyden zur Herstellung von Carbonsäuren;
- Cyclopropenierung eins Alkins zur Herstellung einer organischen Verbindung, die eine oder mehrere Cyclopropen-Struktureinheit(en) hat;
- Hydrocyanierung von α-ethylenisch ungesättigten Verbindungen zur Herstellung von gesättigten Nitrilen oder von Alkylen zur Herstellung von ungesättigten Nitrilen oder von α,β-ungesättigten Aldehyden oder Ketonen zur Herstellung von β-Cyanocarbonylverbindungen;
- Hydrosilylierung von Olefinen zur Herstellung von gesättigten Silanen oder von Alkinen zur Herstellung von ungesättigten Silanen oder von Ketonen zur Herstellung von Silylethern oder Trimethylsilylcyanierung von Aldehyden zur Herstellung von Cyanhydrintrimethylsilylethern;
- Aziridinierung von Iminen oder Alkenen zur Herstellung von organischen Verbindungen, die eine oder mehrere Aziridin-Struktureinheit(en) haben;
- Hydroamidierung von Olefinen zur Herstellung von gesättigten Amiden;
- Hydrierung von Olefinen zur Herstellung von Alkanen oder von Ketonen zur Herstellung von Alkoholen;
- Aminolyse von Olefinen zur Herstellung von gesättigten primären oder sekundären Aminen;
- Isomerisierung von Alkoholen, vorzugsweise allylischen Alkoholen, zur Herstellung von Aldehyden;
- Grignard-Kupplung von Alkyl- oder Arylhalogeniden zur Herstellung von Alkanen oder Arylalkanen;
- Hydroborierung von Olefinen zur Herstellung von Alkylboranen und Trialkylboranen;
- Hydridreduktion von Aldehyden und Ketonen zur Herstellung von Alkoholen;
- Aldolkondensation von gesättigten Carboxylverbindungen zur Herstellung von α,β-ungesättigen Carboxylverbindungen oder β-Hydroxycarbonylverbindungen und intramolekulare Aldolkondensation von Dialdehyden oder Dionen zur Herstellung von cyclischen α,β-ungesättigten Carboxylverbindungen;
- Michael-Addition eines Ketons oder einer β-Dicarbonylverbindung an eine α,β-ungesättigte Carboxylverbindung zur Herstellung von gesättigten Polycarboxylverbindungen;
- Robinson-Anellierung zur Herstellung von gesättigten polycyclischen Carboxylverbindungen;
- Heck-Reaktionen eines Arylhalogenids oder eines 1-Hetero-2,4-cyclopentadiens oder eines benzo-kondensierten Derivats davon mit einer α-ethylenisch ungesättigten Verbindung zur Herstellung von Arylalkenen oder Heteroarylalkenen;
- Codimerisierung von Alkenen zur Herstellung von höheren gesättigten Kohlenwasserstoffen oder von Alkinen zur Herstellung höherer Alkene;
- Hydroxylierung von Olefinen zur Herstellung von Alkoholen;
- Hydroaminierung von Olefinen und Alkinen zur Herstellung von Aminen;
- Alkylierung, vorzugsweise allylischer Alkylierung, von Ketonen zur Herstellung alkylierter Ketone, vorzugsweise allylischer Ketone, und
- Diels-Alder-Reaktionen, zum Beispiel die Cycloaddition eines konjugierten Diens an eine α-ethylenisch ungesättigte Verbindung zur Herstellung gegebenenfalls substituierter Cyclohexene oder die Cycloaddition von Furan an eine α-ethylenisch ungesättigte Verbindung zur Herstellung gegebenenfalls substituierter 7-Oxanorbornene.

49. Verfahren gemäß Anspruch 47, wobei die Metathesereaktion die Ringöffnungs-Metathese-Polymerisation von gespannten cyclischen Olefinen ist.

50. Verfahren gemäß Anspruch 47, wobei die katalytische Komponente auf einem Träger getragen wird.

51. Verfahren gemäß Anspruch 50, wobei der Träger ausgewählt wird aus der Gruppe, bestehend aus porösen anorganischen Feststoffen, zum Beispiel amorphen oder parakristallinen Materialien, kristallinen Molekularsieben und modifizierten Schichtmaterialien, umfassend ein oder mehrere anorganische Oxide und organische Polymerharze.

## Revendications

1. Procédé de modification d'un complexe métallique multi-coordonné, d'un sel, d'un solvate ou d'un énantiomère de celui-ci, ledit complexe métallique multi-coordonné comprenant (i) au moins un ligand multidenté de type base de Schiff comprenant un groupe imino et étant coordonné au métal, en plus de l'atome d'azote dudit groupe imino, par l'intermédiaire d'au moins un autre hétéroatome choisi dans le groupe constitué par un atome d'oxygène, un atome de soufre et un atome de sélénium, et (ii) un ou plusieurs autres ligands, dans lequel ledit métal est choisi dans le groupe constitué par le ruthénium, l'osmium et le fer, et dans lequel le nombre d'atomes de carbone dudit au moins un ligand multidenté de type base de Schiff (i), entre l'atome d'azote dudit groupe imino et ledit hétéroatome de coordination dudit au moins un ligand multidenté de type base de Schiff (i), est de 2 à 4, et dans lequel au moins un desdits autres ligands (ii) est un ligand de type carbène choisi dans le groupe constitué par les carbènes N-hétérocycliques, les ligands de type alkylidène, les ligands de type vinylidène, les ligands de type indénylidène et les ligands de type allénylidène, ledit procédé comprenant la mise en contact dudit complexe métallique multi-coordonné avec un acide dans des conditions telles que ledit acide est capable de couper au moins partiellement une liaison entre le métal et ledit au moins un ligand multidenté de type base de Schiff (i), et dans lequel lesdits autres ligands (ii) sont choisis de sorte à ne pas pouvoir être protonés par ledit acide dans lesdites conditions, et ne sont pas choisis dans le groupe constitué par les phosphines, les amines, les arsines et les stibines.

2. Procédé selon la revendication 1, **caractérisé en ce que** lesdites conditions comprennent un rapport molaire entre ledit acide et ledit complexe métallique multi-coordonné supérieur à 1,2 et inférieur à 40.

3. Procédé selon la revendication 1 ou 2, **caractérisé en ce que** lesdites conditions comprennent un temps de contact de 5 secondes à 100 heures.

4. Procédé selon l'une quelconque des revendications 1 à 3, **caractérisé en ce que** lesdites conditions comprennent une température de contact d'environ -50°C à environ +80 °C.

5. Procédé selon l'une quelconque des revendications 1 à 4, dans lequel le pKa dudit acide est inférieur au pKa dudit ligand multidenté de type base de Schiff (i).

6. Procédé selon l'une quelconque des revendications 1 à 5, **caractérisé en ce qu'**au moins un desdits autres ligands (ii) est un ligand stériquement encombré ayant un pKa d'au moins 15.

7. Procédé selon l'une quelconque des revendications 1 à 6, **caractérisé en ce qu'**au moins un desdits autres ligands (ii) est un ligand anionique.

8. Procédé selon l'une quelconque des revendications 1 à 6, **caractérisé en ce qu'**au moins un desdits autres ligands (ii) est un ligand non anionique.

9. Procédé selon l'une quelconque des revendications 1 à 8, **caractérisé en ce que** ledit acide est l'acide chlorhydrique ou l'acide bromhydrique.

10. Procédé selon l'une quelconque des revendications 1 à 9, **caractérisé en ce que** lesdites conditions sont capables de protoner le ligand multidenté de type base de Schiff et de décoordonner l'atome d'azote, du groupe imino dudit ligand multidenté de type base de Schiff, du métal.

11. Procédé selon l'une quelconque des revendications 1 à 10, **caractérisé en ce que** lesdites conditions sont capables de décoordonner l'autre hétéroatome, dudit ligand multidenté de type base de Schiff, du métal.

12. Produit réactionnel de :
(a) un complexe métallique multi-coordonné, un sel, un solvate ou un énantiomère de celui-ci, ledit complexe métallique multi-coordonné comprenant (i) au moins un ligand multidenté de type base de Schiff comprenant un groupe imino et étant coordonné au métal, en plus de l'atome d'azote dudit groupe imino, par l'intermédiaire d'au moins un autre hétéroatome choisi dans le groupe constitué par un atome d'oxygène, un atome de soufre et un atome de sélénium, dans lequel le nombre d'atomes de carbone dudit au moins un ligand multidenté de type base de Schiff (i), entre l'atome d'azote dudit groupe imino et ledit hétéroatome de coordination dudit au moins un ligand multidenté de type base de Schiff (i), est de 2 à 4, et (ii) un ou plusieurs autres ligands, dans lequel au moins un desdits autres ligands (ii) est un ligand de type carbène choisi dans le groupe constitué par les carbènes N-hétérocycliques, les ligands de type alkylidène, les ligands de type vinylidène, les ligands de type indénylidène et les ligands de type allénylidène, dans lequel ledit métal est choisi dans le groupe constitué par le ruthénium, l'osmium et le fer, et
(b) un acide ayant réagi selon un rapport molaire au-dessus d'environ 1,2 par rapport audit complexe métallique multi-coordonné (a),
à condition que lesdits autres ligands (ii) ne puissent pas être protonés par ledit acide et ne soient pas choisis dans le groupe constitué par les amines, les phosphines, les arsines et les stibines.

13. Produit selon la revendication 12, **caractérisé en ce que** le pKa dudit acide (b) est inférieur au pKa dudit au moins un ligand multidenté de type base de Schiff (i).

14. Produit selon l'une quelconque des revendications 12 à 13, **caractérisé en ce qu'**au moins un desdits autres ligands (ii) dudit complexe métallique multi-coordonné (a) est un ligand stériquement encombré ayant un pKa d'au moins 15.

15. Produit selon l'une quelconque des revendications 12 à 14, **caractérisé en ce qu'**au moins un desdits autres ligands (ii) dudit complexe métallique multi-coordonné (a) est un ligand anionique.

16. Produit selon l'une quelconque des revendications 12 à 14, **caractérisé en ce qu'**au moins un desdits autres ligands (ii) dudit complexe métallique multi-coordonné (a) est un ligand non anionique.

17. Produit selon l'une quelconque des revendications 12 à 16, **caractérisé en ce que** ledit acide est l'acide chlorhydrique ou l'acide bromhydrique.

18. Produit selon l'une quelconque des revendications 12 à 17, qui est une espèce monométallique représentée par la formule générale :
[M (L_{c}) (L₂) (X) (SB⁺)]X⁻
dans laquelle
- M est un métal choisi dans le groupe constitué par le ruthénium, l'osmium et le fer ;
- SB⁺ est un ligand protoné de type base de Schiff ;
- L_{c} est un ligand de type carbène choisi dans le groupe constitué par les ligands de type alkylidène, les ligands de type vinylidène, les ligands de type indénylidène et les ligands de type allénylidène ;
- L₂ est un ligand non anionique ;
- X est un ligand anionique ; et
- X⁻ est un anion ;
des sels, des solvates et des énantiomères de celui-ci.

19. Produit selon l'une quelconque des revendications 12 à 17, qui est une espèce bimétallique représentée par la formule générale :
[M(L_{c}) (SB⁺) (X₁) (X₂) (M') (X₃) (L)]X⁻
dans laquelle
- M et M' sont chacun un métal indépendamment choisi dans le groupe constitué par le ruthénium, l'osmium et le fer ;
- SB⁺ est un ligand protoné de type base de Schiff ;
- L_{c} est un ligand de type carbène choisi dans le groupe constitué par les ligands de type alkylidène, les ligands de type vinylidène, les ligands de type indénylidène et les ligands de type allénylidène ;
- L est un ligand non anionique ;
- X₁, X₂ et X₃ sont chacun indépendamment choisis parmi des ligands anioniques ; et
- X⁻ est un anion ;
des sels, des solvates et des énantiomères de celui-ci.

20. Produit selon l'une quelconque des revendications 12 à 17, qui est une espèce monométallique cationique représentée par la formule générale (VI) : 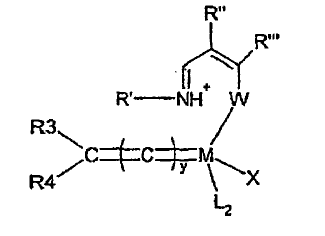 ou une espèce monométallique cationique représentée par la formule générale (VII) : 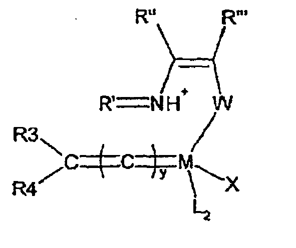 dans lesquelles
- M est un métal choisi dans le groupe constitué par le ruthénium, l'osmium et le fer ;
- W est choisi dans le groupe constitué par un atome d'oxygène, un atome de soufre, un atome de sélénium, NR"", PR"", AsR"" et SbR"" ;
- R", R"' et R"" sont chacun un groupe indépendamment choisi dans le groupe constitué par un atome d'hydrogène, un groupe alkyle en C₁-C₆, un groupe cycloalkyle en C₃-C₈, un groupe (alkyle en C₁-C₆)(alcoxy en C₁-C₆)silyle, un groupe (alkyle en C₁-C₆)aryloxysilyle, un groupe (alkyle en C₁-C₆)(cycloalcoxy en C₃-C₁₀)silyle, un groupe aryle et un groupe hétéroaryle, ou R" et R'" forment ensemble un groupe aryle ou hétéroaryle, chacun desdits groupes (lorsqu'ils ne sont pas un atome d'hydrogène) étant facultativement substitué par un ou plusieurs, de préférence 1 à 3, substituants R₅, chacun indépendamment choisi dans le groupe constitué par un atome d'halogène, un groupe alkyle en C₁-C₆, un groupe alcoxy en C₁-C₆, un groupe aryle, un groupe alkylsulfonate, un groupe arylsulfonate, un groupe alkylphosphonate, un groupe arylphosphonate, un groupe (alkyle en C₁-C₆)(alcoxy en C₁-C₆)silyle, un groupe (alkyle en C₁-C₆)aryloxysilyle, un groupe (alkyle en C₁-C₆) (cycloalcoxy en C₃-C₁₀)silyle, un groupe alkylammonium et un groupe arylammonium ;
R' est tel que défini pour R", R"' et R"" lorsqu'il est inclus dans un composé représenté par la formule générale (IA) ou, lorsqu'il est inclus dans un composé représenté par formule générale (IB), est choisi dans le groupe constitué par un groupe alkylène en C₁-C₆ et un groupe cycloalkylène en C₃-C₈, ledit groupe alkylène ou cycloalkylène étant facultativement substitué par un ou plusieurs substituants R₅ ;
- L₂ est un ligand non anionique ;
- X est un ligand anionique ;
- R₃ et R₄ sont chacun un atome d'hydrogène ou un groupe choisi dans le groupe constitué par un groupe alkyle en C₁-C₂₀, un groupe alcényle en C₂-C₂₀, un groupe alcynyle en C₂-C₂₀, un groupe carboxylate en C₁-C₂₀, un groupe alcoxy en C₁-C₂₀, un groupe alcényloxy en C₂-C₂₀, un groupe alcynyloxy en C₂-C₂₀, un groupe aryle, un groupe aryloxy, un groupe (alcoxy en C₁-C₂₀)carbonyle, un groupe (alkyle en C₁-C₈)thio, un groupe (alkyle en C₁-C₂₀)sulfonyle, un groupe (alkyle en C₁-C₂₀)sulfinyle, un groupe (alkyle en C₁-C₂₀)sulfonate, un groupe arylsulfonate, un groupe (alkyle en C₁-C₂₀)phosphonate, un groupe arylphosphonate, un groupe (alkyle en C₁-C₂₀)ammonium et un groupe arylammonium ;
- R' et l'un de R₃ et R₄ peuvent être attachés l'un à l'autre pour former un ligand bidenté ;
- R"' et R"" peuvent être attachés l'un à l'autre pour former un système de cycles aliphatiques comprenant un hétéroatome choisi dans le groupe constitué par un atome d'azote, un atome de phosphore, un atome d'arsenic et un atome d'antimoine ;
- R₃ et R₄ peuvent former ensemble un système de cycles aromatiques condensés, et
- y représente le nombre d'atomes de carbone sp₂ entre M et l'atome de carbone portant R₃ et R₄ et est un nombre entier de 0 à 3 inclus,
des sels, des solvates et des énantiomères de celui-ci, et lesdites espèces cationiques étant associées à un anion.

21. Produit selon l'une quelconque des revendications 12 à 17, qui est une espèce bimétallique cationique représentée par la formule générale (X) : 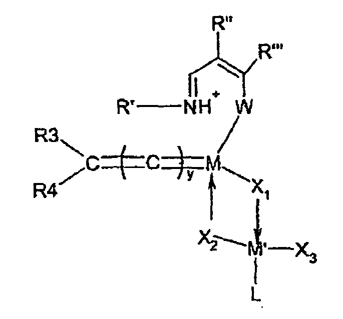 ou une espèce bimétallique cationique représentée par la formule générale (XI) : 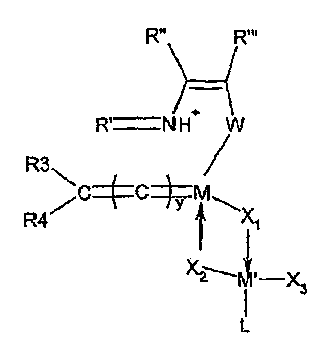 dans lesquelles
- M et M' sont chacun un métal indépendamment choisi dans le groupe constitué par le ruthénium, l'osmium et le fer ;
- W est choisi dans le groupe constitué par un atome d'oxygène, un atome de soufre, un atome de sélénium, NR"", PR"", AsR"" et SbR"" ;
- R", R"' et R"" sont chacun un groupe indépendamment choisi dans le groupe constitué par un atome d'hydrogène, un groupe alkyle en C₁-C₆, un groupe cycloalkyle en C₃-C₈, un groupe (alkyle en C₁-C₆)(alcoxy en C₁-C₆)silyle, un groupe (alkyle en C₁-C₆)aryloxysilyle, un groupe (alkyle en C₁-C₆)(cycloalcoxy en C₃-C₁₀) silyle, un groupe aryle et un groupe hétéroaryle, ou R" et R'" forment ensemble un groupe aryle ou hétéroaryle, chacun desdits groupes (lorsqu'ils ne sont pas un atome d'hydrogène) étant facultativement substitué par un ou plusieurs, de préférence 1 à 3, substituants R₅, chacun indépendamment choisi dans le groupe constitué par un atome d'halogène, un groupe alkyle en C₁-C₆, un groupe alcoxy en C₁-C₆, un groupe aryle, un groupe alkylsulfonate, un groupe arylsulfonate, un groupe alkylphosphonate, un groupe arylphosphonate, un groupe (alkyle en C₁-C₆)(alcoxy en C₁-C₆)silyle, un groupe (alkyle en C₁-C₆)aryloxysilyle, un groupe (alkyle en C₁-C₆)(cycloalcoxy en C₃-C₁₀) silyle, un groupe alkylammonium et un groupe arylammonium ;
- R' est tel que défini pour R", R"' et R"" lorsqu'il est inclus dans un composé représenté par la formule générale (IA) ou, lorsqu'il est inclus dans un composé représenté par la formule générale (IB), est choisi dans le groupe constitué par un groupe alkylène en C₁-C₆ et un groupe cycloalkylène en C₃-C₈, ledit groupe alkylène ou cycloalkylène étant facultativement substitué par un ou plusieurs substituants R₅ ;
- R₃ et R₄ sont chacun un atome d'hydrogène ou un groupe choisi dans le groupe constitué par un groupe alkyle en C₁-C₂₀, un groupe alcényle en C₂-C₂₀, un groupe alcynyle en C₂-C₂₀, un groupe carboxylate en C₁-C₂₀, un groupe alcoxy en C₁-C₂₀, un groupe alcényloxy en C₂-C₂₀, un groupe alcynyloxy en C₂-C₂₀, un groupe aryle, un groupe aryloxy, un groupe (alcoxy en C₁-C₂₀) carbonyle, un groupe (alkyle en C₁-C₈)thio, un groupe (alkyle en C₁-C₂₀) sulfonyle, un groupe (alkyle en C₁-C₂₀)sulfinyle, un groupe (alkyle en C₁-C₂₀)sulfonate, un groupe arylsulfonate, un groupe (alkyle en C₁-C₂₀)phosphonate, un groupe arylphosphonate, un groupe (alkyle en C₁-C₂₀)ammonium et un groupe arylammonium ;
- R' et l'un de R₃ et R₄ peuvent être attachés l'un à l'autre pour former un ligand bidenté ;
- R"' et R"" peuvent être attachés l'un à l'autre pour former un système de cycles aliphatiques comprenant un hétéroatome choisi dans le groupe constitué par un atome d'azote, un atome de phosphore, un atome d'arsenic et un atome d'antimoine ;
- R₃ et R₄ peuvent former ensemble un système de cycles aromatiques condensés, et
- y représente le nombre d'atomes de carbone sp₂ entre M et l'atome de carbone portant R₃ et R₄ et est un nombre entier de 0 à 3 inclus,
- X₁, X₂ et X₃ sont chacun indépendamment choisis parmi des ligands anioniques ; et
- L est un ligand non anionique
y compris les sels, les solvates et les énantiomères de celui-ci.

22. Produit selon l'une quelconque des revendication 12 à 17, **caractérisé en ce qu'**au moins un desdits autres ligands (ii) dudit complexe métallique multi-coordonné (a) est un solvant S et ledit complexe (a) est une espèce cationique associée à un anion A.

23. Produit selon l'une quelconque des revendications 12 à 17, **caractérisé en ce que** ledit complexe métallique multi-coordonné (a) est un complexe bimétallique.

24. Produit selon la revendication 23, **caractérisé en ce qu'**un métal dudit complexe bimétallique est penta-coordonné avec ledit au moins un ligand multidenté de type base de Schiff (i) et avec ledit ou lesdits autres ligands (ii), et l'autre métal est tétra-coordonné avec un ou plusieurs ligands neutres et un ou plusieurs ligands anioniques.

25. Produit selon la revendication 23, **caractérisé en ce que** chaque métal dudit complexe bimétallique est hexa-coordonné avec ledit au moins un ligand multidenté de type base de Schiff (i) et avec ledit ou lesdits autres ligands (ii).

26. Produit selon la revendication 23, **caractérisé en ce que** les deux métaux du complexe bimétallique sont identiques.

27. Produit selon la revendication 23, **caractérisé en ce que** les deux métaux du complexe bimétallique sont différents.

28. Produit selon l'une quelconque des revendications 12 à 17, **caractérisé en ce que** ledit complexe métallique multi-coordonné (a) est un complexe monométallique.

29. Produit selon l'une quelconque des revendications 12 à 17, **caractérisé en ce que** ledit complexe métallique multi-coordonné (a) est un complexe métallique penta-coordonné ou un complexe métallique tétra-coordonné.

30. Produit selon la revendication 29, **caractérisé en ce que** ledit au moins un ligand multidenté de type base de Schiff (i) est un ligand bidenté et ledit complexe métallique multi-coordonné (a) comprend deux autres ligands (ii).

31. Produit selon la revendication 29, **caractérisé en ce que** ledit au moins un ligand multidenté de type base de Schiff (i) est un ligand tridenté et ledit complexe métallique multi-coordonné (a) comprend un seul autre ligand (ii).

32. Produit selon la revendication 29 ou la revendication 30, **caractérisé en ce que** ledit au moins un ligand multidenté de type base de Schiff (i) est représenté par l'une des formules générales (IA) et (IB) : 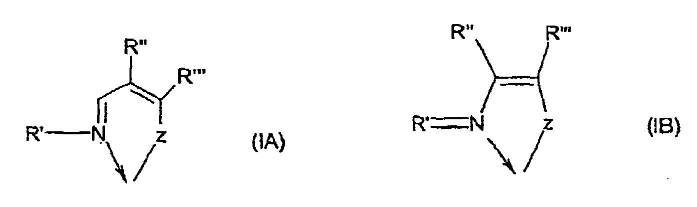 dans lesquelles
- Z est choisi dans le groupe constitué par un atome d'oxygène, un atome de soufre et un atome de sélénium ;
- R" et R"' sont chacun un groupe indépendamment choisi dans le groupe constitué par un atome d'hydrogène, un groupe alkyle en C₁-C₇, un groupe cycloalkyle en C₃-C₁₀, un groupe (alkyle en C₁-C₆)(alcoxy en C₁-C₆)silyle, un groupe (alkyle en C₁-C₆) aryloxysilyle, un groupe (alkyle en C₁-C₆)(cycloalcoxy en C₃-C₁₀)silyle, un groupe aryle et un groupe hétéroaryle, ou R" et R'" forment ensemble un groupe aryle ou hétéroaryle, chacun desdits groupes étant facultativement substitué par un ou plusieurs, de préférence 1 à 3, substituants R₅, chacun indépendamment choisi dans le groupe constitué par un atome d'halogène, un groupe alkyle en C₁-C₆, un groupe alcoxy en C₁-C₆, un groupe aryle, un groupe alkylsulfonate, un groupe arylsulfonate, un groupe alkylphosphonate, un groupe arylphosphonate, un groupe (alkyle en C₁-C₆)(alcoxy en C₁-C₆)silyle, un groupe (alkyle en C₁-C₆)aryloxysilyle, un groupe (alkyle en C₁-C₆) (cycloalcoxy en C₃-C₁₀) silyle, un groupe alkylammonium et un groupe arylammonium ;
- R' est tel que défini pour R" et R'" lorsqu'il est inclus dans un composé représenté par la formule générale (IA) ou, lorsqu'il est inclus dans un composé représenté par formule générale (IB), est choisi dans le groupe constitué par un groupe alkylène en C₁-C₇ et un groupe cycloalkylène en C₃-C₁₀, ledit groupe alkylène ou cycloalkylène étant facultativement substitué par un ou plusieurs substituants R₅.

33. Produit selon la revendication 29, **caractérisé en ce qu'**au moins un desdits autres ligands (ii) dudit complexe métallique multi-coordonné (a) est un dérivé, dans lequel un ou plusieurs atomes d'hydrogène sont substitués par un groupe apportant un encombrement stérique, d'un carbène N-hétérocyclique choisi dans le groupe constitué par l'imidazol-2-ylidène, le dihydroimidazol-2-ylidène, l'oxazol-2-ylidène, le triazol-5-ylidène, le thiazol-2-ylidène, le bis(imidazolin-2-ylidène), le bis(imidazolidin-2-ylidène), le pyrrolylidène, le pyrazolylidène, le dihydropyrrolylidène, le pyrrolylidinylidène et des dérivés benzocondensés de ceux-ci, ou une superbase prophosphatrane non ionique.

34. Produit selon la revendication 29, **caractérisé en ce qu'**au moins un desdits autres ligands (ii) dudit complexe métallique multi-coordonné (a) est un ligand anionique choisi dans le groupe constitué par un groupe alkyle en C₁-C₂₀, un groupe alcényle en C₁-C₂₀, un groupe alcynyle en C₁-C₂₀, un groupe carboxylate en C₁-C₂₀, un groupe alcoxy en C₁-C₂₀, un groupe alcényloxy en C₁-C₂₀, un groupe alcynyloxy en C₁-C₂₀, un groupe aryle, un groupe aryloxy, un groupe (alcoxy en C₁-C₂₀) carbonyle, un groupe (alkyle en C₁-C₈)thio, un groupe (alkyle en C₁-C₂₀) sulfonyle, un groupe (alkyle en C₁-C₂₀)sulfinyle, un groupe (alkyle en C₁-C₂₀)sulfonate, un groupe arylsulfonate, un groupe (alkyle en C₁-C₂₀)phosphonate, un groupe arylphosphonate, un groupe (alkyle en C₁-C₂₀) ammonium, un groupe arylammonium, un atome d'halogène et un groupe cyano.

35. Produit selon la revendication 29, **caractérisé en ce qu'**au moins un desdits autres ligands (ii) dudit complexe métallique multi-coordonné (a) est un ligand de type carbène représenté par la formule générale = [C=] _{y}CR₃R₄, dans laquelle :
- y est un nombre entier de 0 à 3 inclus, et
- R₃ et R₄ sont chacun un atome d'hydrogène ou un groupe hydrocarboné choisi dans le groupe constitué par un groupe alkyle en C₁-C₂₀, un groupe alcényle en C₁-C₂₀, un groupe alcynyle en C₁-C₂₀, un groupe carboxylate en C₁-C₂₀, un groupe alcoxy en C₁-C₂₀, un groupe alcényloxy en C₁-C₂₀, un groupe alcynyloxy en C₁-C₂₀, un groupe aryle, un groupe aryloxy, un groupe (alcoxy en C₁-C₂₀) carbonyle, un groupe (alkyle en C₁-C₈)thio, un groupe (alkyle en C₁-C₂₀) sulfonyle, un groupe (alkyle en C₁-C₂₀) sulfinyle, un groupe (alkyle en C₁-C₂₀)sulfonate, un groupe arylsulfonate, un groupe (alkyle en C₁-C₂₀)phosphonate, un groupe arylphosphonate, un groupe (alkyle en C₁-C₂₀) ammonium et un groupe arylammonium ; ou R₃ et R₄ peuvent former ensemble un système de cycles aromatiques condensés.

36. Produit selon la revendication 35, **caractérisé en ce que** R₃ et R₄ forment ensemble un système de cycles aromatiques condensés de sorte que ledit autre ligand (ii) dudit complexe métallique multi-coordonné (a) est un ligand de type phénylindénylidène.

37. Produit selon la revendication 29, **caractérisé en ce qu'**au moins un desdits autres ligands (ii) dudit complexe métallique multi-coordonné (a) est un ligand insaturé non anionique L¹ choisi dans le groupe constitué par les groupes cycloaliphatiques aromatiques et insaturés, de préférence les groupes aryle, hétéroaryle et cycloalcényle en C₄-C₂₀, ledit groupe cycloaliphatique aromatique ou insaturé étant facultativement substitué par un ou plusieurs groupes alkyle en C₁-C₇ ou groupes attracteurs d'électrons tels que, sans limitation, un atome d'halogène, un groupe nitro, un groupe cyano, un acide (thio)carboxylique, un (thio)ester d'un acide (thio)carboxylique, un (thio)amide d'un acide (thio)carboxylique, un anhydride d'un acide (thio)carboxylique et un halogénure d'un acide (thio)carboxylique.

38. Produit selon la revendication 29, **caractérisé en ce qu'**au moins un desdits autres ligands (ii) dudit complexe métallique multi-coordonné (a) est un ligand non anionique L² choisi dans le groupe constitué par un groupe alkyle en C₁-C₇, un groupe cycloalkyle en C₃-C₁₀, un groupe arylalkyle et un groupe hétérocyclique, ledit groupe étant facultativement substitué par un ou plusieurs susbtituants attracteurs d'électrons tels que, sans limitation, un atome d'halogène, un groupe nitro, un groupe cyano, un acide (thio)carboxylique, un (thio)ester d'un acide (thio) carboxylique, un (thio)amide d'un acide (thio)carboxylique, un anhydride d'un acide (thio)carboxylique et un halogénure d'un acide (thio)carboxylique.

39. Produit selon la revendication 29, **caractérisé en ce que** ledit au moins un ligand multidenté de type base de Schiff (i) est un ligand tétradenté et ledit complexe métallique multi-coordonné (a) comprend un ou deux autres ligands (ii) qui sont des ligands non anionique L⁷ choisis dans le groupe constitué par les groupes cycloaliphatiques aromatiques et insaturés, de préférence les groupes aryle, hétéroaryle et cycloalcényle en C₄-C₂₀, ledit groupe cycloaliphatique aromatique ou insaturé étant facultativement substitué par un ou plusieurs groupes alkyle en C₁-C₇ ou groupes attracteurs d'électrons tels que, sans limitation, un atome d'halogène, un groupe nitro, un groupe cyano, un acide (thio)carboxylique, un (thio)ester d'un acide (thio)carboxylique, un (thio)amide d'un acide (thio)carboxylique, un anhydride d'un acide (thio)carboxylique et un halogénure d'un acide (thio)carboxylique.

40. Produit selon la revendication 12, dans lequel ledit acide (b) est un acide généré *in situ* à partir d'un générateur photoacide.

41. Produit selon la revendication 12, comprenant le produit d'un clivage au moins partiel d'une liaison entre le métal et ledit au moins un ligand multidenté de type base de Schiff (i).

42. Système catalytique comprenant :
(a) comme espèce catalytique principale, un produit réactionnel selon l'une quelconque des revendications 12 à 41, et
(b) un ou plusieurs composants catalytiques secondaires choisis dans le groupe constitué par les co-catalyseurs de type acide de Lewis (b₁), les activateurs de catalyseur (b₂) et les initiateurs possédant un atome ou un groupe transférable de manière radicalaire (b₃).

43. Système catalytique selon la revendication 42, dans lequel le composant catalytique secondaire comprend un co-catalyseur (b₁) choisi dans le groupe constitué par les trihalogénures de bore ; les trihalogénures de phosphore ; les composés trialkylbores ; les composés triarylbores ; les composés d'organoaluminium ; les dihalogénures de magnésium ; les trihalogénures d'aluminium ; le tétrachlorure d'étain ; les trihalogénures ou les tétrahalogénures ou les tétraalcoxydes de titane ou de vanadium ; les pentahalogénures d'antimoine et de bismuth.

44. Système catalytique selon la revendication 42, dans lequel le composant catalytique secondaire comprend, en tant qu'activateur de catalyseur (b₂), un composé diazo.

45. Système catalytique selon la revendication 42, dans lequel le composant catalytique secondaire comprend, en tant qu'initiateur possédant un atome ou un groupe transférable de manière radicalaire (b₃), un composé représenté par la formule R₃₅R₃₆R₃₇CX₁, dans laquelle :
- X₁ est choisi dans le groupe constitué par un atome d'halogène, OR₃₈ (où R₃₈ est choisi parmi un groupe alkyle en C₁-C₂₀, un groupe polyhalogénoalkyle en C₁-C₂₀, un groupe alcynyle en C₂-C₂₀ (de préférence un groupe acétylènyle), un groupe alcényle en C₂-C₂₀ (de préférence un groupe vinyle), un groupe phényle facultativement substitué par 1 à 5 atomes d'halogène ou groupes alkyle en C₁-C₇ et un groupe alkyle en C₁-C₇ substitué par un groupe phényle, SR₃₉, OC (=O) R₃₉, OP(=O)R₃₉, OP(=O)(OR₃₉)₂, OP(=O)OR₃₉, O-N(R₃₉)₂ et S-C(=S)N(R₃₉)₂, où R₃₉ est un groupe aryle ou un groupe alkyle en C₁-C₂₀, ou lorsqu'un groupe N(R₃₉)₂ est présent, les deux groupes R₃₉ peuvent être reliés pour former un cycle hétérocyclique de 5, 6 ou 7 éléments (conformément à la définition d'un groupe hétéroaryle ci-dessus), et
- R₃₅, R₃₆ et R₃₇ sont chacun indépendamment choisis dans le groupe constitué par un atome d'hydrogène, un atome d'halogène, un groupe alkyle en C₁-C₂₀ (de préférence un groupe alkyle en C₁-C₆), un groupe cycloalkyle en C₃-C₈, C(=O)R₄₀ (où R₄₀ est choisi dans le groupe constitué par un groupe alkyle en C₁-C₂₀, un groupe alcoxy en C₁-C₂₀, un groupe aryloxy ou un groupe hétéroaryloxy), C(=O)NR₄₁R₄₂ (où R₄₁ et R₄₂ sont indépendamment choisis dans le groupe constitué par un atome d'hydrogène et un groupe alkyle en C₁-C₂₀ ou R₄₁ et R₄₂ peuvent être reliés pour former un groupe alkylène de 2 à 5 atomes de carbone), COCl, OH, CN, un groupe alcényle en C₂-C₂₀ (de préférence un groupe vinyle), un groupe alcynyle en C₂-C₂₀, un groupe oxiranyle, un groupe glycidyle, un groupe aryle, un groupe hétéroaryle, un groupe arylalkyle et un groupe alcényle en C₂-C₂₀ substitué par un groupe aryle.

46. Catalyseur sur support, comprenant :
(a) un produit réactionnel catalytiquement actif selon l'une quelconque des revendications 12 à 41, ou un système catalytique selon la revendication 46, et
(b) une quantité d'un support approprié pour le support dudit produit catalytiquement actif ou dudit système catalytique (a).

47. Procédé de réalisation d'une réaction de métathèse d'oléfine ou d'acétylène ou d'une réaction impliquant le transfert d'un atome ou d'un groupe vers un composé éthyléniquement ou acétyléniquement insaturé ou un autre substrat réactif en présence d'un composant catalytique, ledit composant catalytique comprenant un produit selon l'une quelconque des revendications 12 à 41 ou un système catalytique selon l'une quelconque des revendications 42 à 45.

48. Procédé selon la revendication 47, dans lequel ladite réaction impliquant le transfert d'un atome ou d'un groupe vers une oléfine ou un autre substrat réactif est choisie dans le groupe constitué par :
- la polymérisation radicalaire par transfert d'atome ou de groupe d'un ou de plusieurs monomères (co)polymérisables de manière radicalaire, en particulier les monomères mono- et diéthyléniquement insaturés ;
- l'addition radicalaire par transfert d'atome d'un polyhalogénométhane représenté par la formule CXₙH₄₋ₙ, dans laquelle X est un atome d'halogène et n est un nombre entier de 2 à 4, sur un composé éthyléniquement insaturé pour produire l'adduit polyhalogéné saturé correspondant ;
- la réaction de vinylation d'un mono- ou di-alcyne avec un acide mono- ou di-carboxylique pour produire des esters d'alk-1-ényle ou des esters énoliques ou des adduits de type Markovnikov ou des adduits de type anti-Markovnikov ou des mélanges de ceux-ci ;
- la cyclopropanation d'un composé α-éthyléniquement insaturé pour produire un composé organique comprenant un ou plusieurs motifs structuraux cyclopropane ;
- la synthèse de quinoléine par cyclisation oxydante d'un alcool 2-aminobenzylique avec des cétones ;
- l'époxydation de composés α-éthyliquement insaturés pour la production d'époxydes ;
- l'oxydation de composés organiques comprenant l'oxydation d'hydrocarbures saturés pour la production d'alcools, ou de sulfures pour la production de sulfoxydes et de sulfones, ou de phosphines pour la production de phosphonates, ou d'alcools et d'aldéhydes pour la production d'acides carboxyliques ;
- la cyclopropénation d'un alcyne pour la production d'un composé organique possédant un ou plusieurs motifs structuraux cyclopropène ;
- l'hydrocyanation de composés α-éthyliquement insaturés pour la production de nitriles saturés, ou d'alcynes pour la production de nitriles insaturés, ou d'aldéhydes ou de cétones α,β-insaturé(e)s pour la production de composés β-cyano-carbonyle ;
- l'hydrosilylation d'oléfines pour la production de silanes saturés, ou d'alcynes pour la production de silanes insaturés, ou de cétones pour la production d'éthers silyliques, ou la triméthylsilylcyanation d'aldéhydes pour la production d'éthers triméthylsilyliques de cyanohydrine ;
- l'aziridination d'imines ou d'alcènes pour la production de composés organiques comprenant un ou plusieurs motifs structuraux aziridine ;
- l'hydroamidation d'oléfines pour la production d'amides saturés ;
- l'hydrogénation d'oléfines pour la production d'alcanes, ou de cétones pour la production d'alcools ;
- l'aminolyse d'oléfines pour la production d'amines primaires ou secondaires saturées ;
- l'isomérisation d'alcools, de préférence d'alcools allyliques, pour la production d'aldéhydes ;
- la réticulation de Grignard d'halogénures d'alkyle ou d'aryle pour la production d'alcanes ou d'arylalcanes ;
- l'hydroboration d'oléfines pour la production d'alkylboranes et de trialkylboranes ;
- la réduction par des hydrures d'aldéhydes et de cétones pour la production d'alcools ;
- la condensation aldolique de composés carboxyliques saturés pour la production de composés carboxyliques α,β-insaturés ou de composés β-hydroxycarbonyliques, et la condensation aldolique intramoléculaire de dialdéhydes ou de diones pour la production de composés carboxyliques α,β-insaturés cycliques ;
- l'addition de Michael d'une cétone ou d'un composé β-dicarbonylique sur un composé carboxylique α,β-insaturé pour la production de composés polycarboxyliques saturés ;
- l'annulation de Robinson pour la production de composés carboxyliques polycyliques saturés ;
- les réactions de Heck d'un halogénure d'aryle ou d'un 1-hétéro-2,4-cyclopentadiène ou d'un dérivé benzocondensé de celui-ci avec un composé α-éthylèniquement insaturé pour la production d'arylalcènes ou d'hétéroarylalcènes ;
- la codimérisation d'alcènes pour la production d'hydrocarbures saturés supérieurs ou d'alcynes pour la production d'alcènes supérieurs ;
- l'hydroxylation d'oléfines pour la production d'alcools ;
- l'hydroamination d'oléfines et d'alcynes pour la production d'amines ;
- l'alkylation, de préférence l'alkylation allylique, de cétones pour la production de cétones alkylées, de préférence de cétones allyliques ; et
- les réactions de Diels-Alder telles que la cycloaddition d'un diène conjugué sur un composé α-éthyléniquement insaturé pour la production de cyclohexènes facultativement substitués, ou la cycloaddition d'un furane sur un composé α-éthyléniquement insaturé pour la production de 7-oxanorbornènes facultativement substitués.

49. Procédé selon la revendication 47, dans lequel ladite réaction de métathèse est la polymérisation par métathèse avec ouverture de cycle d'oléfines cycliques sous contrainte.

50. Procédé selon la revendication 47, dans lequel le composant catalytique est supporté sur un support.

51. Procédé selon la revendication 50, dans lequel ledit support est choisi dans le groupe constitué par les solides inorganiques poreux, tels que les matériaux amorphes ou paracristallins, les tamis moléculaires cristallins et les matériaux stratifiés modifiés comprenant un ou plusieurs oxydes inorganiques, et les résines polymères organiques.
